# FASCICULE DE BREVET EUROPEEN

(11) **EP 1 183 237 B1**
(45) Date de publication et mention de la délivrance du brevet: **28.01.2004**
(21) Numéro de dépôt: 00936951.3
(22) Date de dépôt: 26.05.2000
(51) Int. Cl.: C07C 327/30, C07C 327/34, A61K 31/265, A61P 31/18, A61K 7/06, A61K 7/48

(54) **NOUVEAUX ANTIOXYDANTS, PROCEDES DE PREPARATION ET UTILISATIONS**
ANTIOXIDANTIEN, VERFAHREN ZU IHRER HERSTELLUNG UND IHRE VERWENDUNGEN
NOVEL ANTIOXIDANTS, PREPARATION METHODS AND USES

(30) Priorité: 27.05.1999 FR 9906708
(43) Date de publication de la demande: 06.03.2002
(73) Titulaire: CENTRE NATIONAL DE LA RECHERCHE SCIENTIFIQUE (CNRS), 75794 Paris Cedex 16 (FR); COMMISSARIAT A L'ENERGIE ATOMIQUE, 75015 Paris (FR)
(72) Inventeur: Oiry, Joûl, F-34070 Montpellier (FR); Puy, Jean-Yves, F-34000 Montpellier (FR); IMBACH, Jean-Louis, F-34000 Montpellier (FR); CLAYETTE, Pascal, F-78000 Versailles (FR); FRETIER, Philippe, F-94130 Nogent-sur-Marne (FR)
(74) Mandataire: Warcoin, Jacques
(86) Numéro de dépôt international: PCT/FR2000/001447
(87) Numéro de publication internationale: WO 2000/073266

(56) Documents cités:
- WO-A-92/21368
- WO-A-95/10268
- US-A- 4 927 808
- US-A- 5 580 577
- US-A- 5 624 955

## Description

La présente invention concerne de nouveaux composés à activité antioxydante, leurs procédés de préparation et leurs utilisations notamment pour la préparation de médicaments destinés à augmenter le taux intracellulaire et/ou extracellulaire de glutathion (GSH).

De plus en plus de travaux montrent que les espèces réactives de l'oxygène jouent un rôle important dans de multiples processus biologiques et notamment dans le développement de multiples pathologies humaines et plus particulièrement dans les infections rétrovirales par le virus de l'immunodéficience humaine (VIH).

Les espèces réactives de l'oxygène (ERO, ion superoxyde, peroxyde d'hydrogène, ion hypochloreux, radicaux hydroxyles, etc...) sont des produits naturels dont l'origine est variable; elles proviennent des cellules inflammatoires activées, des cellules métabolisant des xénobiotiques ou des cellules exposées à des milieux environnementaux particuliers tels la fumée de cigarette.

Diverses substances sont connues pour avoir une activité de piégeur de ces espèces réactives de l'oxygène au niveau intracellulaire ou extracellulaire.

On peut citer par exemple le glutathion qui est un tripeptide (L-γ-glutamyl-L-cystéinylglycine, GSH) trouvé dans toutes les cellules eucaryotes. Il est synthétisé et dégradé dans la cellule, principalement via sa forme réduite, le GSH. Ce tripeptide réduit, qui prend part à de nombreuses fonctions cellulaires comme par exemple, la synthèse protéique et nucléique, et le transport d'acides aminés, constitue le principal mécanisme de défense intracellulaire contre le stress oxydatif. Les facteurs qui favorisent la formation d'espèces réactives de l'oxygène conduisent à la consommation des réserves de glutathion.

La N-acétyl-L-cystéine (NAC) est connue depuis de nombreuses années comme médicament pour la cornée, comme antidote à l'empoisonnement par l'acétaminophène et comme agent mucolytique en coupant les liaisons disulfures dans les mucoprotéines. Parce que la NAC est utilisée thérapeutiquement dans de multiples pathologies dans lesquelles les oxydants semblent jouer un rôle, il a été suggéré qu'elle fonctionne comme un antioxydant. Le mécanisme d'action de la NAC repose sur son aptitude à réduire la cystine extracellulaire en cystéine ou à être une source de métabolites SH (fonction thiol). Comme source de groupes SH, la NAC stimule la synthèse de GSH, augmente l'activité glutathione-S-transférase, favorise la détoxification et agit directement sur les espèces oxydantes réactives. La NAC et par extension les variants acylés de l'acide aminé L-cystéine sont une excellente source de groupes SH et sont convertis dans l'organisme en métabolites capables de stimuler la synthèse de glutathion, favorisant ainsi la détoxification et agissant directement comme piègeurs d'espèces réactives de l'oxygène.

Compte-tenu du rôle central du GSH dans les mécanismes de détoxification cellulaire, de nombreuses alternatives visant à remonter son taux intracellulaire ont été envisagées comme stratégie thérapeutique adjuvante dans de nombreuses pathologies humaines et plus particulièrement dans l'infection par le virus de l'immunodéficience humaine (VIH) au cours des dernières années (WO 92/21368; WO 95/10268; US 4 927 808; US 5 580 577). Tout d'abord, les approches ont cherché à supplémenter la molécule déficiente. Elles ont ensuite visé à alimenter le cycle du γ-glutamyl. Ainsi, la L-cystéine, le GSH lui-même, la NAC, l'acide 2-oxothiazolidine-4(R)-carboxylique (OTC), et la cystéamine (MEA), dont les formules sont reproduites ci-après, ont été testés comme adjuvants potentiels aux antirétroviraux dans le traitement des infections par le VIH.

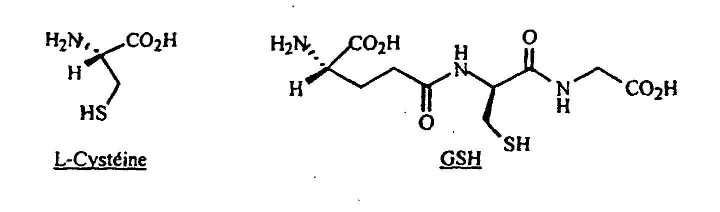

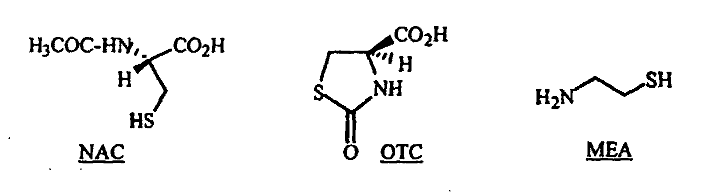

Les effets bénéfiques de ces molécules ont été limités *in vivo* par une faible biodisponibilité, une métabolisation trop rapide, et des concentrations administrables insuffisantes. La NAC par exemple est une molécule relativement labile qui, lors de sa rapide dégradation, relargue des composés contenant des sulfures malodorants tels l'H₂S. Ce problème d'instabilité a limité l'utilisation de la NAC, ou d'autres composés fournisseurs d'une source de -SH, tels que la L-cystéine ou ses variants acylés, pour la préparation de formulations utilisables en pharmacie, dermatologie ou cosmétique.

Les inventeurs ont maintenant mis au point de nouveaux composés, susceptibles d'agir sur le taux intracellulaire ou extracellulaire de glutathion, qui ne présentent pas l'instabilité qui a été constatée pour les produits connus. C'est pourquoi, la présente invention a pour objet les composés de formule générale (I) suivante: 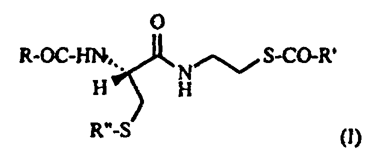 et dans laquelle:
- R et R' représentent indépendamment un radical alkyle en C₁-C₇, linéaire ou ramifié, ou un groupe aryle non substitué ou substitué par un ou plusieurs radicaux choisis parmi les halogènes, les radicaux alkyles en C₁-C₃ linéaires ou ramifiés, et les radicaux -OH (hydroxyle).
- R" est l'hydrogène ou un groupe CO-R¹ dans lequel R¹ est un radical alkyle en C₁-C₇ linéaire ou ramifié, ou un groupe aryle non substitué ou substitué par un ou plusieurs radicaux choisis parmi les halogènes, les radicaux alkyles en C₁-C₃ linéaires ou ramifiés, et les radicaux -OH ;
ainsi que les dimères formés par un pont disulfure à partir de l'un et/ou l'autre des deux atomes de soufre de la molécule de formule générale I constitué par les radicaux R" ou par les radicaux R'CO- des deux molécules ainsi que les formes thiazolidines correspondantes.

De préférence, les radicaux alkyles R, R' et R" sont en C₁-C₃. Les halogènes sont de préférence le chlore et le fluor.

Dans ces composés, la NAC et la MEA sont associées et protégées ou non par des groupements biolabiles.

Selon un mode préféré de réalisation, la présente invention concerne un composé de formule générale I tel que R est un groupe méthyle (-CH₃). Dans un mode plus préféré de réalisation, la présente invention concerne un composé de formule générale I tel que R et R' sont des groupes méthyles (-CH₃). Dans le mode préféré de réalisation, l'invention concerne le composé dénommé N-(N-acétyl-L-cystéinyl)-S-acétylcystéamine appelé ci-après **I-152,** tel que, dans la formule gênérale I, R et R' sont des groupes méthyles (-CH₃) et R" est l'hydrogène. 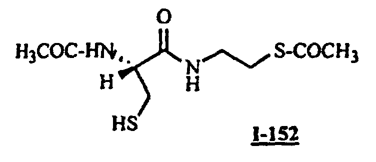 Ce composé est particulièrement intéressant pour ses propriétés qui le rendent actif pour le traitement et/ ou la prévention de pathologies ou de troubles liés à une déplétion en glutathion intra et/ou extracellulaire, notamment le traitement des infections virales et plus particulièrement les infections par le virus de l'immunodéficience humaine (VIH).

Selon un autre mode préféré de réalisation, l'invention concerne le composé dénommé N-(N,S-Bis-acétyl-L-cystéinyl)-S-acétylcystéamine, appelé ci-après **I-176,** tel que, dans la formule générale I, R et R' sont des groupes méthyles (-CH₃) et R" est un groupe acétyle (-COCH₃).

Selon un autre mode préféré de réalisation, l'invention concerne le composé dénommé N-(N-acétyl-S-isobutyryl-L-cystéinyl)-S-acétylcystéamine, appelé ci-après **I-177,** tel que dans la formule générale I, R et R' sont des groupes méthyles (-CH₃) et R" est un groupe isobutyryle (-COCH(CH₃)₂).

Selon un autre mode préféré de réalisation, l'invention concerne le composé dénommé N-(N-acétyl-S-pivaloyl-L-cystéinyl)-S-acétylcystéamine, appelé ci-après **I-178,** tel que dans la formule générale I, R et R' sont des groupes méthyles (-CH₃) et R" est un groupe pivaloyle (-COC(CH₃)₃).

C'est également un des objets de la présente invention de fournir un composé de formule générale I dans lequel R est un groupe méthyle (-CH₃) et R' est sélectionné parmi le groupe isopropyle (-CH(CH₃)₂), tertiobutyle (-C(CH₃)₃) et phényle (-C₆H₅); un tel composé présente de préférence un groupe R" sélectionné parmi l'hydrogène (-H), le groupe acétyle (-COCH₃), le groupe isobutyryle (-COCH(CH₃)₂), le groupe pivaloyle (-COC(CH₃)₃), le groupe benzoyle (-CO-C₆H₅). Plus particulièrement l'invention vise à fournir le composé ci-après dénommé:
- **I-188** tel que dans la formule générale I, R est un groupe méthyle (-CH₃), R' est un groupe isopropyle (-CH(CH₃)₂) et R" est l'hydrogène (-H).
- **I-189** tel que dans la formule générale I, R est un groupe méthyle (-CH₃), R' est un groupe isopropyle (-CH(CH₃)₂) et R" est le groupe acétyle (-COCH₃).
- **I-190** tel que dans la formule générale I, R est un groupe méthyle (-CH₃), R' est un groupe isopropyle (-CH(CH₃)₂) et R" est le groupe isobutyryle (-COCH(CH₃)₂).
- **I-191** tel que dans la formule générale I, R est un groupe méthyle (-CH₃), R' est un groupe isopropyle (-CH(CH₃)₂) et R" est le groupe pivaloyle (-COC(CH₃)₃).
- **I-192** tel que dans la formule générale I, R est un groupe méthyle (-CH₃), R' est un groupe isopropyle (-CH(CH₃)₂) et R" est le groupe benzoyle (-CO-C₆H₅).
- **I-193** tel que dans la formule générale I, R est un groupe méthyle (-CH₃), R' est un groupe tertiobutyle (-C(CH₃)₃) et R" est l'hydrogène (-H).
- **I-194** tel que dans la formule générale I, R est un groupe méthyle (-CH₃), R' est un groupe tertiobutyle (-C(CH₃)₃) et R" est le groupe acétyle (-COCH₃).
- **I-195** tel que dans la formule générale I, R est un groupe méthyle (-CH₃), R' est un groupe tertiobutyle (-C(CH₃)₃) et R" est le groupe isobutyryle (-COCH(CH₃)₂).
- **I-196** tel que dans la formule générale I, R est un groupe méthyle (-CH₃), R' est un groupe tertiobutyle (-C(CH₃)₃) et R" est le groupe pivaloyle (-COC(CH₃)₃).
- **I-197** tel que dans la formule générale I, R est un groupe méthyle (-CH₃), R' est un groupe tertiobutyle (-C(CH₃)₃) et R" est le groupe benzoyle (-CO-C₆H₅).
- **I-198** tel que dans la formule générale I, R est un groupe méthyle (-CH₃), R' est un groupe phényle (-C₆H₅) et R" est l'hydrogène (-H).
- **I-199** tel que dans la formule générale I, R est un groupe méthyle (-CH₃), R' est un groupe phényle (-C₆H₅) et R" est le groupe acétyle (-COCH₃).
- **I-200** tel que dans la formule générale I, R est un groupe méthyle (-CH₃), R' est un groupe phényle (-C₆H₅) et R" est le groupe isobutyryle (-COCH(CH₃)₂).
- **I-201** tel que dans la formule générale I, R est un groupe méthyle (-CH₃), R' est un groupe phényle (-C₆H₅) et R" est le groupe pivaloyle (-COC(CH₃)₃).
- **I-202** tel que dans la formule générale I, R est un groupe méthyle (-CH₃), R' est un groupe phényle (-C₆H₅) et R" est le groupe benzoyle (-CO-C₆H₅).

C'est également un des objets de la présente invention de fournir un composé de formule générale I qui constitue un intermédiaire dans la synthèse du composé I-152, de ses dérivés acylés ou de ses analogues. Ainsi, l'invention concerne également le composé de formule générale 1 dénommé **10** dans lequel R est un groupe méthyle (-CH₃), R' est le groupe isopropyle (-CH(CH₃)₂) et R" est un groupe trityl. L'invention concerne également le composé de formule générale I dénommé **11** dans lequel R est un groupe méthyle (-CH₃), R' est le groupe tertiobutyle (-C(CH₃)₃) et R" est un groupe trityl. L'invention concerne également le composé de formule générale I dénommé **12** dans lequel R est un groupe méthyle (-CH₃), R' est le groupe phényle (-C₆H₅) et R" est un groupe trityl.

C'est également un des objets de la présente invention de fournir un composé de formule générale I dans lequel R est un groupe isopropyle (-CH(CH₃)₂) ; un tel composé de l'invention se caractérise de préférence en ce que R' est sélectionné parmi le groupe méthyle (-CH₃), isopropyle (-CH(CH₃)₂), tertiobutyle (-C(CH₃)₃) et phényle (-C₆H₅); un tel composé présente de préférence un groupe R" sélectionné parmi l'hydrogène (-H), le groupe acétyle (-COCH₃), le groupe isobutyryle (-COCH(CH₃)₂), le groupe pivaloyle (-COC(CH₃)₃), le groupe benzoyle (-CO-C₆H₅). Plus particulièrement l'invention vise à fournir le composé ci-après dénommé :
- **I-203** tel que dans la formule générale I, R est un groupe isopropyle (-CH(CH₃)₂), R' est un groupe méthyle (-CH₃) et R" est l'hydrogène (-H).
- **I-204** tel que dans la formule générale I, R est un groupe isopropyle (-CH(CH₃)₂), R' est un groupe méthyle (-CH₃) et R" est le groupe acétyle (-COCH₃).
- **I-205** tel que dans la formule générale I, R est un groupe isopropyle (-CH(CH₃)₂), R' est un groupe méthyle (-CH₃) et R" est le groupe isobutyryle (-COCH(CH₃)₂).
- **I-206** tel que dans la formule générale I, R est un groupe isopropyle (-CH(CH₃)₂), R' est un groupe méthyle (-CH₃) et R" est le groupe pivaloyle (-COC(CH₃)₃).
- **I-207** tel que dans la formule générale I, R est un groupe isopropyle (-CH(CH₃)₂), R' est un groupe méthyle (-CH₃) et R" est le groupe benzoyle (-CO-C₆H₅).
- **I-208** tel que dans la formule générale I, R est un groupe isopropyle (-CH(CH₃)₂), R' est un groupe isopropyle (-CH(CH₃)₂) et R" est l'hydrogène (-H).
- **I-209** tel que dans la formule générale I, R est un groupe isopropyle (-CH(CH₃)₂), R' est un groupe isopropyle (-CH(CH₃)₂) et R" est le groupe acétyle (-COCH₃).
- **I-210** tel que dans la formule générale I, R est un groupe isopropyle (-CH(CH₃)₂), R' est un groupe isopropyle (-CH(CH₃)₂) et R" est le groupe isobutyryle (-COCH(CH₃)₂).
- **I-211** tel que dans la formule générale I, R est un groupe isopropyle (-CH(CH₃)₂), R' est un groupe isopropyle (-CH(CH₃)₂) et R" est le groupe benzoyle (-CO-C₆H₅).
- **I-214** tel que dans la formule générale I, R est un groupe isopropyle (-CH(CH₃)₂), R' est un groupe tertiobutyle (-C(CH₃)₃) et R" est l'hydrogène (-H).
- **I-215** tel que dans la formule générale I, R est un groupe isopropyle (-CH(CH₃)₂), R' est un groupe tertiobutyle (-C(CH₃)₃) et R" est le groupe acétyle (-COCH₃).
- **I-216** tel que dans la formule générale I, R est un groupe isopropyle (-CH(CH₃)₂), R' est un groupe tertiobutyle (-C(CH₃)₃) et R" est le groupe isobutyryle (-COCH(CH₃)₂).
- **I-217** tel que dans la formule générale I, R est un groupe isopropyle (-CH(CH₃)₂), R' est un groupe tertiobutyle (-C(CH₃)₃) et R" est le groupe pivaloyle (-COC(CH₃)₃).
- **I-218** tel que dans la formule générale I, R est un groupe isopropyle (-CH(CH₃)₂), R' est un groupe tertiobutyle (-C(CH₃)₃) et R" est le groupe benzoyle (-CO-C₆H₅).
- **I-219** tel que dans la formule générale I, R est un groupe isopropyle (-CH(CH₃)₂), R' est un groupe phényle (-C₆H₅) et R" est l'hydrogène (-H).
- **I-220** tel que dans la formule générale I, R est un groupe isopropyle (-CH(CH₃)₂), R' est un groupe phényle (-C₆H₅) et R" est le groupe acétyle (-COCH₃).
- **I-221** tel que dans la formule générale I, R est un groupe isopropyle (-CH(CH₃)₂), R' est un groupe phényle (-C₆H₅) et R" est le groupe isobutyryle (-COCH(CH₃)₂).
- **I-222** tel que dans la formule générale I, R est un groupe isopropyle (-CH(CH₃)₂), R' est un groupe phényle (-C₆H₅) et R" est le groupe groupe pivaloyle (-COC(CH₃)₃).
- **I-223** tel que dans la formule générale I, R est un groupe isopropyle (-CH(CH₃)₂), R' est un groupe phényle (-C₆H₅) et R" est le groupe benzoyle (-CO-C₆H₅).

C'est également un des objets de la présente invention de fournir un composé de formule générale 1 qui constitue un intermédiaire dans la synthèse du composé I-152, de ses dérivés acylés ou de ses analogues. Ainsi, l'invention concerne également le composé de formule générale I dénommé **14** dans lequel R est un groupe isopropyle (-CH(CH₃)₂), R' est le groupe méthyle (-CH₃) et R" est un groupe trityl. L'invention concerne également le composé de formule générale I dénommé **15** dans lequel R est un groupe isopropyle (-CH(CH₃)₂), R' est le groupe isopropyle (-CH(CH₃)₂), et R" est un groupe trityl. L'invention concerne également le composé de formule générale I dénommé **16** dans lequel R est un groupe isopropyle (-CH(CH₃)₂), R' est le groupe tertiobutyle (-C(CH₃)₃) et R" est un groupe trityl. L'invention concerne également le composé de formule générale I dénommé **17** dans lequel R est un groupe isopropyle (-CH(CH₃)₂), R' est le groupe phényle (-C₆H₅) et R" est un groupe trityl.

L'invention concerne également les différents composés de l'invention précédemment évoqués sous la forme thiazolidine. Plus particulièrement, l'invention concerne les composés **I-212** et **I-213** dont la formule chimique est donnée dans le schéma 5 ci-après.

L'invention concerne les composés de formule I sous la forme libre.

La présente invention a également pour objet un procédé de préparation des composés de formule générale (I) selon des procédés analogues à ceux utilisés en synthèse peptidique.

Selon un premier mode de préparation des composés de l'invention, le procédé selon l'invention comprend les étapes suivantes:
a) protection de la N-acyl-L-cystéine pour fournir le composé N-acyl-S-trityl-L-cystéine;
b) couplage de la N-acyl-S-trityl-L-cystéine avec le chlorhydrate de S-acylcystéamine pour fournir le composé N-(N-acyl-S-trityl-L-cystéinyl)-S-acylcystéamine.

Toujours selon un premier mode de préparation, les composés de l'invention sous forme thiazolidine peuvent être préparés selon le procédé qui comprend les étapes suivantes :
a) Protection de la N-acyl-L-cystéine pour fournir le composé N-acyl-S-trityl-L-cystéine ; puis
b) Couplage de la N-acyl-S-trityl-L-cystéine protégée avec la thiazolidine.

Les composés ainsi obtenus peuvent subir les étapes suivantes de :
c) Déprotection dudit composé obtenu à l'étape b) précédente, puis
d) Libération du thiol libre de formule (I).

Ainsi, selon un mode préféré de réalisation, le procédé de préparation de composés de l'invention comprend les étapes de :
a) protection de la N-acyl-L-cystéine pour fournir le composé N-acyl-S-trityl-L-cystéine;
b) couplage de la N-acyl-S-trityl-L-cystéine avec le chlorhydrate de S-acylcystéamine pour fournir le composé N-(N-acyl-S-trityl-L-cystéinyl)-S-acylcystéamine ;
c) réaction de S-détritylation du composé N-(N-acyl-S-trityl-L-cystéinyl)-S-acylcystéamine en solution méthanolique et chloroformique avec un mélange de nitrate d'argent, de pyridine et de méthanol pour fournir le sulfure d'argent correspondant;
d) mise en suspension dudit sulfure d'argent correspondant dans du chloroforme puis libération du thiol libre en présence d'HCl ou d'H₂S.

Plus particulièrement, la préparation du composé I-152 de l'invention peut être réalisée par le procédé précédent ; pour ce faire, ledit composé N-acyl-S-trityl-L-cystéine de l'étape a) est le N-acétyl-S-trityl-L-cystéine et ledit chlorhydrate de S-acylcystéamine de l'étape b) est le chlorhydrate de S-acétylcystéamine.

Une étape supplémentaire de S-acylation peut être adjointe au procédé précédent pour la préparation de composé selon l'invention.

Selon un second mode de réalisation, le procédé selon l'invention, adapté pour la préparation du composé de formule générale I, dans laquelle R = R' et R" est un hydrogène, comprend les étapes suivantes:
a) estérification de la fonction carboxylique de la N-Boc-L-sérine (1) par la N-hydroxysuccinimide dans du N, N-diméthylformamide (DMF) en présence de 1,3-dicyclohexylcarbodiimide (DCC) pour former l'ester actif (1'); puis,
b) condensation *in situ* de l'ester actif formé (1') avec l'éthanolamine (2) pour fournir le composé N-(N-Boc-L-séryl)-2-aminoéthanol (3); puis,
c) réaction de Mitsunobu sur le composé (3) avec de la triphénylphosphine et du diisopropyl azodicarboxylate en présence d'acide thiocarboxylique dans le tétrahydrofuranne pour fournir le composé N-(N-Boc-S-acyl-L-cystéinyl)-S-acylcystéamine (4); dans le cadre de la préparation du composé I-152, l'acide thiocarboxylique est l'acide thioacétique ; puis,
d) déprotection du composé (4) avec l'acide trifluoroacétique.

Dans un mode particulier de réalisation, l'invention concerne deux procédés de préparation du composé I-152.

Le premier procédé de préparation du composé I-152 (Schéma 1) correspond au procédé de préparation du composé de formule générale (I) décrit précédemment et fait intervenir la L-cystéine correctement protégée. Ce procédé de préparation se caractérise en ce qu'il comprend les étapes suivantes (i) de couplage de la N-acétyl-S-trityl-L-cystéine (7) avec le chlorhydrate de S-acétylcystéamine pour fournir le composé N-(N-acétyl-S-trityl-L-cystéinyl)-S-acétylcystéamine (8); puis (ii) de réaction de S-détrytilation du composé N-(N-acétyl-S-trityl-L-cystéinyl)-S-acétylcystéamine en solution méthanolique et chloroformique avec un mélange de nitrate d'argent, de pyridine et de méthanol pour fournir le sulfure d'argent correspondant (9); puis (iii) de mise en suspension dudit sulfure d'argent correspondant dans du chloroforme; puis (iv) de libération du thiol libre en présence d'HCl ou d'H₂S.

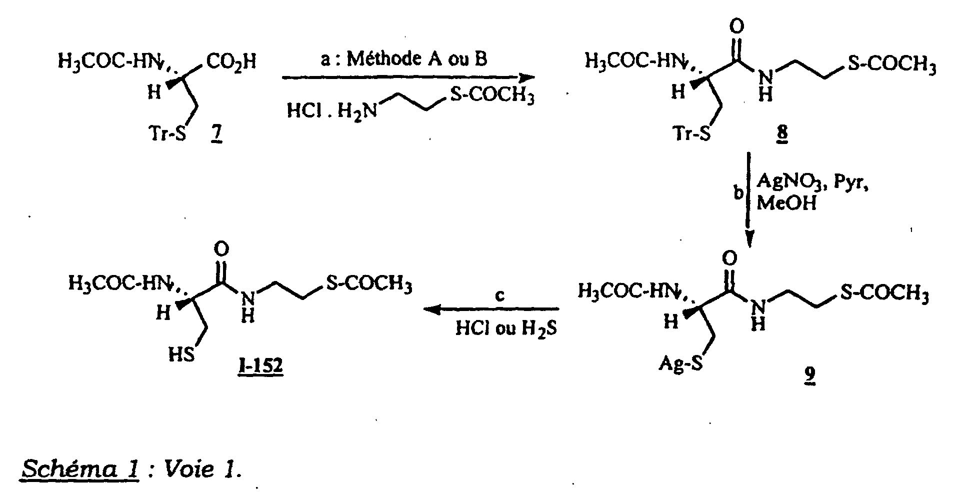

### Méthode A: Couplage, via un anhydride mixte formé in situ, de la N-acétyl-S-trityl-L-cystéine (7) avec le chlorhydrate de S-acétytcystéamine; Méthode B: même réaction de couplage, via un ester activé formé in situ, de 7. (b) S-détritylation avec formation du sulfure d'argent correspondant. (c) Libération du thiol libre.

■ *La méthode A* est basée sur la formation, *in situ*, d'un anhydride mixte par réaction de 7 avec le chloroformiate d'isobutyle dans de l'AcOEt, en présence de N-méthylmorpholine (NMM). L'anhydride est ensuite condensé avec la S-acétylcystéamine, libérée de son . chlorhydrate par de la NMM, pour fournir 8 avec un rendement après traitements de 55%.
■ *La méthode B* utilise, *in situ*, l'ester actif N-succinimidyl de 7 qui après condensation avec la S-acétylcystéamine, libérée de son chlorhydrate par de la NMM, permet d'obtenir 8 avec un rendement après traitements de 70%. L'ester actif a été formé par réaction de l'anhydride mixte précédent (Méthode A) avec la N-hydroxysuccinimide dans de l'AcOEt.
Le composé 8, en solution méthanolique et chloroformique, est ensuite S-détritylé par traitement avec un mélange constitué de nitrate d'argent, de pyridine et de méthanol pour fournir le sulfure d'argent correspondant 9. Ce sulfure, qui peut être isolé, est alors mis en suspension dans du CHCl₃ puis est additionné d'HCl (l'usage d'H₂S conduit au même résultat) pour libérer le thiol libre I-152.

Le second procédé de préparation du composé I-152 (Schéma 2) utilise la L-sérine, N-protégée par un t-butoxycarbonyl (Boc) (1), comme produit de départ. 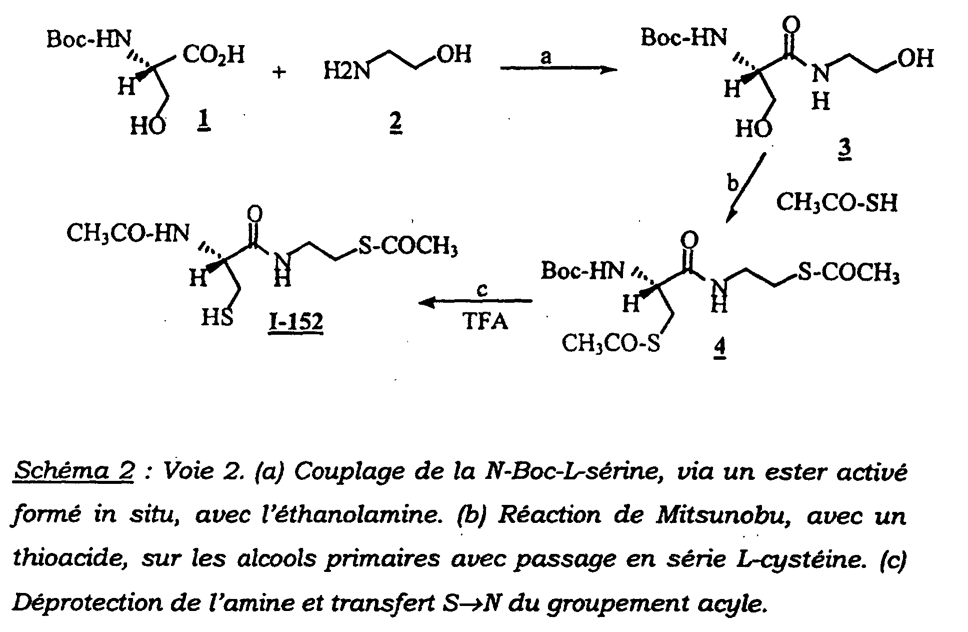 Ce procédé se caractérise en ce qu'il comprend les étapes suivantes (i) d'activation de la fonction carboxylique de la N-Boc-L-sérine (1) par la N-hydroxysuccinimide dans du DMF en présence de DCC; puis (ii) de condensation *in situ* de l'ester actif formé (1') avec l'éthanolamine (2) pour fournir le composé N-(N-Boc-L-séryl)-2-aminoéthanol (3); puis (iii) de traitement, selon une réaction de Mitsunobu, modifiée par R.P. Volante (*Tetrahedron Lett.* 1981, 22, 3119-3122), du N-(N-Boc-L-séryl)-2-aminoéthanol avec de la triphénylphosphine et du diisopropyl azodicarboxylate en présence d'acide thioacétique dans le tétrahydrofuranne (THF) pour fournir le composé N-(N-Boc-S-acétyl-L-cystéinyl)-S-acétylcystéamine (4). Ainsi, le fait de transformer l'alcool de la L-sérine en thioester, tout en conservant la configuration du carbone asymétrique, a permis le passage en série L-cystéine; puis (iv) de déprotection du N-(N-Boc-S-acétyl-L-cystéinyl)-S-acétylcystéamine avec du TFA; la déprotection classique du N-Boc de 4 avec le TFA ne permet pas d'isoler l'amine correspondante formée 5, qui est instable dans nos conditions opérationnelles, mais elle permet de synthétiser le composé I-152 par réaction de transfert intramoléculaire S→N du groupement acétyle de 5 via la thiazoline correspondante 6 (Schéma 3). De tels transferts, en particulier sur la S-acétylcystéamine, ont déjà été observés et étudiés (R.E. Barnett *et coll*. ainsi que les références citées, *J. Amer. Chem. Soc.* 1969, 91, 2358-2369). Ces auteurs montrent que le mécanisme de transfert passe, dans certaines conditions de pH, par la formation d'une thiazoline intermédiaire qui ensuite s'hydrolyse pour générer la N-acétylcystéamine. Nous constatons que la formation de la I-152 fait l'objet du même mécanisme puisque nous avons isolé et identifié l'intermédiaire cyclique 6 qui résulte de la N-déprotection de 4 via 5 (Schéma 3).

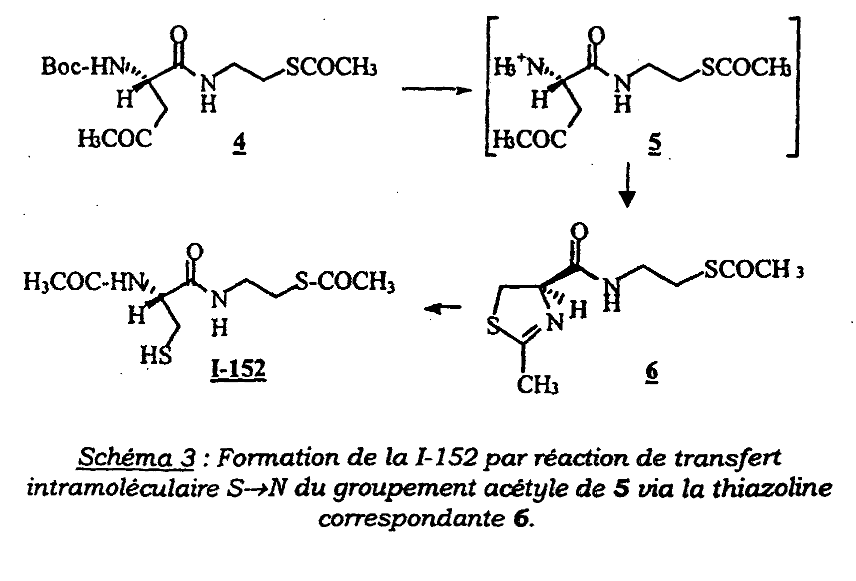

La réaction de transfert S→N du S-acyl, sur le résidu cystéine, permet d'obtenir dans les conditions réactionnelles indiquées le composé I-152.

La présente invention concerne également un procédé de préparation du composé de formule générale I, dans laquelle R, R' sont des groupes méthyles (-CH₃) et R" des groupes acyles; la préparation se fait par S-acylation du composé I-152 en solution dans la pyridine en présence d'un anhydride R₂O ou d'un chlorure d'acide R-Cl caractérisé en ce que R est choisi dans le groupe CO-R¹ dans lequel R¹ est un radical alkyle en C1-C7, linéaire ou ramifié ou un groupe aryle substitué ou non par un ou plusieurs atomes d'halogènes. Ainsi, la préparation du composé I-176 est réalisée par S-acylation du composé I-152, en solution dans la pyridine, avec de l'anhydride acétique. La préparation du composé I-177 est réalisée par S-acylation du composé I-152, en solution dans la pyridine, avec du chlorure d'isobutyryle. La préparation du composé I-178 est réalisée par S-acylation du composé I-152, en solution dans la pyridine, avec du chlorure de pivaloyle.

Plus généralement, c'est un objet de la présente invention de fournir un procédé de préparation d'analogues acylés du composé I-152 ou de ses dérivés (Schéma 4) en utilisant la méthode B du premier mode de préparation du composé selon l'invention décrit dans le schéma 1 voie 1. Ce procédé comprend les étapes de :
a) protection de la N-acétyl-L-cystéine ou de la N-isobutyryl-L-cystéine pour fournir respectivement la N-acétyl-S-trityl-L-cystéine (7) et la N-isobutyryl-S-trityl-L-cystéine (13) ;
b) différents couplages de (7) ou de (13) avec des chlorhydrates de S-acyl-cystéamines pour fournir différents pseudopeptides correspondants. Parmi ceux-ci il convient de citer les composés 10, 11, 12 obtenus à partir de 7, et les composés 14, 15, 16, 17 obtenus à partir de 13.
Alternativement, ce procédé de préparation d'analogues du composé I-152 peut être poursuivi par une étape :
c) de réaction de S-détritylation telle que précédemment décrite lors de la préparation de I-152.
Ainsi, les réactions de S-détritylation des composés 10, 11, 12 sont réalisées pour donner les composés thiols correspondants I-188, I-193, I-198. Ces composés peuvent subir une étape :
d) de S-acylation pour fournir les composés précédemment décrits I-189, I-190, I-191, I-192, I-194, I-195, I-196, I-197, I-199, I-200, I-201, I-202.

Ainsi, la réaction de S-acylation de I-188 permet d'obtenir les composés I-189, I-190, I-191, I-192. Plus particulièrement, la réaction de S-acétylation de I-188 fournit I-189, la réaction de S-isobutyrylation de I-188 fournit I-190, la réaction de S-pivaloylation de I-188 fournit 1-191, la réaction de S-benzoylation de I-188 fournit I-192.

Ainsi, la réaction de S-acylation de I-193 permet d'obtenir les composés I-194, I-195, I-196, I-197. Plus particulièrement, la réaction de S-acétylation de I-193 fournit I-194, la réaction de S-isobutyrylation de I-193 fournit I-195, la réaction de S-pivaloylation de I-193 fournit 1-196, la réaction de S-benzoylation de I-193 fournit I-197.

Ainsi, la réaction de S-acylation de I-198 permet d'obtenir les composés I-199, I-200, I-201, I-202. Plus particulièrement, la réaction de S-acétylation de I-198 fournit I-199, la réaction de S-isobutyrylation de I-198 fournit I-200, la réaction de S-pivaloylation de I-198 fournit 1-201, la réaction de S-benzoylation de I-198 fournit I-202.

Ainsi, les réactions de S-détritylation des composés 14, 15, 16, 17 sont réalisées pour donner les composés thiols correspondants I-203, I-208, I-214, I-219. Ces composés peuvent subir une étape :
d) de S-acylation pour fournir les composés précédemment décrits I-204, I-205, I-206, I-207, I-209, I-210, I-221, I-215, I-216, I-217, I-218

Ainsi, la réaction de S-acylation de I-203 permet d'obtenir les composés I-204, I-205, I-206, I-207. Plus particulièrement, la réaction de S-acétylation de I-203 fournit I-204, la réaction de S-isobutyrylation de I-203 fournit I-205, la réaction de S-pivaloylation de 203 fournit I-206, la réaction de S-benzoylation de I-203 fournit I-207.

Ainsi, la réaction de S-acylation de I-208 permet d'obtenir les composés I-209, I-210, I-211. Plus particulièrement, la réaction de S-acétylation de I-208 fournit I-209, la réaction de S-isobutyrylation de I-208 fournit I-210, la réaction de S-benzoylation de I-208 fournit I-211.

Ainsi, la réaction de S-acylation de I-214 permet d'obtenir les composés I-215, I-216, I-217, I-218. Plus particulièrement, la réaction de S-acétylation de I-214 fournit I-215, la réaction de S-isobutyrylation de I-214 fournit I-216, la réaction de S-pivaloylation de I-214 fournit 1-217, la réaction de S-benzoylation de I-214 fournit I-218.

Ainsi, la réaction de S-acylation de I-219 permet d'obtenir les composés I-220, I-221, I-222, I-223. Plus particulièrement, la réaction de S-acétylation de I-219 fournit I-220, la réaction de S-isobutyrylation de I-219 fournit I-221, la réaction de S-pivaloylation de I-219 fournit 1-222, la réaction de S-benzoylation de I-219 fournit I-223.

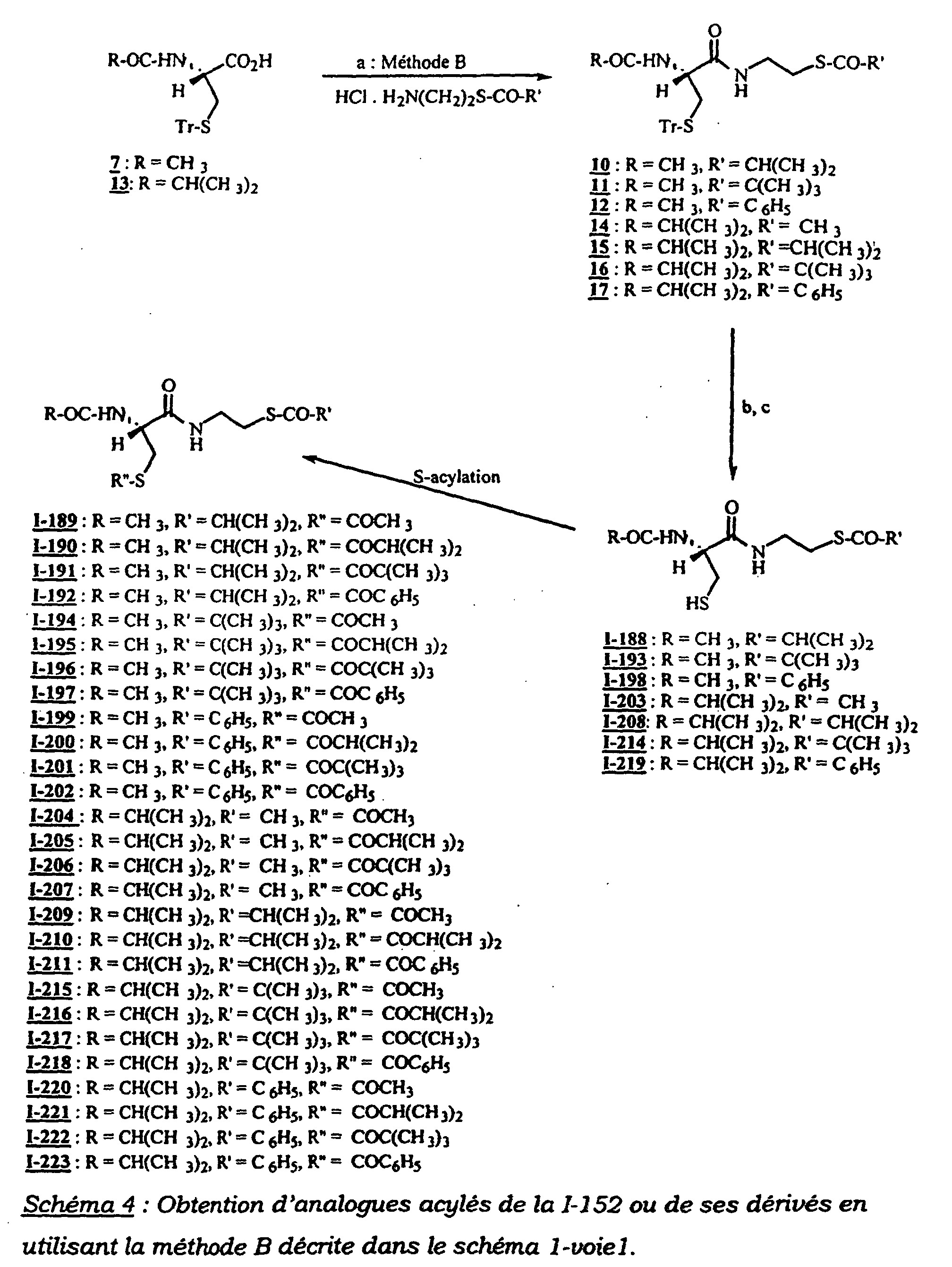

Concernant les composés de l'invention sous forme thiazolidine, ceux-ci sont de préférence obtenus par couplage de N-acyl-S-trityl-L-cystéine avec la thiazolidine selon la méthode A décrite dans le schéma 1 voie 1. L'invention concerne plus particulièrement, les composés I-212 et I-213 qui sont susceptibles d'être obtenus par le procédé décrit au schéma 5. Dans ce cas, la N-acétyl-S-trityl-L-cystéine (7) a été couplée selon la méthode A, schéma 1 : voie 1) avec la thiazolidine pour fomer le composé 18. La S-détritylation de ce dernier, selon le protocole précédemment décrit, notamment pour l'obtention de I-152, permet de générer un thiol libre dénommé I-212. La préparation du composé I-213 est réalisée par S-acétylation de la I-212. Les trois produits de couplage (18, I-212 et I-213) ont été respectectivement isolés sous forme d'un mélange de deux isomères conformationnels. Ces isomères sont dus à la présence du carbonyl, de la liaison pseudopeptidique en alpha de l'atome d'azote de la thiazolidine.

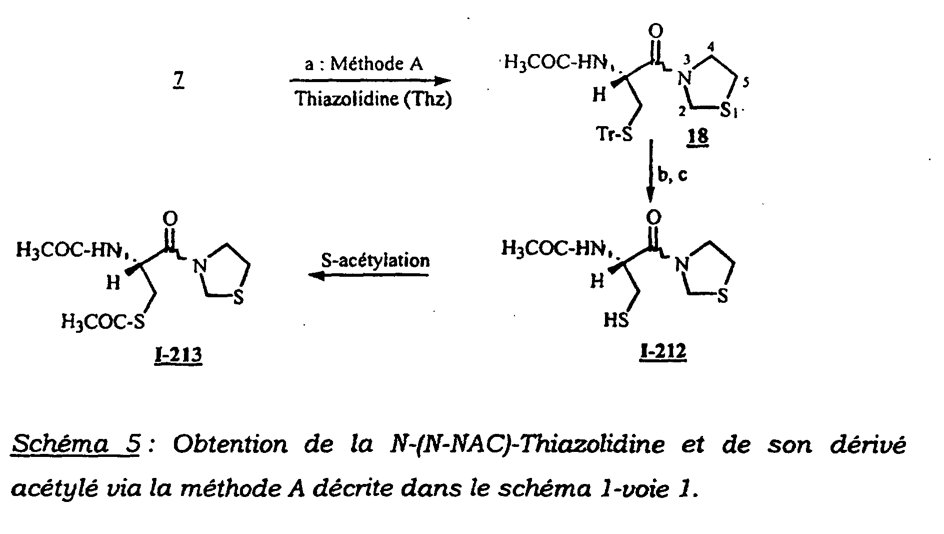

Tous les composés de l'invention présentent une caractéristique commune; il s'agit de précurseurs de composés intervenant dans la voie de biosynthèse du glutathion. En d'autres termes, ces composés peuvent être utilisés comme des intermédiaires intervenant dans la voie de biosynthèse du glutathion. Il peut s'agir par exemple d'un produit choisi dans le groupe formé par la NAC, la MEA, la L-cystéine.

Les composés de l'invention présentent une activité antioxydante. L'invention a donc également pour objet l'utilisation des composés tels que décrits ci-dessus comme agents antioxydants; de tels composés possèdent une large panoplie d'utilisations telles que l'utilisation dans le traitement préventif et curatif de syndromes pathologiques pour lesquels sont observés un stress oxydatif et un déficit en GSH, les utilisations en cosmétologie ou les utilisations dans l'industrie agroalimentaire.

L'invention vise à fournir des agents antioxydants pour lutter contre le stress oxydatif et pour augmenter le taux intracellulaire de glutathion. Les composés de la présente invention peuvent être utilisés, à titre de médicament, en particulier pour augmenter le taux intracellulaire et/ou extracellulaire de glutathion. L'invention couvre également l'utilisation d'un composé selon l'invention pour la préparation d'un médicament destiné à augmenter le taux intracellulaire et/ou extracellulaire de glutathion. La présente invention concerne également une composition pharmaceutique caractérisée en ce qu'elle comprend une quantité efficace d'un composé selon l'invention et un véhicule pharmaceutiquement acceptable. L'invention porte également sur l'utilisation d'un composé selon l'invention pour la préparation d'un médicament ou d'une composition pharmaceutique pour le traitement et/ou la prévention de pathologies ou de troubles liés à une déplétion en glutathion intra et/ou extracellulaire.

Les pathologies qui peuvent faire l'objet d'une prophylaxie ou d'un traitement par les composés de l'invention sont notamment les infections virales, les infections bactériennes, les infections parasitaires, les maladies du tractus respiratoire, les maladies neurodégénératives, les maladies auto-immunes, les maladies cardio-vasculaires, les cancers, les maladies du système immunitaire, le diabète, et de préférence le diabète de type I, les pathologies ophtalmiques, les maladies dermatologiques.

L'invention porte plus particulièrement sur l'utilisation d'un composé tel que décrit précédemment pour la préparation d'un médicament ou d'une composition pharmaceutique pour le traitement et/ou la prévention des infections virales; il s'agit en particulier des infections virales causées par les virus à ADN et les virus à ARN, et plus particulièrement par les virus rétroïdes, plus particulièrement le virus de l'immunodéficience humaine (VIH) et de manière préférée le virus de l'immunodéficience humaine de type 1 (VIH-1). L'infection par le VIH est responsable du syndrome d'immunodéficience acquise (SIDA) qui constitue une pathologie humaine pour laquelle les oxydants jouent un rôle important. Le SIDA constitue un problème de santé publique pour beaucoup de pays dans le monde depuis 1981, date à laquelle la maladie a été pour la première fois identifiée. Lorsque le SIDA est déclaré, la mort survient généralement deux à trois ans après le diagnostic suite à un effondrement des défenses immunitaires du patient et à de multiples infections opportunistes. Au cours de l'infection par le VIH, une diminution des taux cellulaires et plasmatiques des molécules antioxydantes est observée. Ce dérèglement immunitaire baptisé « stress oxydatif » est critique pour le malade. Il semble jouer un rôle majeur dans la physiopathologie des infections par le VIH en augmentant la réplication virale, le syndrome inflammatoire, l'apoptose, la perte de poids des patients (cachexie), et les intoxications médicamenteuses. Si les mécanismes contribuant à ce stress oxydatif sont mal connus, il semble probable que le syndrome inflammatoire chronique associé aux infections à VIH, l'amplifie. De même, le VIH via la protéine Tat semble lui-même jouer un rôle majeur. En effet, cette protéine bloque la production et la sécrétion de la superoxyde dismutase à manganèse (MnSOD), une enzyme susceptible de prévenir le stress oxydatif, et diminue fortement l'activité de la glucose-6-phosphate déshydrogénase (G6PD), une enzyme nécessaire au maintien du glutathion sous sa forme réduite.

Chez les individus infectées par le VIH, les thiols et tout particulièrement le GSH sont diminués dans le plasma, et les cellules mononucléées du sang périphérique (CMSP). Les atteintes sont aussi bien sanguines que tissulaires puisque le déficit en GSH est retrouvé dans les lavages broncho-alvéolaires et dans le système nerveux central (SNC). Ces doubles localisations d'une part confirment que les deux cibles majeures du VIH, les lymphocytes et les macrophages sont touchés, et d'autre part illustrent l'ampleur du déficit. Ceci peut probablement expliquer, tout du moins pour une part, les résultats publiés par L.A. Herzenberg et coll. (*Proc. Natl. Acad. Sci*. 1997, 94, 1967-1972). Ces auteurs montrent l'existence d'un lien direct entre la survie des malades et le taux de GSH.

La thérapeutique antirétrovirale actuelle repose sur deux familles de molécules, les inhibiteurs de la transcriptase inverse (TI), (AZT, ddI, névirapine, etc...) et les inhibiteurs de la protéase virale (indinavir, saquinavir, etc...). Elles sont dotées d'une certaine activité *in vivo* lorsqu'elles sont associées entre elles. Toutefois, ces molécules sont incapables de réorganiser pleinement les atteintes tissulaires telles par exemple les syndromes inflammatoire et oxydatif dans le SNC et ont une efficacité réduite ou nulle vis-à-vis des pathologies associées à l'infection par le VIH. Compte-tenu du rôle majeur du GSH dans le contrôle de ces deux syndromes et de sa pluralité d'effets dans la physiopathologie des infections à VIH, de nombreuses alternatives visant à remonter son taux intracellulaire ont été envisagées sans succès comme stratégie thérapeutique adjuvante au cours des dernières années.

L'expansion continue des infections par le virus VIH et des infections opportunistes associées rend nécessaire de disposer d'un traitement efficace contre le SIDA et les atteintes tissulaires associées. L'un des buts de la présente invention est donc d'utiliser les composés répondant à la formule générale (I), de préférence les composés I-152 et/ou I-176 et/ou I-I77 et/ou I-178, pour la préparation d'un médicament ou d'une composition pharmaceutique pour le traitement et/ou la prévention des infections virales provoquées par le virus de l'immunodéficience humaine (VIH) et plus particulièrement le virus de l'immunodéficience humaine de type 1 (VIH-1). La présente invention fournit également une composition pharmaceutique, pour le traitement préventif et curatif du SIDA et des atteintes tissulaires associées, caractérisée en ce qu'elle contient une quantité thérapeutiquement efficace d'un composé selon l'invention et un véhicule pharmaceutiquement acceptable. La présente invention porte également sur un produit comprenant au moins un composé selon l'invention et au moins un inhibiteur de la transcriptase inverse et/ou comme produit de combinaison pour une utilisation simultanée, séparée ou étalée dans le temps en thérapie antivirale. L'inhibiteur de la transcriptase inverse est choisi par exemple parmi le 3'-azido-3'désoxythymidine (AZT), le 2',3'-didésoxyinosine (ddI), le 2',3'-didésoxycytidine (ddC), le (-)-2',3'-didésoxy-3'-thiacytidine (3TC), le 2',3'-didéshydro-2',3'-didésoxy-thymidine (d4T) et le (-)-2'-désoxy-5-fluoro-3'-thiacytidine (FTC), le TIBO, le HEPT, le TSAO, l'α-APA, la névirapine, le BAHP, l'acide phosphonoformique (PFA). L'inhibiteur de la protéase virale est choisi plus particulièrement parmi l'indinavir et le saquinavir.

Il est également dans l'étendue de l'invention d'utiliser les composés répondant à la formule générale (I), de préférence les composés I-152 et/ou I-176 et/ou I-177 et/ou I-178, pour la préparation d'un médicament ou d'une composition pharmaceutique pour le traitement et/ou la prévention de maladies cardio-vasculaires choisies de préférence parmi le groupe composé de l'hypertension artérielle, de l'artériosclérose, des ischémies cérébrales, des ischémies cardiaques, des arythmies ventriculaires, des fibrillations ventriculaires, de l'infarctus du myocarde. En effet, les patients atteints d'hypertension artérielle traités avec des nitrates organiques développent fréquemment des résistances aux effets de ces drogues. Il a été suggéré que cette tolérance est associée entre autres facteurs à la déplétion en groupes thiols dans les muscles lisses vasculaires. Il a été démontré que des précurseurs métaboliques du glutathion tels la NAC préviennent le développement de la tolérance ou au moins restaurent les effets des nitrates organiques (Abrams 1991, Horowitz 1991, Bosegaard *et al*. 1993). La présente invention se propose donc de fournir de nouveaux composés pour prévenir le développement de la tolérance ou au moins restaurer les effets des nitrates organiques utilisés dans le traitement de l'hypertension artérielle. Heller et al. (1997) ont expérimentalement démontré l'action de différentes espèces réactives de l'oxygène dans l'inflammation des ilots de Langerhans et dans la destruction des cellules β. La présente invention a donc pour objet de fournir de nouveaux composés pour le traitement et la prévention du diabète de type I (IDDM) (Rabinovitch *et al*, 1992).

Le stress oxydatif et le déficit en GSH interviennent également dans d'autres pathologies. Ainsi, dans le domaine de l'ophtalmologie, ils sont à relier avec l'apparition de la cataracte. Il est donc dans l'étendue de l'invention d'utiliser les composés répondant à la formule générale (I), de préférence les composés I-152 et/ou I-176 et/ou I-177 et/ ou I-178, pour la préparation d'un médicament ou d'une composition pharmaceutique pour le traitement et/ou la prévention de pathologies ophtalmiques telles que les atteintes oculaires du syndrome de Sjogren et la cataracte.

Il est également dans l'étendue de l'invention d'utiliser les composés répondant à la formule générale (I), de préférence les composés I-152 et/ou I-176 et/ou I-177 et/ou I-178, pour la préparation d'un médicament ou d'une composition pharmaceutique pour le traitement et/ou la prévention des maladies du tractus respiratoire, notamment l'emphysème pulmonaire, la fibrose pulmonaire idiopathique, la mucoviscidose, la bronchite chronique, la bronchite aiguë, le syndrome de détresse respiratoire de l'adulte.

L'invention porte également sur l'utilisation d'un composé tel que décrit précédemment pour la préparation de médicaments destinés au traitement préventif et/ou curatif des pertes auditives liées au bruit.

L'invention porte également sur l'utilisation d'un composé tel que décrit précédemment pour la préparation de médicaments destinés au traitement des empoisonnements liés à l'administration par voie orale ou parentérale, en surdose ou non, de substances choisies de préférence parmi le groupe composé de l'acétaminophène, les nitrites, l'éthanol, l'acrylonitrile, les métaux lourds et plus particulièrement l'or, l'argent, le mercure.

Les propriétés antioxydantes du composé de l'invention recommandent son utilisation dans le domaine de la cosmétique. En effet, les antioxydants sont déjà utilisés en cosmétologie pour ralentir le vieillissement. Les composés de la présente invention sont capables de promouvoir la reconstruction du contenu cellulaire de GSH et de fournir une protection efficace contre les dommages cellulaires causés par des facteurs toxiques extrinsèques et intrinsèques; la peau est le lieu d'agression de ces facteurs. Les facteurs extrinsèques incluent par exemple les radiations ultraviolettes, le vent, la faible humidité, les abrasifs et les agents tensioactifs forts. Les facteurs intrinsèques incluent le vieillissement chronologique et les modifications biochimiques de la peau. Qu'ils soient extrinsèques ou intrinsèques, ces facteurs provoquent l'apparition de rides et d'autres changements histologiques associés au vieillissement de la peau. Les agents antirides connus à l'heure actuelle incluent des composés tels la N-acétyl-L-cystéine, les rétinoïdes tels que l'acide rétinoïque et les acides alpha-hydroxy tels que l'acide glycolique et l'acide lactique. C'est donc l'un des objets de l'invention d'utiliser les propriétés antioxydantes des composés selon l'invention pour (i) prévenir, effacer et traiter les rides, les ridules de la peau; et/ou (ii) lutter contre le relâchement cutané et/ou sous cutané; et/ou (iii) améliorer la texture de la peau et raviver l'éclat de la peau; et/ou (iv) éliminer les poils indésirés de la peau; et/ou (v) diminuer les tailles des pores de la peau; et/ou (vi) déformer en permanence les cheveux. Concernant ce dernier point, il convient de noter que des molécules organiques porteuses de groupes fonctionnels thiols tels les composés selon l'invention sont des produits ayant de multiples applications. Une de ces applications est la déformation (frisage et défrisage) permanente des cheveux, qui consiste, dans un premier temps, à réaliser l'ouverture des liaisons disulfures (S-S) des unités cystine de la kératine à l'aide d'une composition contenant au moins un composé organique porteur de groupe fonctionnel thiol agissant comme réducteur (étape de réduction) ce qui permet de conférer aux cheveux la forme que l'on souhaite; puis après avoir rincé la chevelure, à reconstituer, dans un second temps, lesdites liaisons disulfures en appliquant, sur les cheveux une composition oxydante (étape d'oxydation, dite aussi de fixation), de façon à fixer les cheveux dans la forme qui leur a été donnée. L'invention concerne donc une composition cosmétique pour le traitement de la peau et/ou des cheveux et/ou des poils caractérisée en ce qu'elle contient un composé selon l'invention et un excipient cosmétiquement acceptable. L'invention porte également sur un procédé de traitement cosmétique de la peau pour prévenir, effacer et traiter les rides, les ridules de la peau et/ou lutter contre le relâchement cutané et/ou sous-cutané et/ou améliorer la texture de la peau et raviver l'éclat de la peau et/ou éliminer les poils indésirés de la peau et/ou diminuer les tailles des pores de la peau comprenant l'application sur la peau d'une composition cosmétique telle que précédemment décrite.

Les propriétés antioxydantes du composé de l'invention recommandent son utilisation dans le domaine agroalimentaire. Il est donc dans la portée de cette invention d'utiliser les composés de l'invention en tant qu'agents antioxydants pour la conservation des propriétés organoleptiques et nutritionnelles des boissons notamment les jus de fruits, et les aliments.

D'autres caractéristiques et avantages de la présente invention seront mieux mis en évidence à la lecture des exemples suivants ; dans ces exemples on se référera aux figures suivantes :
**Figure 1 :** décomposition probable de la I-152.
**Figure 2 :** comparaison des activités antivirales de la NAC, de la MEA et de la I-152 dans des cultures de MDM infectés par 10 000 TCID50 de l'isolat VIH-1/Ba-L : effet-doses. Les résultats sont exprimés selon la moyenne ± écart-type des pourcentages d'inhibition. La réplication virale a été mesurée par le dosage de l'activité transcriptase inverse dans les surnageants de culture.
**Figure 3 :** cytoxicité de la NAC, MEA et I-152 vis-à-vis des MDM.
**Figure 4 :** mesure de l'activité TI et de la production de la protéine p25 dans les surnageants de culture de MDM infectés par la souche VIH-1/Ba-L, et traités par la I-152.
**Figure 5 :** effet de la m.o.i. sur l'activité antivirale de la I-152. Les MDM ont été infectés par 1 000 ou 10 000 TCID50 de l'isolat VIH-1/Ba-L.
**Figure 6 :** Effets de la I-152 sur la réplication virale selon le mode de traitement : pré-traitement 24 heures, traitement 24 heures après l'infection, traitement 7 jours après l'infection.
**Figure 7 :** activité antivirale de la I-152 dans les CMSP quiescentes ou activées par la PHA-P, et infectées par la souche VIH-1-LAI.
**Figure 8 :** activité antivirale de la I-152 dans des LSP quiescents ou activés par la PHA-P, puis infectés par la souche VIH-1-LAI.
**Figure 9 :** effets de la I-152 sur l'intégration du provirus au sein du génome cellulaire.
**Figure 10 :** effets de la I-152 sur l'activité enzymatique de la TI du VIH-1. Les expériences ont été réalisées en triplicat.
**Figure 11 :** dosage du glutathion total intracellulaire dans les CMSP quiescentes, traitées 24 heures avant par la NAC, la MEA ou la I-152.
**Figure 12 :** activité antivirale des dérivés de la I-152 (la I-176 est cytotoxique à la dose testée).
**Figure 13 :** Concentration intracellulaire de GSH dans les MDM infectés ou non *in vitro* par la souche de référence à tropisme macrophagique VIH-1/Ba-L, et traités ou non par le composé I-152.
**Figure 14 :** Effets du composé I-152 sur la concentration intracellulaire de GSH et la synthèse de TNF-α dans les MDM stimulés *in vitro* par un lipopolysacchride bactérien (LPS; 1 µg/mL) et l'IFN-γ (100 UI/mL).
**Figure 15 :** Concentration intracellulaire de GSH dans les macrophages spléniques après traitement ou non par I-152. La concentration intracellulaire de GSH dans les macrophages spléniques humains non-traités est de 22 ± 2 µM.
**Figure 16 :** Concentration intracellulaire de GSH dans les macrophages spléniques infectés ou non *in vitro* par la souche de référence à tropisme macrophagique VIH-1/Ba-L.
**Figure 17** : Effets de I-152 sur l'activité anti-VIH de l'AZT dans les MDM infectés par la souche VIH-1/Ba-L.

**Tableau I :** Comparaison des activités antivirales de la NAC, de la MEA et de la I-152 dans des cultures de MDM infectés par 10 000 TCID50 de l'isolat VIH-1/Ba-L : doses effectrices 50, 70 et 90%.
**Tableau II :** Effets dé la m.o.i. sur l'activité antivirale de la I-152 : doses effectrices 50%.
**Tableau III** : Activité antivirale de la I-152 dans les MDM spléniques infectés par 10 000 TCID50 de l'isolat VIH-1/Ba-L : doses effectrices 50, 70 et 90%.
**Tableau IV :** Comparaison des activités antivirales de la I-152 et de ses analogues S-acylés dans des cultures de MDM infectés par 10 000 TCID50 de l'isolat VIH-1/Ba-L : doses effectrices 50, 70 et 90%.
**Tableau V :** Comparaison des activités antivirales de la I-152 et de ses dérivés (N-isobutyryl) diversement S-acylés dans des cultures de MDM infectés par 10 000 TCID50 de l'isolat VIH-1/Ba-L : doses effectrices 50, 70 et 90%.

### EXEMPLE 1: SYNTHESE DE LA N-(N-ACETYL-L-CYSTEINYL)-S-ACETYLCYSTEAMINE (I-152)

### 1.1. Première voie de synthèse de la I-152 utilisant la L-cystéine S, N protégée :

### 1.1.1. N-(N-ACETYL-S-TRITYL-L-CYSTEINYL)-S-ACETYLCYSTEAMINE (8)

### a)- Méthode de couplage faisant intervenir in situ un anhydride mixte

Une solution contenant 290 mg (0,71 mmole) de N-acétyl-S-trityl-L-cystéine (**7,** Bachem) et 80 µL (0,72 mmole) de N-méthylmorpholine (NMM) dans 5 ml d'AcOEt est agitée à -15°C puis additionnée de 93 µl (0,71 mmole) de chloroformiate d'isobutyle. Après 15 min d'agitation et en maintenant la température initiale, on ajoute 111,4 mg (0,71 mmole) de chlorhydrate de S-acétylcystéamine (préparé selon T. Wieland et E. Bokelman, *Ann. Chem*., 1952, 576, 20-34) puis 80 µl (0,72 mmole) de NMM. Le mélange réactionnel est maintenu 15 min à -15°C puis, après retour à la température ambiante, on poursuit l'agitation durant 3 h. Le chlorhydrate de NMM formé est filtré et lavé avec 2 x 2,5 ml d'AcOEt et les phases organiques rassemblées sont évaporées à sec sous vide. Le produit de couplage est ensuite isolé de la gomme obtenue par « flash » chromatographie sur colonne de gel de silice (éluant : AcOEt/éther de pétrole 30%). On recueille 198 mg (Rdt = 55%) du composé attendu. R_{f} (AcOEt/éther de pétrole, 9 : 1) : 0,41. Cristallise dans un mélange d' AcOEt/éther de pétrole sous forme d'une poudre incolore. F = 111-113°C. [α]_{D}²⁰ = +10,5° (c 0,8; CHCl₃).
RMN ¹H (CDCl₃) δ ppm : 1,90 (s, 3H, NCOC*H*_{*3*}); 2,29 (s, 3H, SCOC*H*_{*3*}); 2,48 (dd, J = 5,7; 12,9 Hz, 1H, β Ha cys); 2,82 (dd, J = 6,4; 12,9 Hz, 1H, β *H*b cys); 2,92-3,01 (m, 2H, NCH₂C*H*_{*2*}S); 3,32-3,42 (m, 2H, NC*H*_{*2*}CH₂S); 4,07-4,20 (m, 1H, α *H* cys); 5,70 (d, J = 7,6 Hz, 1H, N*H* cys); 6,34 (t, J = 5,5 Hz, 1H, N*H*CH₂); 7,19-7,35 et 7,40-7,47 (2m, 15H, *H* aromatiques).
SM : (FAB⁺/NBA+K⁺) m/z 545 (M+K)⁺, 507 (M+H)⁺; (FAB⁻/NBA) *m*/*z* 505 (M-H)-.

| Analyse : C₂₈H₃₀N₂O₃S₂ (506) | | | |
|---|---|---|---|
| Calc. % | C 66,40 | H 5,93 | N 5,53 |
| Tr. % | 66,17 | 6,00 | 5,81 |

### b)- Méthode de couplage faisant intervenir in situ un ester activé

Une solution contenant 1,5 g (3,70 mmoles) de **7** et 410 µL (3,73 mmoles) de NMM dans 30 ml d'AcOEt est agitée à -15°C puis additionnée de 480 µl (3,70 mmoles) de chloroformiate d'isobutyle. Après 15 min d'agitation et en maintenant la température initiale, on ajoute 426 mg (3,70 mmoles) de N-hydroxysuccinimide. Le mélange réactionnel est maintenu 15 min à -15°C puis, après retour à la température ambiante, on poursuit l'agitation durant 2 h. Le chlorhydrate de NMM formé est filtré et lavé avec 2 x 5 ml d'AcOEt. Les phases organiques contenant l'ester actif O-N-succinimide de 7 sont rassemblées et agitées à -15°C. La solution est alors additionnée successivement de 575 mg (3,70 mmoles) de chlorhydrate de S-acétylcystéamine et de 410 µL (3,73 mmoles) de NMM. Le mélange réactionnel est maintenu 15 min à -15°C puis, après retour à la température ambiante, on poursuit l'agitation durant 12 h. La solution est ensuite diluée avec 300 ml d'AcOEt, lavée (eau, 30 ml; bicarbonate de sodium saturé et glacé, 30 ml; eau, 30 ml; acide citrique 0,1N glacé, 30 ml; eau, 3 x 30 ml), séchée sur sulfate de sodium, filtrée et évaporée à sec sous vide. Le résidu obtenu est ensuite purifié comme ci-dessus pour donner, avec un rendement de 70% (1,31 g) et avec rigoureusement les mêmes critères physico-chimiques, le produit de couplage **8** précédemment décrit.

### 1.1.2. N-(N-ACETYL-L-CYSTEINYL)-S-ACETYLCYSTEAMINE (I-152)

Une solution, saturée et protégée de la lumière, de 1,26 g (2,49 mmoles) de **8** dans 20 ml de MeOH et 1,5 ml de CHCl₃ est agitée à la température ambiante et additionnée d'un mélange, également protégé de la lumière, contenant 449 mg (2,64 mmoles) de nitrate d'argent et 213 µl (2,64 mmoles) de pyridine dans 13 ml de MeOH. Instantanément, il y a formation d'un précipité du sulfure d'argent correspondant **9**. A la fin de l'ajout, l'agitation est arrêtée et le mélange réactionnel est abandonné une nuit à la température ambiante. Le précipité est ensuite filtré et lavé avec du MeOH (2 x 10 ml) puis avec du CHCl₃ (2 x 10 ml).

Un échantillon analytique de **9** est prélevé puis séché sous vide à l'abri de la lumière.

| Analyse : C₉H₁₅N₂O₃S₂Ag (371) | |
|---|---|
| Calc. % | Ag 29,11 |
| Tr. % | 29,16 |

Le sulfure précédent **9** est mis en suspension dans 15 ml de CHCl₃ et agité à la température ambiante et à l'abri de la lumière, puis additionné de 400 µl d'acide chlorhydrique concentré. L'agitation est poursuivie 2 h à la température ambiante puis 2 min à 30-35 °C. Le mélange est alors dilué avec 70 ml de CHCl₃ et le chlorure d'argent formé est filtré puis lavé avec 3 x 10 ml du même solvant. Les phases organiques sont rassemblées, lavées rapidement à l'eau glacée (3 x 10 ml), séchées sur sulfate de sodium, filtrées et évaporées à sec sous vide. On recueille une pâte semi-cristalline qui cristallise dans un mélange d' AcOEt/éther de pétrole sous forme de microcristaux incolores (368 mg, Rdt = 56%). F = 121-122°C. [α]_{D}²⁰ = -39,1° (c 0,9; CHCl₃). Les autres données (microanalyses et spectres) sont en tous points identiques à celles décrites dans la deuxième voie de synthèse.
L'H₂S a aussi été utilisé et conduit au même résultat.

### 1.2. Deuxième voie de synthèse de la I-152 utilisant la L-sérine N-protégée:

### 1.2.1. N-(N-Boc-L-SERYL)-2-AMINOETHANOL (3)

Une solution contenant 6,15 g (30 mmoles) de N-Boc-L-sérine (1, Fluka) et 3,45 g de N-hydroxysuccinimide (30 mmoles) dans 80 ml de DMF est agitée à 0 °C et additionnée de 6,2 g (30 mmoles) de DCC. Le mélange réactionnel est maintenu 15 min à 0 °C puis on le laisse revenir à la température ambiante et on poursuit l'agitation durant 1 h 30. On ajoute ensuite 2,75 ml (60 mmoles) d'éthanolamine . Après 12 h d'agitation, la DCU formée est filtrée et lavée avec 2 x 15 ml de DMF. Les phases organiques rassemblées sont évaporées à sec sous vide. Le produit est isolé de la pâte résiduelle par « flash » chromatographie sur colonne de gel de silice (Kieselgel Merck 60, 230-400 mesh; éluant : CH₂Cl₂/MeOH 6%,). On recueille le composé attendu sous forme d'une gomme qui cristallise dans un mélange d'AcOEt/hexane pour fournir 5,21 g (Rdt = 70%) d'aiguilles incolores. R_{f} (CH₂Cl₂/MeOH, 9,3 : 0,7) : 0,23; (CH₂Cl₂/MeOH/ACOH, 9 : 0,9 : 0,1) : 0,47. F = 74-76°C. [α]_{D}²⁰ = -2,2° (c 0,9; CHCl₃).
RMN ¹H (DMSO-*d*_{*6*}) δ ppm : 1,50 (s, 9H, *H t*-butyl); 3,18-3,29 (m, 2H, NC*H*_{*2*}CH₂O); 3,45-3,55 (m, 2H, NCH₂C*H*_{*2*}O); 3,57-3,70 (m, 2H, β C*H*_{*2*} ser); 4,01-4,10 (m, 1H, α *H* ser); 4,77 (t, J = 5,4 Hz, 1H, O*H* ser); 4,91 (t, J = 5,7 Hz, 1H, NCH₂CH₂O*H*); 6,71 (d, J = 8,2 Hz, 1H, N*H* ser); 7,86 (t, J = 5,5 Hz, 1H, N*H*CH₂).
SM : (FAB⁺/G-T) *m*/*z* 745 (3M+H)⁺, 497 (2M+H)⁺, 249 (M+H)⁺.

| Analyse : C₁₀H₂₀N₂O₅ (248). | | | |
|---|---|---|---|
| Calc. % | C 48,39 | H 8,06 | N 11,29 |
| Tr. % | 8,57 | 8,08 | 11,08 |

### 1.2. 2. N-(N-Boc-S-ACETYL-L-CYSTEINYL)-S-ACETYLCYSTEAMINE (4)

Une solution contenant 2,597 g (9,91 mmoles) de triphénylphosphine et 1,95 ml (9,91 mmoles) de diisopropyl azodicarboxylate dans 15 ml de THF est agitée 30 min à 0 °C (au bout de 30 s d'agitation, on observe la formation d'un intense précipité). En conservant cette température, on additionne successivement 1,116 g (4,50 mmoles) de **3** en solution dans 6 ml de THF puis 707 µl (9,91 mmoles) d'acide thioacétique. On laisse ensuite la solution obtenue revenir à la température ambiante et on poursuit l'agitation durant 12 h. Le mélange réactionnel est alors évaporé à sec sous vide. Le produit est isolé de la pâte résiduelle par « flash » chromatographie sur colonne de gel de silice (éluant : hexane puis AcOEt/éther de pétrole 75%). On recueille le composé attendu sous forme d'une gomme qui cristallise dans un mélange d'AcOEt/éther de pétrole sous forme d'aiguilles incolores (1,21 g; Rdt = 74%). R_{f} (AcOEt/éther de pétrole, 4 : 6) : 0,35. F = 111-113 °C. [α]_{D}²⁰ = -13,9° (c 0,86; CHCl₃).
RMN ¹H (CDCl₃) δ ppm : 1,45 (s, 9H, *H t*-butyl); 2,36, 2,38 (2s, 2 x 3H, 2 x SCOC*H*_{*3*}); 2,99-3,07 ( m, 2H, NCH₂C*H*_{*2*}S); 3,19 (dd, J = 7,8; 14,3 Hz, 1H, β *H*a cys); 3,34 (dd, J = 4,5; 14,3 Hz, 1H, β *H*b cys); 3,40-3,50 (m, 2H, NC*H*_{*2*}CH₂S); 4,19-4,34 (m, 1H, a *H* cys); 5,25 (d, J = 7,1 Hz, 1H, N*H* cys); 6,69 (t, J = 5,2 Hz, 1H, N*H*CH₂).
SM : (FAB⁺/NBA) *m*/*z* 729 (2M+H)⁺, 365 (M+H)⁺.

| Analyse : C₁₄H₂₄N₂O₅S₂ (364) | | | |
|---|---|---|---|
| Calc. % | C 46,15 | H 6,59 | N 7,69 |
| Tr. % | 46,45 | 6,51 | 7,47 |

### 1.2.3. N-(N-ACETYL-L-CYSTEINYL)-S-ACETYLCYSTEAMINE (I-152)

Une solution contenant 500 mg (1,37 mmole) de **4** dans 5 ml de CH₂Cl₂ est agitée, sous argon à 0 °C, puis additionnée de 1 ml (13,07 mmoles) de TFA. On laisse ensuite la solution revenir à la température ambiante et on poursuit l'agitation durant 7 h. A ce stade, la réaction contrôlée par CCM (CH₂Cl₂/MeOH, 9,4: 0,6) montre la disparition du composé de départ (Rf : 0,75) et l'apparition de deux spots plus polaires (R_{f} : 0,5 et 0,16). Le mélange réactionnel est évaporé à sec sous vide (température du bain-marie : <40 °C).

Un nouveau contrôle par CCM, de la gomme obtenue, indique que le spot majoritaire précédent à R_{f} : 0,16 est devenu minoritaire au profit de celui à Rf : 0,5. On note également l'apparition d'un troisième spot, de moindre importance, à R_{f} : 0,4. A ce stade, nous avons réalisé une « flash » chromatographie sur une aliquote de la gomme ( 20 mg) afin d'identifier les composés présents pour élucider ce phénomène:
- Avec un mélange d'éluants constitué de CH₂Cl₂ /Et₂O (5 : 5), on isole le produit de Rf : 0,5. L'étude de ses spectres (RMN ¹H et SM) montre, sans ambiguïté, qu'il s'agit de la N-(2-méthyl-Δ²-thiazolinyl-4(R)-carbonyl)-S-acétylcystéamine **6 :**
   RMN ¹H (CDCl₃) δ ppm : 2,32 (s, 3H, SCOC*H*_{*3*}); 2,39 (d, J = 1,3 Hz, 3H, 2-C*H*_{*3*} thiazoline); 3,05 (t app., J = 6,4 Hz, 2H, NCH₂C*H*_{*2*}S); 3,36-3,60 (m, 2H, NC*H*_{*2*}CH₂S); 3,69 (dd, J = 10,2; 11,4 Hz, 1H, 5-H thiazoline); 3,85 (dd, J = 7,2; 11,4 Hz, 1H, 5'-*H* thiazoline); 5,08 (ddd, J = 1,3; 7,2; 10,2 Hz, 1H, 4-*H* thiazoline); 7,45-7,58 (m, 1H, N*H*CH2).
   SM : (FAB⁺/G-T) *m*/*z* 493 (2M+H)⁺, 247 (M+H)+.
- En augmentant la polarité du solvant d'élution (CH₂Cl₂/MeOH, 9,5 : 0,5), on isole le composé intermédiaire (Rf : 0,4). L'étude de ses spectres (RMN ¹H et SM) montre qu'il s'agit de la I-152. Ses données physico-chimiques sont rapportées à la fin de cette description.
- La poursuite de la chromatographie avec des éluants plus polaires (CH₂Cl₂/MeOH 5-20%) n'a pas permis d'obtenir le produit de R_{f} : 0,16. Ce composé qui est le premier formé dans la réaction de déprotection de la fonction amine terminale du N-Boc de départ ne peut être que le trifluoroacétate de la N-(S-acétyl-L-cystéinyl)-S-acétyleystéamine **5.**
   * Tous les essais réalisés et contrôlés par CCM nous ont montré, dans nos conditions opérationnelles (temps nécessaire à la consommation totale du produit de départ, température et pH du milieu réactionnel), que **5,** qui est le premier formé lors de déprotection du N-Boc par le TFA, génère l'intermédiaire cyclique **6** qui s' hydrolyse ensuite lentement pour donner la I-152.

Ainsi, après ces différentes constatations, la gomme restante obtenue après évaporation du brut réactionnel a été solubilisée dans 150 ml de CH₂Cl₂ puis additonnée, à la température ambiante et sous forte agitation, de 5 ml d'eau. Après 6 h d'agitation, le contrôle par CCM ne montre plus qu'un seul spot correspondant au produit désiré. La phase organique est décantée et l'eau résiduelle est extraite avec du CH₂Cl₂ (3 x 10 ml). Les phases organiques sont ensuite rassemblées, séchées sur sulfate de sodium, filtrées et évaporées à sec sous vide. On recueille le composé attendu sous forme d'une pâte semi-cristalline. R_{f} (CH₂Cl₂/MeOH, 9,5 : 0,5) : 0,4. Cristallise dans un mélange d'AcOEt/éther de pétrole sous forme de microcristaux incolores (245 mg, Rdt = ≥67%). F = 122-124°C. [α]_{D}²⁰ = -40° (c 0,87; CHCl₃).
RMN ¹H (CDCl₃) δ ppm : 1,60 (dd, J = 7,6; 10,3 Hz, 1H, S*H*); 2,07 (s, 3H, NCOC*H*_{*3*}); 2,36 (s, 3H, SCOC*H*_{*3*}); 2,70 (ddd, J = 6,5; 10,3; 13,9 Hz, 1 H, β Ha cys); 3,03 (t, J = 6,3 Hz, 2H, NCH₂C*H*_{*2*}S); 3,06 (ddd, J = 4,3; 7,6; 13,9 Hz, 1H, β *H*b cys); 3,46 (td, J = 6,0; 6,3 Hz, 2H, NC*H*_{*2*}CH₂S); 4,59 (ddd, J = 4,3; 6,5; 7,9 Hz, 1H, α *H* cys); 6,52 (d, J = 7,9 Hz, 1H, N*H* cys); 6,75-6,90 (m, 1H, N*H*CH₂).
SM : (FAB⁺/G-T) *m*/*z* 529 (2M+H)⁺, 265 (M+H)⁺.

| Analyse : C₉H₁₆N₂O₃S₂ (264) | | | | |
|---|---|---|---|---|
| Calc. % | C 40,91 | H 6,06 | N 10,61 | S 24,24 |
| Tr. % | 41,21 | 6,00 | 10,91 | 23,99 |

### EXEMPLE 2: SYNTHESE DE DERIVES ACYLES DE LA N-(N-ACETYL-L-CYSTEINYL)-S-ACETYLCYSTEAMINE (I-152) OU DE SES DERIVES

### 2.1. N-(N,S-BIS-ACETYL-L-CYSTEINYL)-S-ACETYLCYSTEAMINE (I-176) (Méthode générale de S-acylation)

Une solution contenant 83 mg (0,31 mmole) de I-152 dans 1 ml de pyridine est agitée à 0 °C et additionnée de 90 µl (0,95 mmole) d'anhydride acétique. Le mélange réactionnel est maintenu 15 min à 0 °C puis on le laisse revenir à la température ambiante et on poursuit l'agitation durant 12 h. La solution est ensuite évaporée à sec sous vide et le résidu formé est repris avec 30 ml de CH₂Cl₂. La phase organique est lavée à l'eau (3 x 20 ml), séchée sur sulfate de sodium, filtrée et évaporée à sec sous vide. La gomme résiduelle est cristallisée dans de l'AcOEt et fournit 73 mg (Rdt = 75%) du composé attendu sous forme de plaquettes incolores. R_{f} (CH₂Cl₂/MeOH, 9,5 : 0,5) : 0,46. F = 153-154 °C, [α]_{D}²⁰ = -33,7° (c 0,8; CHCl₃).
RMN ¹H (CDCl₃) δ ppm : 2,02 (s, 3H, NCOC*H*_{*3*}); 2,37, 2,39 (2s, 2 x 3H, 2 x SCOC*H*_{*3*}); 2,93, 3,12 (m, 2H, NCH₂C*H*_{*2*}S); 3,24 (dd, J = 7,2; 14,5 Hz, 1H, β *H*a cys); 3,31 (dd, J = 5,2; 14,5 Hz, 1H, β *H*b cys); 3,39-3,49 (m, 2H, NC*H*_{*2*}CH₂S); 4,54 (ddd, J = 5,3; 7,2; 7,3 Hz, 1H, a *H* cys); 6,44 (d, J = 7,3 Hz, 1H, N*H* cys); 6,83 (t, J = 5,1 Hz,1H, N*H*CH₂).
SM : (FAB⁺/G-T) *m*/*z* 613 (2M+H)⁺, 307 (M+H)⁺.

| Analyse : C₁₁H₁₈N₂O₄S₂ (306) | | | | |
|---|---|---|---|---|
| Calc. % | C 43,14 | H 5,88 | N 9,15 | S 20,92 |
| Tr. % | 42,95 | 5,96 | 8,93 | 20,64 |

### 2.2. N-(N-ACETYL-S-ISOBUTYRYL-L-CYSTEINYL)-S-ACETYL CYSTEAMINE (I-177)

La réaction d'acylation de 85 mg (0,32 mmole) de **I-152** est réalisée, selon la méthode générale précédemment décrite, en utilisant 137 µl (1,30 mmole) de chlorure d'isobutyryle à la place de l'anhydride acétique. Le mélange visqueux obtenu, après évaporation à sec sous vide, est ensuite dilué avec 30ml de CH₂Cl₂. La solution est ensuite lavée (eau, 10 ml; bicarbonate de sodium saturé et glacé, 10 ml; eau, 10 ml; acide citrique 0,1N glacé, 10 ml; eau, 3 x 10 ml), séchée sur sulfate de sodium, filtrée et évaporée à sec sous vide. Le produit d'acylation est isolé de la gomme obtenue par « flash " chromatographie sur colonne de gel de silice (éluant : CH₂Cl₂/éther 50%). On recueille 60 mg (Rdt = 56%) du composé attendu. R_{f} (CH₂Cl₂/MeOH, 9,4 : 0,6) : 0,6. Cristallise dans un mélange d' AcOEt/éther de pétrole sous forme de plaquettes incolores. F = 116-118 °C. [α]_{D}²⁰ = -18,4° (c 0,87; CHCl₃).
RMN ¹H (CDCl₃) δ ppm : 1,20 (d, J = 6,9 Hz, 6H, C(C*H*_{*3*})_{*2*}; 2,00 (s, 3H, NCOC*H*_{*3*}); 2,37 (s, 3H, SCOC*H*_{*3*}); 2,80 (hept, J = 6,9 Hz, 1H, C*H*(CH₃)₂; 2,93, 3,12 (m, 2H, NCH₂C*H*_{*2*}S); 3,23-3,30 (m, 2H, β C*H*_{*2*} cys); 3,38-3,49 (m, 2H, NC*H*_{*2*}CH₂S); 4,46-4,57 (m, 1H, α *H* cys); 6,42 (d, J = 7,3 Hz, 1H, N*H* cys); 6,80 (t, J = 5,2 Hz, 1H, N*H*CH₂).
SM : (FAB⁺/G-T) *m*/*z* 669 (2M+H)⁺, 335 (M+H)⁺.

| Analyse : C₁₃H₂₂N₂O₄S₂ (334) | | | | |
|---|---|---|---|---|
| Calc. % | C 46,71 | H 6,59 | N 8,38 | S 19,16 |
| Tr. % | 46,76 | 6,89 | 8,24 | 19,32 |

### 2.3. N-(N-ACETYL-S-PIVALOYL-L-CYSTEINYL)-S-ACETYLCYSTEAMINE (I-178)

La réaction d'acylation de 95 mg (0,36 mmole) de **I-152** est réalisée, selon la méthode générale précédemment décrite, en utilisant 176 µl (1,44 mmole) de chlorure de pivaloyle à la place de l'anhydride acétique. Le mélange visqueux obtenu, après évaporation à sec sous vide, est ensuite dilué avec 30 ml de CH₂Cl₂. La solution est ensuite lavée (eau, 10 ml; bicarbonate de sodium saturé et glacé, 10 ml; eau, 10 ml; acide citrique 0,1N glacé, 10 ml; eau, 3 x 10 ml), séchée sur sulfate de sodium, filtrée et évaporée à sec sous vide. Le produit d'acylation est isolé de la gomme obtenue par « flash » chromatographie sur colonne de gel de silice (éluant : CH₂Cl₂/éther 50%). On recueille 70 mg (Rdt = 56%) du composé attendu. R_{f} (CH₂Cl₂/éther, 4 : 6) : 0,23. Cristallise dans un mélange d' AcOEt/éther de pétrole sous forme de plaquettes incolores. F = 92-94 °C. [α]_{D}²⁰ = -11,1° (c 1,08; CHCl₃).
RMN ¹H (CDCl₃) δ ppm : 1,25 (s, 9H, C(C*H*_{*3*})_{*3*}; 2,00 (s, 3H, NCOC*H*_{*3*}); 2,37 (s, 3H, SCOC*H*_{*3*}); 2,94, 3,12 (m, 2H, NCH₂C*H*_{*2*}S); 3,25 (d, J = 6,5 Hz, 2H, β *CH*_{*2*} cys); 3,38-3,49 (m, 2H, NC*H*_{*2*}CH₂S); 4,51 (td, J = 6,5, 7,3 Hz, 1H, α *H* cys); 6,42 (d, J = 7,3 Hz, 1H, N*H* cys); 6,80 (t, J = 5,2 Hz, 1H, N*H*CH₂).
SM : (FAB⁺/G-T) m/z 697 (2M+H)⁺, 349 (M+H)⁺.

| Analyse : C₁₄H₂₄N₂O₄S₂ (348) | | | | |
|---|---|---|---|---|
| Calc. % | C 48,28 | H 6,90 | N 8,05 | S 18,39 |
| Tr. % | 48,32 | 6,95 | 7,94 | 18,43 |

### 2.4. N-(N-ACETYL-S-TRITYL-L-CYSTEINYL)-S-ISOBUTYRYLCYSTEAMINE (10)

La réaction de couplage de **7** (4,5 mmoles) avec le chlorhydrate de S-isobutyrylcystéamine [(composé obtenu selon les mêmes méthodes que celles décrites par T. Wieland et E. Bokelman, *Ann. Chem*., 1952, 576, 20-34; lors des synthèses des chlorhydrates des S-acétyl et S-benzoylcystéamines) F = 147-148°C] est réalisée selon la méthode B décrite dans la première voie de synthèse (exemple 1). Après les différents traitements, le composé attendu est isolé par " flash " chromatographie sur colonne de gel de silice (éluant : CH₂Cl₂/éther 30%). On recueille **10,** sous forme d'une mousse incolore, avec un rendement de 80%. R_{f} (AcOEt/éther de pétrole, 8 : 2) : 0,37. [α]_{D}²⁰ = +10° (c 1,1; CHCl₃).
RMN ¹H (CDCl₃) δ ppm : 1,16 (d, J = 6,9 Hz, 6H, C(C*H*₃)_{*2*}); 1,90 (s, 3H, NCOC*H*_{*3*}); 2,49 (dd, J = 5,7; 12,9 Hz, 1H, β Ha cys); 2,70 (hept, J = 6,9 Hz, 1H, C*H*(CH₃)₂); 2,79 (dd, J = 6,4; 12,9 Hz, 1H, β *H*b cys); 2,88-3,01 (m, 2H, NCH₂C*H*_{*2*}S); 3,29-3,41 (m, 2H, NC*H*_{*2*}CH₂S); 4,08-4,19 (m, 1H, α *H* cys); 5,76 (d, J = 7,7 Hz, 1H, N*H* cys); 6,36 (t, J = 5,5 Hz, 1H, N*H*CH₂); 7,15-7,35, 7,38-7,52 (2m, 15H, H aromatiques).
SM : (FAB⁺/G-T) *m*/*z* 535 (M+H)⁺.

| Analyse : C₃₀H₃₄N₂O₃S₂ (534) | | | |
|---|---|---|---|
| Calc. % : | C 67,42 | H 6,37 | N 5,24 |
| Tr. % : | 67,25 | 6,58 | 5,30 |

### 2.5. N-(N-ACETYL-L-CYSTEINYL)-S-ISOBUTYRYLCYSTEAMINE (I-188)

Ce composé est obtenu par la S-détritylation de **10** (2,38 mmoles). Le protocole utilisé est le même que celui décrit dans l'exemple 1 pour la synthèse de la **I-152**. Après les différents traitements, on recueille une gomme qui est purifiée par " flash " chromatographie sur colonne de gel de silice (éluant : CH₂Cl₂/MeOH 1,5%). On isole la **I-188,** sous forme d'une gomme semi-critalline incolore, avec un rendement de 57%. R_{f} (CH₂Cl₂/MeOH, 9,5 : 0,5) : 0,45. [α]_{D}²⁰ = -26,6° (c 1,09; CHCl₃).
RMN ¹H (CDCl₃) δ ppm : 1,21 (d, J = 6,9 Hz, 6H, C(C*H*₃)_{*2*}); 1,61 (dd, J = 7,6; 10,3 Hz, 1H, S*H*); 2,09 (s, 3H, NCOC*H*_{*3*}); 2,70 (ddd, J = 6,4; 10,3; 13,8 Hz, 1H, β *H*a cys); 2,77 (hept, J = 6,9 Hz, 1H, C*H*(CH₃)₂); 2,99-3,08 (m, 2H, NCH₂C*H*_{*2*}S); 3,09 (ddd, J = 4,1; 7,6; 13,8 Hz, 1H, β *H*b cys); 3,41-3,53 (m, 2H, NC*H*_{*2*}CH₂S); 4,60 (ddd, J = 4,1; 6,4; 7,5 Hz, 1H, α *H* cys); 6,48 (d, J = 7,5 Hz, 1H, N*H* cys); 6,68-6,88 (m, 1H, N*H*CH₂).
SM : (FAB⁺/G-T) *m*/*z* 585 (2M+H)⁺, 293 (M+H)⁺.

| Analyse : C₁₁H₂₀N₂O₃S₂ (292) | | | |
|---|---|---|---|
| Calc. % | C 45,20 | H 6,85 | N 9,59 |
| Tr. % | 45,31 | 7,09 | 9,41 |

### 2. 6. N-(N,S-BIS-ACETYL-L-CYSTEINYL)-S-ISOBUTYRYLCYSTEAMINE (I-189)

La S-acylation de la **I-188** (0,32 mmole), avec de l'anhydride acétique, est réalisée selon la méthode générale décrite dans l'exemple 2. Le mélange réactionnel est ensuite traité suivant le protocole décrit pour la synthèse de la **I-177.** Après les différents traitements, on recueille une gomme qui est purifiée par " flash " chromatographie sur colonne de gel de silice (éluant : CH₂Cl₂/éther 55%). On isole la **I-189** sous forme d'une gomme (Rdt = 64%) qui, après trituration dans de l'hexane, fournit une poudre incolore. R_{f} (CH₂Cl₂/MeOH, 9,5 : 0,5) : 0,58. F = 115-117°C. [α]_{D}²⁰ = -20,2° (c 1,04; CHCl₃).
RMN ¹H (CDCl₃) δ ppm : 1,20 (d, J = 6,9 Hz, 6H, C(C*H*₃)_{*2*}); 2,02 (s, 3H, NCOC*H*_{*3*}); 2,38 (s, 3H, SCOC*H*₃); 2,78 (hept, J = 6,9 Hz, 1H, C*H*(CH₃)₂); 2,96-3,06 (m, 2H, NCH₂C*H*_{*2*}S); 3,19-3,36 (m, 2H, C*H*_{*2*} cys); 3,38-3,48 (m, 2H, NC*H*_{*2*}CH₂S); 4,47-4,60 (m, 1H, α *H* cys); 6,37 (d, J = 7,1 Hz, 1H, N*H*cys); 6,70-6,83 (m, 1H, N*H*CH₂).
SM : (FAB⁺/G-T) *m*/*z* 669 (2M+H)⁺, 335 (M+H)⁺.

| Analyse : C₁₃H₂₂N₂O₄S₂ (334) | | | |
|---|---|---|---|
| Calc. % | C 46,71 | H 6,59 | N 8,38 |
| Tr. % | 46,81 | 6,62 | 8,36 |

### 2.7. N-(N-ACETYL-S-ISOBUTYRYL-L-CYSTEINYL)-S-ISOBUTYRYLCYSTEAMINE (I-190)

La S-acylation de la **I-188** (0,32 mmole), avec du chlorure d'isobutyryle, est réalisée selon la méthode générale décrite dans l'exemple 2. Le mélange réactionnel est ensuite traité suivant le protocole décrit pour la synthèse de la **I-177.** Après les différents traitements, on recueille une gomme qui est purifiée par " flash " chromatographie sur colonne de gel de silice (éluant : CH₂Cl₂/éther 50%). On isole la **I-190** sous forme d'une gomme (Rdt = 63%) qui, après trituration dans de l'hexane, fournit une poudre incolore. R_{f} (CH₂Cl₂/éther, 3,5 : 6,5) : 0,34. F = 99-100°C. [α]_{D}²⁰ = -9,1° (c 0,88; CHCl₃).
RMN ¹H (CDCl₃) δ ppm : 1,20 (d, J = 6,9 Hz, 12H, C(C*H*₃)_{*2*}); 2,01 (s, 3H, NCOC*H*_{*3*}); 2,78 (hept, J = 6,9 Hz, 2H, C*H*(CH₃)₂); 2,92-3,10 (m, 2H, NCH₂C*H*_{*2*}S); 3,26 (d, J = 6,4 Hz, 2H, C*H*_{*2*} cys); 3,37-3,48 (m, 2H, NC*H*_{*2*}CH₂S); 4,46-4,58 (m, 1H, α *H* cys); 6,38 (d, J = 7,3 Hz, 1H, N*H* cys); 6,73-6,83 (m, 1H, N*H*CH₂).
SM : (FAB⁺/G-T) *m*/*z*725 (2M+H)⁺, 363 (M+H)⁺.

| Analyse : C₁₅H₂₆N₂O₄S₂ (362) | | | |
|---|---|---|---|
| Calc. % | C 49,72 | H 7,18 | N 7,73 |
| Tr. % | 49,57 | 7,19 | 7,68 |

### 2.8. N-(N-ACETYL-S-PIVALOYL-L-CYSTEINYL)-S- ISOBUTYRYLCYSTEAMINE (I-191)

La S-acylation de la **I-188** (0,32 mmole), avec du chlorure d'isobutyryle, est réalisée selon la méthode générale décrite dans l'exemple 2. Le mélange réactionnel est ensuite traité suivant le protocole décrit pour la synthèse de la **I-177.** Après les différents traitements, on recueille une gomme qui cristallise dans un mélange d'AcOEt/ éther de pétrole en aiguilles incolores (Rdt = 68%). R_{f} (CH₂Cl₂/éther, 5 : 5) : 0,27. F = 103-104°C. [α]_{D}²⁰ = -8,3° (c 0,97; CHCl₃).
RMN ¹H (CDCl₃) δ ppm : 1,20 (d, J = 6,9 Hz, 6H, C(C*H*₃)_{*2*}); 1,25 (s, 9H, C(C*H*_{*3*})_{*3*}); 2,01 (s, 3H, NCOC*H*_{*3*}); 2,77 (hept, J = 6,9 Hz, 1H, C*H*(CH₃)₂); 2,94-3,08 (m, 2H, NCH₂C*H*_{*2*}S); 3,25 (d, J = 6,4 Hz, 2H, C*H*_{*2*} cys); 3,37-3,49 (m, 2H, NC*H*_{*2*}CH₂S); 4,44-4,57 (m, 1H, α *H* cys); 6,35 (d, J = 7,3 Hz, 1H, NHcys); 6,69-6,80 (m, 1H, N*H*CH₂).
SM : (FAB⁺/G-T) *m*/*z* 753 (2M+H)⁺, 377 (M+H)⁺.

| Analyse : C₁₆H₂₈N₂O₄S₂ (376) | | | |
|---|---|---|---|
| Calc. % | C 51,06 | H 7,45 | N 7,45 |
| Tr. % | 51,16 | 7,53 | 7,44 |

### 2.9. N-(N-ACETYL-S-BENZOYL-L-CYSTEINYL)-S-ISOBUTYRYLCYSTEAMINE (I-192)

La S-acylation de la **I-188** (0,32 mmole), avec du chlorure de benzoyle, est réalisée selon la méthode générale décrite dans l'exemple 2. Le mélange réactionnel est ensuite traité suivant le protocole décrit pour la synthèse de la **I-177.** Après les différents traitements, on recueille une gomme qui est purifiée par " flash " chromatographie sur colonne de gel de silice (éluant : CH₂Cl₂/éther 35%). On isole la **I-192** sous forme d'une gomme (Rdt = 63%) qui, après trituration dans de l'hexane, fournit une poudre incolore. R_{f} (CH₂Cl₂/éther, 5 : 5) : 0,27. F = 137-138°C. [α]_{D}²⁰ = +2,8° (c 1,08; CHCl₃).
RMN ¹H (CDCl₃) δ ppm : 1,18 (d, J = 6,9 Hz, 6H, C(C*H*₃)_{*2*}); 2,02 (s, 3H, NCOC*H*_{*3*}); 2,73 (hept, J = 6,9 Hz, 1H, C*H*(CH₃)₂); 2,96-3,06 (m, 2H, NCH₂C*H*_{*2*}S); 3,39-3,49 (m, 2H, NC*H*_{*2*}CH₂S); 3,50 (d, J = 6,2 Hz, 2H, C*H*_{*2*} cys); 4,60-4,72 (m, 1H, α *H* cys); 6,59 (d, J = 7,3 Hz, 1H, N*H* cys); 6,85-6,97 (m, 1H, N*H*CH₂); 7,41-7,52, 7,57-7,65, 7,93-8,01 (3m, 5H, *H* aromatiques).
SM : (FAB⁺/G-T) *m*/*z* 793 (2M+H)⁺, 397 (M+H)⁺.

| Analyse : C₁₈H₂₄N₂O₄S₂ (396) | | | |
|---|---|---|---|
| Calc. % | C 54,55 | H 6,06 | N 7,07 |
| Tr. % | 54,89 | 6,11 | 7,06 |

### 2.10. N-(N-ACETYL-S-TRITYL-L-CYSTEINYL)-S-PIVALOYLCYSTEAMINE (11)

La réaction de couplage de **7** (7,4 mmoles) avec le chlorhydrate de S-pivaloylcystéamine [(composé obtenu selon les mêmes méthodes que celles décrites par T. Wieland et E. Bokelman, *Ann. Chem*., 1952, 576, 20-34 lors des synthèses des chlorhydrates des S-acétyl et S-benzoylcystéamines) F = 212-213°C] est réalisée selon la méthode B décrite dans la première voie de synthèse (exemple 1). Après les différents traitements, le composé attendu est isolé par " flash " chromatographie sur colonne de gel de silice (éluant : CH₂Cl₂/éther 30%). On recueille **11,** sous forme d'une mousse incolore, avec un rendement de 86%. R_{f} (AcOEt/éther de pétrole, 7 : 3) : 0,5. [α]_{D}²⁰ = +8,5° (c 1,29; CHCl₃).
RMN ¹H (CDCl₃) δ ppm : 1,21 (s, 9H, C(C*H*₃)_{*3*}); 1,90 (s, 3H, NCOC*H*_{*3*}); 2,49 (dd, J = 5,9; 12,9 Hz, 1H, □ *H*a cys); 2,78 (dd, J = 6,5; 12,9 Hz, 1H, β Hb cys); 2,88-2,98 (m, 2H, NCH₂C*H*_{*2*}S); 3,28-3,40 (m, 2H, NC*H*_{*2*}CH₂S); 4,06-4,19 (m, 1H, a *H* cys); 5,77 (d, J = 7,6 Hz, 1H, N*H* cys); 6,27-6,41 (m, 1H, N*H*CH₂); 7,16-7,35, 7,40-7,48 (2m, 15H, *H* aromatiques).
SM : (FAB⁺/G-T) *m*/*z* 549 (M+H)⁺.

| Analyse : C₃₁H₃₆N₂O₃S₂ (548) | | | |
|---|---|---|---|
| Calc. % | C 67,88 | H 6,57 | N 5,11 |
| Tr. % | 66,73 | 6,70 | 5,17 |

### 2.11. N-(N-ACETYL-L-CYSTEINYL)-S-PIVALOYLCYSTEAMINE (I-193)

Ce composé est obtenu par la S-détritylation de **11** (4,57 mmoles). Le protocole utilisé est le même que celui décrit dans l'exemple 1 pour la synthèse de la **I-152.** Après les différents traitements, on recueille une gomme qui est purifiée par " flash " chromatographie sur colonne de gel de silice (éluant : CH₂Cl₂/MeOH 1,5%). On isole la **I-193**, sous forme d'une gomme semi-critalline incolore, avec un rendement de 60%. R_{f} (CH₂Cl₂/MeOH, 9,5 : 0,5) : 0,49. [α]_{D}²⁰ = -20,2° (c 0,94; CHCl₃).
RMN ¹H (CDCl₃) δ ppm : 1,24 (s, 9H, C(C*H*₃)_{*3*}); 1,61 (dd, J = 7,6; 10,3 Hz, 1H, S*H*); 2,08 (s, 3H, NCOC*H*_{*3*}); 2,70 (ddd, J = 6,4; 10,3; 13,9 Hz, 1H, β *H*a cys); 2,97-3,09 (m, 2H, NCH₂C*H*_{*2*}S); 3,08 (ddd, J = 4,1; 7,6; 13,9 Hz, 1H, β *H*b cys); 3,40-3,52 (m, 2H, NC*H*_{*2*}CH₂S); 4,60 (ddd, J = 4,1; 6,4; 7,8 Hz, 1H, α *H* cys); 6,49 (d, J = 7,8 Hz, 1H, N*H* cys); 6,69-6,82 (m, 1H, N*H*CH₂).
SM : (FAB⁺/G-T) *m*/*z* 613 (2M+H)⁺, 307 (M+H)⁺.

| Analyse : C₁₂H₂₂N₂O₃S₂ (306) | | | |
|---|---|---|---|
| Calc. % | C 47,06 | H 7,19 | N 9,15 |
| Tr. % | 47,37 | 7,23 | 9,22 |

### 2.12. N-(N,S-BIS-ACETYL-L-CYSTEINYL)-S-PIVALOYLCYSTEAMINE (I-194)

La S-acylation de la **I-193** (0,33 mmole), avec de l'anhydride acétique, est réalisée selon la méthode générale décrite dans l'exemple 2. Le mélange réactionnel est ensuite traité suivant le protocole décrit pour la synthèse de la **I-177.** Après les différents traitements, on recueille une gomme qui est purifiée par " flash " chromatographie sur colonne de gel de silice (éluant : CH₂Cl₂/éther 55%). On isole la **I-194** sous forme d'une gomme (Rdt = 71%) qui cristallise dans un mélange d'AcOEt/éther de pétrole en plaquettes incolores. R_{f} (CH₂Cl₂/MeOH, 9,5 : 0,5) : 0,58. F = 112-114°C. [α]_{D}²⁰ = -13,8° (c 0,94; CHCl₃).
RMN ¹H (CDCl₃) δ ppm : 1,24 (s, 9H, C(C*H*₃)_{*3*}); 2,03 (s, 3H, NCOC*H*_{*3*}); 2,38 (s, 3H, SCOC*H*_{*3*}); 2,94-3,04 (m, 2H, NCH₂C*H*_{*2*}S); 3,18-3,36 (m, 2H, C*H*_{*2*} cys); 3,36-3,48 (m, 2H, NC*H*_{*2*}CH₂S); 4,48-4,60 (m, 1H, α *H* cys); 6,40 (d, J = 7,5 Hz, 1H, N*H*cys); 6,71-6,83 (m, 1H, N*H*CH₂).
SM : (FAB⁺/G-T) *m*/*z* 697 (2M+H)⁺, 349 (M+H)⁺.

| Analyse : C₁₄H₂₄N₂O₄S₂ (348) | | | |
|---|---|---|---|
| Calc. % | C 48,28 | H 6,90 | N 8,05 |
| Tr. % | 48,34 | 7,00 | 7,97 |

### 2.13. N-(N-ACETYL-S-ISOBUTYRYL-L-CYSTEINYL)-S- PIVALOYLCYSTEAMINE (I-195)

La S-acylation de la **I-193** (0,32 mmole), avec du chlorure d'isobutyryle, est réalisée selon la méthode générale décrite dans l'exemple 2. Le mélange réactionnel est ensuite traité suivant le protocole décrit pour la synthèse de la **I-177.** Après les différents traitements, on recueille une gomme qui, après triturations dans de l'hexane, fournit une poudre incolore homogène sur CCM (Rdt = 56%). Rf (CH₂Cl₂/éther, 3,5 : 6,5) : 0,46. F = 101-102°C. [α]_{D}²⁰ = -5,7° (c 1,05; CHCl₃).
RMN ¹H (CDCl₃) δ ppm : 1,20 (d, J = 6,9 Hz, 6H, C(C*H*₃)_{*2*}); 1,25 (s, 9H, C(C*H*₃)_{*3*}); 2,01 (s, 3H, NCOC*H*_{*3*}); 2,79 (hept, J = 6,9 Hz, 1H, C*H*(CH₃)₂); 2,90-3,08 (m, 2H, NCH₂C*H*_{*2*}S); 3,25 (d, J = 6,3 Hz, 2H, C*H*_{*2*} cys); 3,35-3,47 (m, 2H, NC*H*_{*2*}CH₂S); 4,46-4,58 (m, 1H, α *H* cys); 6,38 (d, J = 7,1 Hz, 1H, N*H*cys); 6,71-6,81 (m, 1H, N*H*CH₂).
SM : (FAB⁺/G-T) *m*/*z* 753 (2M+H)⁺, 377 (M+H)⁺.

| Analyse : C₁₆H₂₈N₂O₄S₂ (376) | | | |
|---|---|---|---|
| Calc. % | C 51,06 | H 7,45 | N 7,45 |
| Tr. % | 51,20 | 7,49 | 7,46 |

### 2.14. N-(N-ACETYL-S-PIVALOYL-L-CYSTEINYL)-S-PIVALOYLCYSTEAMINE (I-196)

La S-acylation de la **I-193** (0,34 mmole), avec du chlorure de pivaloyle, est réalisée selon la méthode générale décrite dans l'exemple 2. Le mélange réactionnel est ensuite traité suivant le protocole décrit pour la synthèse de la **I-177.** Après les différents traitements, on recueille une gomme qui, après triturations dans de l'hexane, fournit une poudre incolore (Rdt = 66%). R_{f} (CH₂Cl₂/éther, 5 : 5) : 0,36. Cristallise dans un mélande d'AcOEt/éther de pétrole sous forme de microcristaux incolores. F = 109-111°C. [α]_{D}²⁰ = -4,4° (c 0,91; CHCl₃).
RMN ¹H (CDCl₃) δ ppm : 1,24 (s, 18H, C(C*H*₃)*3*); 2,00 (s, 3H, NCOC*H*_{*3*}); 2,90-3,08 (m, 2H, NCH₂C*H*_{*2*}S); 3,24 (d, J = 6,4 Hz, 2H, C*H*_{*2*} cys); 3,36-3,47 (m, 2H, NC*H*_{*2*}CH₂S); 4,44-4,57 (m, 1H, α *H* cys); 6,38 (d, J = 7,4 Hz, 1H, N*H* cys); 6,68-6,88 (m, 1H, N*H*CH₂).
SM : (FAB⁺/G-T) *m*/*z* 781 (2M+H)⁺, 391 (M+H)⁺.

| Analyse : C₁₇H₃₀N₂O₄S₂ (390) | | | |
|---|---|---|---|
| Calc. % | C 52,31 | H 7,69 | N 7,18 |
| Tr. % | 52,47 | 7,70 | 7,14 |

### 2.15. N-(N-ACETYL-S-BENZOYL-L-CYSTEINYL)-S-PIVALOYLCYSTEAMINE (I-197)

La S-acylation de la **I-193** (0,34 mmole), avec du chlorure de benzoyle, est réalisée selon la méthode générale décrite dans l'exemple 2. Le mélange réactionnel est ensuite traité suivant le protocole décrit pour la synthèse de la **I-177.** Après les différents traitements, on recueille une gomme qui est purifiée par " flash " chromatographie sur colonne de gel de silice (éluant : CH₂Cl₂/éther 35%). On isole la **I-197** sous forme d'une gomme qui, après trituration dans de l'hexane, fournit une poudre incolore (Rdt = 66%). R_{f} (CH₂Cl₂/éther, 5 : 5) : 0,33. F = 133-134°C. [α]_{D}²⁰ = +5,10° (c 0,98; CHCl₃).
RMN ¹H (CDCl₃) δ ppm : 1,21 (s, 9H, C(C*H*₃)_{*3*}); 2,01 (s, 3H, NCOC*H*_{*3*}); 2,90-3,08 (m, 2H, NCH₂C*H*_{*2*}S); 3,33-3,52 (m, 4H, NC*H*_{*2*}CH₂S, C*H*_{*2*} cys); 4,64-4,77 (m, 1H, α *H* cys); 6,76 (d, J = 7,4 Hz, 1H, N*H* cys); 7,06-7,22 (m, 1H, N*H*CH₂); 7,40-7,50, 7,55-7,63, 7,91-8,0 (3m, 5H, *H* aromatiques).
SM : (FAB⁺/G-T) *m*/*z* 821 (2M+H)⁺, 411 (M+H)⁺.

| Analyse : C₁₉H₂₆N₂O₄S₂ (410) | | | |
|---|---|---|---|
| Calc. % | C 55,61 | H 6,34 | N 6,83 |
| Tr. % | 55,29 | 6,35 | 6,75 |

### 2.16. N-(N-ACETYL-S-TRITYL-L-CYSTEINYL)-S-BENZOYL-CYSTEAMINE (12)

La réaction de couplage de 7 (7,41 mmoles) est réalisée selon la méthode B décrite dans la première voie de synthèse (exemple 1): le chlorhydrate de S-acétylcystéamine a été remplacé par le chlorhydrate de S-benzoylcystéamine (T. Wieland et E. Bokelman, *Ann. Chem*., 1952, 576, 20-34). Après les différents traitements, le composé attendu est isolé par " flash " chromatographie sur colonne de gel de silice (éluant : CH₂Cl₂/éther 15%). On recueille **12,** sous forme d'une mousse incolore, avec un rendement de 62%. R_{f} (AcOEt/éther de pétrole, 7 : 3) : 0,42. [α]_{D}²⁰ = +10,8° (c 1,11; CHCl₃).
RMN ¹H (CDCl₃) δ ppm : 1,86 (s, 3H, NCOC*H*_{*3*}); 2,47 (dd, J = 5,7; 13,0 Hz, 1H, β Ha cys); 2,82 (dd, J = 6,4; 13,0 Hz, 1H, β *H*b cys); 3,08-3,27 (m, 2H, NCH₂C*H*_{*2*}S); 3,41-3,53 (m, 2H, NC*H*_{*2*}CH₂S); 4,08-4,21 (m, 1H, α *H* cys); 5,67 (d, J = 7,7 Hz, 1H, N*H* cys); 6,34-6,46 (m, 1H, N*H*CH₂); 7,17-7,32, 7,37-7,45, 7,54-7,61, 7,89-7,96 (4m, 20H, *H* aromatiques).
SM : (FAB⁺/G-T) *m*/*z* 569 (M+H)⁺.

| Analyse : C₃₃H₃₂N₂O₃S₂ (568) | | | |
|---|---|---|---|
| Calc. % | C 69,72 | H 5,63 | N 4,93 |
| Tr. % | 68,96 | 5,62 | 4,89 |

### 2.17. N-(N-ACETYL-L-CYSTEINYL)-S-BENZOYLCYSTEAMINE (I-198)

Ce composé est obtenu par la S-détritylation de **12** (4,44 mmoles). Le protocole utilisé est le même que celui décrit dans l'exemple 1 pour la synthèse de la **I-152.** Après les différents traitements, on recueille une gomme qui est purifiée par " flash " chromatographie sur colonne de gel de silice (éluant : AcOEt/éther de pétrole 50%). On isole la **I-198,** sous forme d'un solide blanc, avec un rendement de 63%. R_{f} (CH₂Cl₂/MeOH, 9,5 : 0,5) : 0,38. F = 128-130°C. [α]_{D}²⁰ = -24,7° (c 1,01; CHCl₃).
RMN ¹H (CDCl₃) δ ppm : 1,59 (dd, J = 7,6; 10,2 Hz, 1H, S*H*); 2,04 (s, 3H, NCOC*H*_{*3*}); 2,71 (ddd, J = 6,5; 10,2; 13,8 Hz, 1H, β *H*a cys); 3,06 (ddd, J = 4,3; 7,6; 13,8 Hz, 1H, β *H*b cys); 3,20-3,31 (m, 2H, NCH₂C*H*_{*2*}S); 3,52-3,64 (m, 2H, NC*H*_{*2*}CH₂S); 4,61 (ddd, J = 4,3; 6,5; 7,4 Hz, 1H, α *H* cys); 6,51 (d, J = 7,4 Hz, 1H, N*H*cys); 6,83-7,00 (m, 1H, N*H*CH₂); 7,43-7,52, 7,56-7,65, 7,92-8,00 (3m, 5H, *H* aromatiques).
SM : (FAB⁺/G-T) *m*/*z* 653 (2M+H)⁺, 327 (M+H)⁺.

| Analyse : C₁₄H₁₈N₂O₃S₂ (326) | | | |
|---|---|---|---|
| Calc. % | C 51,53 | H 5,52 | N 8,59 |
| Tr. % | 51,49 | 5,55 | 8,60 |

### 2.18. N-(N,S-BIS-ACETYL-L-CYSTEINYL)-S-BENZOYLCYSTEAMINE (I-199)

La S-acylation de la **I-198** (0,34 mmole), avec de l'anhydride acétique, est réalisée selon la méthode générale décrite dans l'exemple 2. Le mélange réactionnel est ensuite traité suivant le protocole décrit pour la synthèse de la **I-177**. Après les différents traitements, on recueille une gomme qui est purifiée par " flash " chromatographie sur colonne de gel de silice (éluant : CH₂Cl₂/MeOH 1,5%). On isole la **I-199** sous forme d'une gomme (Rdt = 75%) qui cristallise de l'AcOEt en d'aiguilles incolores. R_{f} (CH₂Cl₂/MeOH, 9,5 : 0,5) : 0,44. F = 166-168°C. [α]_{D}²⁰ = -14,3° (c 0,98; CHCl₃).
RMN ¹H (CDCl₃) δ ppm : 1,99 (s, 3H, NCOC*H*_{*3*}); 2,32 (s, 3H, SCOC*H*_{*3*}); 3,18-3,31 (m, 4H, NCH₂C*H*_{*2*}S, C*H*_{*2*} cys); 3,48-3,61 (m, 2H, NC*H*_{*2*}CH₂S); 4,49-4,62 (m, 1H, α *H* cys); 6,38 (d, J = 7,6 Hz, 1H, N*H* cys); 6,79-6,92 (m, 1H, N*H*CH₂); 7,42-7,51, 7,55-7,64, 7,93-8,01 (3m, 5H, *H* aromatiques).
SM : (FAB⁺/G-T) *m*/*z* 737 (2M+H)⁺, 369 (M+H)⁺.

| Analyse : C₁₆H₂₀N₂O₄S₂ (368) | | | |
|---|---|---|---|
| Calc. % | C 52,17 | H 5,43 | N 7,61 |
| Tr. % | 52,33 | 5,45 | 7,68 |

### 2.19. N-(N-ACETYL-S-ISOBUTYRYL-L-CYSTEINYL)-S-BENZOYLCYSTEAMINE (I-200)

La S-acylation de la **I-198** (0,30 mmole), avec du chlorure d'isobutyryle, est réalisée selon la méthode générale décrite dans l'exemple 2. Le mélange réactionnel est ensuite traité suivant le protocole décrit pour la synthèse de la **I-177.** Après les différents traitements, on recueille une gomme qui est purifiée par " flash " chromatographie sur colonne de gel de silice (éluant : CH₂Cl₂/éther 40%). On isole la **I-200** sous forme d'une gomme (Rdt = 79%) qui cristallise dans un mélange d'AcOEt/éther de pétrole en plaquettes incolores. R_{f} (CH₂Cl₂/éther, 3 : 7) : 0,2. F = 135-136°C. [α]_{D}²⁰ = -6,7° (c 1,2; CHCl₃).
RMN ¹H (CDCl₃) δ ppm : 1,17 (d, J = 6,9 Hz, 6H, C(C*H*₃)_{*2*}); 1,97 (s, 3H, NCOC*H*_{*3*}); 2,75 (hept, J = 6,9 Hz, 1H, C*H*(CH₃)₂); 3,19-3,30 (m, 4H, NCH₂C*H*_{*2*}S, C*H*_{*2*} cys); 3,46-3,62 (m, 2H, NC*H*_{*2*}CH₂S); 4,49-4,61 (m, 1H, α *H* cys); 6,41 (d, J = 7,4 Hz, 1H, N*H* cys); 6,85-6,96 (m, 1H, N*H*CH₂); 7,41-7,52, 7,55-7,64, 7,90-8,02 (3m, 5H, *H* aromatiques).
SM : (FAB⁺/G-T) *m*/*z* 793 (2M+H)⁺, 397 (M+H)⁺.

| Analyse : C₁₈H₂₄N₂O₄S₂ (396) | | | |
|---|---|---|---|
| Calc. % | C 54,54 | H 6,06 | N 7,07 |
| Tr. % | 54,77 | 6,04 | 7,07 |

### 2.20. N-(N-ACETYL-S-PIVALOYL-L-CYSTEINYL)-S-BENZOYLCYSTEAMINE (I-201)

La S-acylation de la **I-198** (0,30 mmole), avec du chlorure de pivaloyle, est réalisée selon la méthode générale décrite dans l'exemple 2. Le mélange réactionnel est ensuite traité suivant le protocole décrit pour la synthèse de la **I-177.** Après les différents traitements, on recueille une gomme qui est purifiée par " flash " chromatographie sur colonne de gel de silice (éluant : CH₂Cl₂/éther 45%). On isole la **I-201** sous forme d'une gomme (Rdt = 83%) qui cristallise dans un mélange d'AcOEt/éther de pétrole en plaquettes incolores. R_{f} (CH₂Cl₂/éther, 3 : 7) : 0,3. F = 101-103°C. [α]_{D}²⁰ = -3,8° (c 1,05; CHCl₃).
RMN ¹H (CDCl₃) δ ppm : 1,22 (s, 9H, C(C*H*₃)_{*3*}); 1,96 (s, 3H, NCOC*H*_{*3*}); 3,19-3,30 (m, 4H, NCH₂C*H*_{*2*}S, C*H*_{*2*} cys); 3,46-3,62 (m, 2H, NC*H*_{*2*}CH₂S); 4,47-4,58 (m, 1H, α *H* cys); 6,38 (d, J = 7,2 Hz, 1H, N*H* cys); 6,81-6,92 (m, 1H, N*H*CH₂); 7,42-7,52, 7,55-7,65, 7,90-8,02 (3m, 5H, *H* aromatiques).
SM : (FAB⁺/G-T) *m*/*z* 821 (2M+H)⁺, 411 (M+H)⁺.

| Analyse : C₁₉H₂₆N₂O₄S₂ (410) | | | |
|---|---|---|---|
| Calc. % | C 55,61 | H 6,34 | N 6,83 |
| Tr. % | 55,73 | 6,29 | 6,85 |

### 2.21. N-(N-ACETYL-S-BENZOYL-L-CYSTEINYL)-S-BENZOYLCYSTEAMINE (I-202)

La S-acylation de la **I-198** (0,30 mmole), avec du chlorure de benzoyle, est réalisée selon la méthode générale décrite dans l'exemple 2. Le mélange réactionnel est ensuite traité suivant le protocole décrit pour la synthèse de la **I-177**. Après les différents traitements, on recueille une gomme qui est purifiée par " flash " chromatographie sur colonne de gel de silice (éluant : CH₂Cl₂/MeOH 1%). On isole la **I-202** sous forme d'une gomme (Rdt = 72%) qui cristallise de l'AcOEt en plaquettes incolores. R_{f} (CH₂Cl₂/MeOH, 9,5 : 0,5) : 0,66. F = 188-190°C. [α]_{D}²⁰ = +4,1° (c 0,98; CHCl₃).
RMN ¹H (CDCl₃) δ ppm : 1,99 (s, 3H, NCOC*H*_{*3*}); 3,19-3,28 (m, 2H, NCH₂C*H*_{*2*}S); 3,49 (d, J = 6,1 Hz, 2H, C*H*_{*2*} cys); 3,52-3,61 (m, 2H, NC*H*_{*2*}CH₂S); 4,63-4,71 (m, 1H, α H cys); 6,56 (d, J = 7,2 Hz, 1H, N*H* cys); 6,92-7,08 (m, 1H, N*H*CH₂); 7,38-7,48, 7,52-7,62, 7,88-7,97 (3m, 10H, *H* aromatiques).
SM : (FAB⁺/G-T) *m*/*z* 861 (2M+H)⁺, 431 (M+H)⁺.

| Analyse : C₂₁H₂₂N₂O₄S₂ (430) | | | |
|---|---|---|---|
| Calc. % | C 58,60 | H 5,12 | N 6,51 |
| Tr. % | 58,37 | 5,10 | 6,51 |

### 2.22. N-ISOBUTYRYL-S-TRITYL-L-CYSTEINE (13)

Ce composé a été synthétisé en adaptant, à la N-isobutyryl-L-cystéine, la méthode de tritylation décrite par K.-Y. Zee-Cheng et C. C. Cheng, *J. Med. Chem*., 1970, 13, 414-418. Une suspension contenant 4,1 g (21,5 mmoles) de N-isobutyryl-L-cystéine [(préparée selon H. Brückner et coll., *J. Chromatogr*., 1989, 476, 73-82), ([α]_{D}²⁰ = +84° (c 1; CHCl₃))], 5,6 g (21,5 mmoles) de triphénylméthanol et 16 ml d'acide acétique glacial est agitée à la température ambiante. Tout en maintenant la température à 20-25°C, le mélange est additionné, goutte à goutte, de 4,1 ml (32,2 mmoles) d'éthérate de BF₃. Après 3 h d'agitation, la solution marron obtenue est ensuite refroidie à 0 °C puis additionnée de 70 ml d'une solution aqueuse d'acétate de sodium et de 140 ml d'eau. A la fin des ajouts, il y a prise en masse du mélange réactionnel sous forme d'un gel. Ce mélange est abandonné une nuit à 0 °C puis est ensuite additionné, sous forte agitation, de 150 ml d'eau glacée et de 120 ml d'éther. La phase éthérée est décantée et les eaux résiduelles sont lavées avec 4 x 80 ml d'éther. Les phases organiques sont rassemblées, lavées avec 4 x 60 ml d'eau glacée, séchées sur sulfate de sodium, filtrées et évaporées à sec sous vide. Le produit brut obtenu est purifié par lavages successifs avec de l'hexane (5 x 50 ml) pour fournir **13,** sous forme d'une gomme homogène sur CCM avec un rendement de 81%. R_{f} (CH₂Cl₂/MeOH/AcOH, 9,4 : 0,6 : 0,07) : 0,53. [α]_{D}²⁰ = +21,4° (c 1,12; CHCl₃).
RMN ¹H (CDCl₃) δ ppm : 1,12; 1,14 (2d, J = 2 x 6,9 Hz, 2 x 3H, C(C*H*_{*3*})_{*2*}); 2,24-2,42 (m, 1H, C*H*(CH₃)₂); 2,64-2,28 (m, 2H, C*H*_{*2*}); 4,31-4,43 (m, 1H, α *H*); 5,67-6,15 (m large, 1H, CO₂*H*); 5,90 (d, J = 7,1 Hz, 1H, N*H* recouvrant partiellement le m à 5,67-6,15); 7,18-7,33, 7,37-7,46, (2m, 15H, *H* aromatiques).
SM : (FAB⁺/G-T) *m*/*z* 867 (2M+H)⁺, 431 (M+H)⁺; (FAB-/G-T) *m*/*z* 865 (2M-H)⁻, 432 (M-H)⁻.

| Analyse : C₂₆H₂₇NO₃S (433) | | | |
|---|---|---|---|
| Calc. % | C 72,06 | H 6,24 | N 3,23 |
| Tr. % | 72,24 | 6,22 | 3,28 |

### 2.23. N-(N-ISOBUTYRYL-S-TRITYL-L-CYSTEINYL)-S-ACETYLCYSTEAMINE (14)

La réaction de couplage de **13** (3,93 mmoles) avec le chlorhydrate de S-acétylcystéamine est réalisée selon la méthode B décrite dans la première voie de synthèse (exemple 1). Après les différents traitements, le composé attendu est isolé par " flash " chromatographie sur colonne de gel de silice (éluant : AcOEt/éther de pétrole 60%). On recueille **14,** sous forme d'une mousse incolore, avec un rendement de 67%. R_{f} (AcOEt/éther de pétrole, 6 : 4) : 0,5. [α]_{D}²⁰ = +9,3° (c 0,97; CHCl₃).
RMN ¹H (CDCl₃) δ ppm : 1,10 (d, J = 6,9 Hz, 6H, C(C*H*_{*3*})_{*2*}); 2,18-2,36 (m, 1H, C*H*(CH₃)₂); 2,29 (s, 3H, SCOC*H*_{*3*} recouvrant partiellement le m à 2,18-2,36); 2,50 (dd, J = 5,6; 12,8 Hz, 1H, β *H*a cys); 2,72 (dd, J = 6,7; 12,8 Hz, 1H, β *H*b cys); 2,88-3,01 (m, 2H, NCH₂C*H*_{*2*}S); 3,29-3,41 (m, 2H, NC*H*_{*2*}CH₂S); 4,06-4,19 (m, 1H, α *H* cys); 5,82 (d, J = 7,4 Hz, 1H, N*H* cys); 6,42 (t, J = 5,5 Hz, 1H, N*H*CH₂); 7,17-7,35, 7,39-7,48 (2m, 15H, *H* aromatiques).
SM : (FAB⁺/G-T) *m*/*z* 535 (M+H)⁺.

| Analyse : C₃₀H₃₄N₂O₃S (534) | | | |
|---|---|---|---|
| Calc. % | C 67,42 | H 6,37 | N 5,24 |
| Tr. % | 67,05 | 6,72 | 5,30 |

### 2.24. N-(N-ISOBUTYRYL-L-CYSTEINYL)-S-ACETYLCYSTEAMINE (I-203)

Ce composé est obtenu par la S-détritylation de **14** (2,56 mmoles). Le protocole utilisé est le même que celui décrit dans l'exemple 1 pour la synthèse de la **I-152.** Après les différents traitements, on recueille une gomme qui est purifiée par " flash " chromatographie sur colonne de gel de silice (éluant : CH₂Cl₂/éther 30%). On isole la **I-203,** sous forme d'un solide blanc, avec un rendement de 70%. R_{f} (CH₂Cl₂/éther, 5 : 5) : 0,44. F = 117-120°C. [α]_{D}²⁰ = -36,5° (c 1,04; CHCl₃).
RMN ¹H (CDCl₃) δ ppm : 1,19, 1,20 (2d, J = 2 x 6,9 Hz, 2 x 3H, C(C*H*_{*3*})_{*2*}); 1,62 (dd, J = 7,5; 10,3 Hz, 1H, S*H*); 2,37 (s, 3H, SCOC*H*_{*3*}); 2,47 (hept, J = 6,9 Hz, 1H, C*H*(CH₃)₂); 2,72 (ddd, J = 6,5; 10,3; 13,9 Hz, 1H, β *H*a cys); 3,00-3,08 (m, 2H, NCH₂C*H*_{*2*}S); 3,06 (ddd, J = 4,2; 7,5; 13,9 Hz, 1H, β *H*b cys); 3,41-3,53 (m, 2H, NC*H*_{*2*}CH₂S); 4,61 (ddd, J = 4,2; 6,5; 7,4 Hz, 1H, α *H* cys); 6,46 (d, J = 7,4 Hz, 1H, N*H* cys); 6,72-6,85 (m, 1H, N*H*CH₂).
SM : (FAB⁺/G-T) *m*/*z* 293 (M+H)⁺.

| Analyse : C₁₁H₂₀N₂O₃S₂ (292) | | | |
|---|---|---|---|
| Calc. % | C 45,20 | H 6,84 | N 9,59 |
| Tr. % | 45,22 | 7,11 | 9,69 |

### 2.25. N-(N-ISOBUTYRYL-S-ACETYL-L-CYSTEINYL)-S-ACETYLCYSTEAMINE (I-204)

La S-acylation de la **I-203** (0,34 mmole), avec de l'anhydride acétique, est réalisée selon la méthode générale décrite dans l'exemple 2. Le mélange réactionnel est ensuite traité suivant le protocole décrit pour la synthèse de la **I-177**. Après les différents traitements, on recueille une gomme qui est purifiée par " flash " chromatographie sur colonne de gel de silice (éluant : CH₂Cl₂/éther 50%). On isole la **I-204** sous forme semi-cristalline (Rdt = 75%) qui, après trituration dans de l'hexane, fournit une poudre incolore. R_{f} (CH₂Cl₂/éther, 2 : 8) : 0,43. F = 125-127°C. [α]D²⁰ = -22,9° (c 1,05; CHCl₃).
RMN ¹H (CDCl₃) δ ppm : 1,16 (d, J = 6,9 Hz, 6H, C(C*H*_{*3*})_{*2*}); 2,34-2,47 (m, 1H, C*H*(CH₃)₂); 2,36, 2,38 (2s, 2 x 3H, 2 x SCOC*H*_{*3*} recouvrant partiellement le m à 2,34-2,47); 2,97-3,06 (m, 2H, NCH₂C*H*_{*2*}S); 3,26-3,32 (m, 2H, C*H*_{*2*} cys); 3,38-3,48 (m, 2H, NC*H*_{*2*}CH₂S); 4,47-4,58 (m, 1H, α *H* cys); 6,39 (d, J = 7,3 Hz, 1H, N*H* cys); 6,80-6,91 (m, 1H, N*H*CH₂).
SM : (FAB⁺/G-T) *m*/*z* 669 (2M+H)⁺, 335 (M+H)⁺.

| Analyse : C₁₃H₂₂N₂O₄S₂ (334) | | | |
|---|---|---|---|
| Calc. % | C 46,71 | H 6,59 | N 8,38 |
| Tr. % | 46,76 | 6,90 | 8,35 |

### 2.26. N-(N,S-Bis-ISOBUTYRYL-L-CYSTEINYL)-S-ACETYLCYSTEAMINE (I-205)

La S-acylation de la **I-203** (0,32 mmole), avec du chlorure d'isobutyryle, est réalisée selon la méthode générale décrite dans l'exemple 2. Le mélange réactionnel est ensuite traité suivant le protocole décrit pour la synthèse de la **I-177.** Après les différents traitements, on recueille une huile qui est purifiée par " flash " chromatographie sur colonne de gel de silice (éluant : AcOEt/éther de pétrole 70%). On isole la **I-205** sous forme d'une gomme incolore (Rdt = 56%). Rf (AcOEt/éther de pétrole, 4 : 6) : 0,20. [α]D²⁰ = -17,3° (c 1,1; CHCl₃).
RMN ¹H (CDCl₃) δ ppm : 1,16 (d, J = 6,9 Hz, 6H, C(C*H*_{*3*})_{*2*} de N-*i*-but.); 1,20, 1,21 (2d, J = 2 x 6,9 Hz, 2 x 3H, C(C*H*_{*3*})_{*2*} de S-*i*-but.); 2,33-2,46 (m, 1H, C*H*(CH₃)₂ de N-*i*-but); 2,36 (s, 3H, SCOC*H*_{*3*} recouvrant partiellement le m à 2,33-2,46); 2,79 (hept app., J = 6,9 Hz, 1H C*H*(CH₃)₂ de S-*i*-but); 2,97-3,07 (m, 2H, NCH₂C*H*_{*2*}S); 3,25 (dd, J = 5,0; 13,8 Hz, 1H, β *H*a cys); 3,32 (dd, J = 7,5; 13,8 Hz, 1H, β *H*b cys); 3,38-3,48 (m, 2H, NC*H*_{*2*}CH₂S); 4,45-4,57 (m, 1H, α *H* cys); 6,44 (d, J = 7,1 Hz, 1H, N*H* cys); 6,86-6,97 (m, 1H, N*H*CH₂).
SM : (FAB⁺/G-T) *m*/*z* 725 (2M+H)⁺, 363 (M+H)⁺.

| Analyse : C₁₅H₂₆N₂O₄S₂ (362) | | | |
|---|---|---|---|
| Calc. % | C 49,72 | H 7,18 | N 7,73 |
| Tr. % | 49,87 | 7,36 | 7,80 |

### 2.27. N-(N-ISOBUTYRYL-S-PIVALOYL-L-CYSTEINYL)-S-ACETYLCYSTEAMINE (I-206)

La S-acylation de la **I-203** (0,31 mmole), avec du chlorure de pivaloyle, est réalisée selon la méthode générale décrite dans l'exemple 2. Le mélange réactionnel est ensuite traité suivant le protocole décrit pour la synthèse de la **I-177**. Après les différents traitements, on recueille une gomme qui est purifiée par " flash " chromatographie sur colonne de gel de silice (éluant : CH₂Cl₂/éther 50%). On isole la **I-206** sous forme semi-cristalline (Rdt = 60%) qui, après trituration dans de l'hexane, fournit une poudre incolore. R_{f} (AcOEt/éther de pétrole, 4 : 6) : 0,3. F = 101-103°C. [α]_{D}²⁰ = -19,3° (c 1,09; CHCl₃).
RMN ¹H (CDCl₃) δ ppm : 1,15 (d, J = 6,9 Hz, 6H, C(C*H*_{*3*})_{*2*}); 1,24 (s, 9H, C(C*H*₃)_{*3*}); 2,30-2,45 (m, 1H, C*H*(CH₃)₂); 2,36 (s, 3H, SCOC*H*_{*3*} recouvrant partiellement le m à 2,30-2,45); 2,96-3,07 (m, 2H, NCH₂C*H*_{*2*}S); 3,23 (dd, J = 5,3; 13,8 Hz, 1H, β *H*a cys); 3,30 (dd, J = 6,9; 13,8 Hz, 1H, β *H*b cys); 3,37-3,49 (m, 2H, NC*H*_{*2*}CH₂S); 4,44-4,57 (m, 1H, a *H* cys); 6,44 (d, J = 7,1 Hz, 1 H, N*H* cys); 6,85-6,98 (m, 1H, NHCH2).
SM : (FAB⁺/G-T) *m*/*z* 753 (2M+H)⁺, 377 (M+H)⁺.

| Analyse : C₁₆H₂₈N₂O₄S₂ (376) | | | |
|---|---|---|---|
| Calc. % | C 51,06 | H 7,45 | N 7,45 |
| Tr. % | 50,98 | 7,72 | 7,54 |

### 2.28. N-(N-ISOBUTYRYL-S-BENZOYL-L-CYSTEINYL)-S-ACETYLCYSTEAMINE (I-207)

La S-acylation de la **I-203** (0,31 mmole), avec du chlorure de benzoyle, est réalisée selon la méthode générale décrite dans l'exemple 2. Le mélange réactionnel est ensuite traité suivant le protocole décrit pour la synthèse de la **I-177**. Après les différents traitements, on recueille une gomme qui est purifiée par "flash" chromatographie sur colonne de gel de silice (éluant : AcOEt/éther de pétrole 75%). On isole la **I-207** sous forme d'un solide incolore (Rdt = 55%) qui cristallise de l'éther en microcristaux incolores. R_{f} (CH₂Cl₂/éther, 7,5 : 2,5) : 0,45. F. = 135-137°C. [α]_{D}²⁰ = +3,5° (c 1,16; CHCl₃).
RMN ¹H (CDCl₃) δ ppm : 1,12, 1,14 (2d, J = 2 x 6,9 Hz, 2 x 3H, C(C*H*_{*3*})_{*2*}); 2,30-2,47 (m, 1H, C*H*(CH₃)₂); 2,33 (s, 3H, SCOC*H*_{*3*} recouvrant partiellement le m à 2,30-2,47); 2,98-3,07 (m, 2H, NCH₂C*H*_{*2*}S); 3,40-3,58 (m, 4H, C*H*_{*2*} cys, NC*H*_{*2*}CH₂S); 4,59-4,70 (m, 1H, a *H* cys); 6,59 (d, J = 7,1 Hz, 1H, N*H* cys); 6,93-7,04 (m, 1H, N*H*CH₂); 7,41-7,53, 7,57-7,61, 7,92-8,01 (3m, 5H, *H* aromatiques).
SM : (FAB⁺/G-T) *m*/*z* 793 (2M+H)⁺, 397 (M+H)⁺.

| Analyse : C₁₈H₂₄N₂O₄S₂ (396) | | | |
|---|---|---|---|
| Calc. % | C 54,54 | H 6,06 | N 7,07 |
| Tr. % | 54,51 | 6,20 | 7,07 |

### 2.29. N-(N-ISOBUTYRYL-S-TRITYL-L-CYSTEINYL)-S-ISOBUTYRYLCYSTEAMINE (15)

La réaction de couplage de **13** (3,93 mmoles) avec le chlorhydrate de S-isobutyrylcystéamine est réalisée selon la méthode B décrite dans la première voie de synthèse (exemple 1). Après les différents traitements, le composé attendu est isolé par " flash " chromatographie sur colonne de gel de silice (éluant : AcOEt/éther de pétrole 65%). On recueille **15,** sous forme d'une mousse incolore, avec un rendement de 75%. R_{f} (AcOEt/éther de pétrole, 5 : 5) : 0,6. [α]_{D}²⁰ = +7,9° (c 1,27; CHCl₃).
RMN ¹H (CDCl₃) δ ppm : 1,107; 1,110; 1,159; 1,162 (4d, J = 4 x 6,9 Hz, 4 x 3H, 2 x C(C*H*_{*3*})_{*2*}); 2,17-2,32 (m, 1H, C*H*(CH₃)₂ de N-*i*-but.); 2,51 (dd, J = 5,7; 12,8 Hz, 1H, β Ha cys); 2,63-2,79 (m, 1H C*H*(CH₃)₂ de S-*i*-but.); 2,72(dd, J = 6,8; 12,8 Hz, 1H, β Hb cys recouvrant partiellement le m à 2,63-2,79); (2,88-2,98 (m, 2H, NCH₂C*H*_{*2*}S); 3,29-3,40 (m, 2H, NC*H*_{*2*}CH₂S); 4,07-4,19 (m, 1H, a *H* cys); 5,81 (d, J = 7,3 Hz, 1H, N*H* cys); 6,32-6,43 (m, 1H, N*H*CH₂); 7,18-7,35, 7,39-7,47 (2m, 15H, *H* aromatiques).
SM : (FAB⁺/G-T) *m*/*z* 563 (M+H)⁺.

| Analyse : C₃₂H₃₈N₂O₃S₂ (562) | | | |
|---|---|---|---|
| Calc. % | C 68,33 | H 6,76 | N 4,98 |
| Tr. % | 68,27 | 6,71 | 4,88 |

### 2.30. N-(N-ISOBUTYRYL-L-CYSTEINYL)-S-ISOBUTYRYLCYSTEAMINE (I-208)

Ce composé est obtenu par la S-détritylation de 15 (2,75 mmoles). Le protocole utilisé est le même que celui décrit dans l'exemple 1 pour la synthèse de la **I-152.** Après les différents traitements, on recueille une gomme qui est purifiée par "flash" chromatographie sur colonne de gel de silice (éluant : CH₂Cl₂/éther 25%). On isole la **I-208** sous forme d'une gomme qui, après trituration dans de l'hexane, fournit une poudre incolore (Rdt = 69%). R_{f} (CH₂Cl₂/éther, 5 : 5) : 0,39. F = 125-127°C. [α]_{D}²⁰ = -25,7° (c 1,05; CHCl₃).
RMN ¹H (CDCl₃) δ ppm : 1,19, 1,20 (2d, J = 2 x 6,9 Hz, 2 x 6H, 2 x C(C*H*_{*3*})_{*2*}); 1,62 (dd, J = 7,5; 10,3 Hz, 1H, S*H*); (2,41-2,54 (m, 1H, C*H*(CH₃)₂ de N-*i*-but.); 2,70-2,84 (m, 1H, C*H*(CH₃)₂ de S-*i*-but.); 2,72(ddd, J = 6,5; 10,3; 13,7 Hz, 1H, β *H*a cys recouvrant partiellement le m à 2,70-2,84); 2,98-3,14 (m, 3H, β *H*b cys, NCH₂C*H*_{*2*}S); 3,42-3,53 (m, 2H, NCH₂CH₂S); 4,54-4,66 (m, 1H, α H cys); 5,81 (d, J = 7,4 Hz, 1H, NH cys); 6,74-6,85 (m, 1H, N*H*CH₂).
SM : (FAB⁺/G-T) *m*/*z* 641 (2M+H)⁺, 321 (M+H)⁺.

| Analyse : C₁₃H₂₄N₂O₃S₂ (320) | | | |
|---|---|---|---|
| Calc. % | C 48,75 | H 7,50 | N 8,75 |
| Tr. % | 48,45 | 7,82 | 8,75 |

### 2.31. N-(N-ISOBUTYRYL-S-ACETYL-L-CYSTEINYL)-S-ISOBUTYRYLCYSTEAMINE (I-209)

La S-acylation de la **I-208** (0,28 mmole), avec de l'anhydride acétique, est réalisée selon la méthode générale décrite dans l'exemple 2. Le mélange réactionnel est ensuite traité suivant le protocole décrit pour la synthèse de la **I-177.** Après les différents traitements on recueille une gomme qui, après triturations dans de l'hexane, fournit une poudre incolore homogène sur CCM avec un rendement de 80%. R_{f} (CH₂Cl₂/éther, 5 : 5) : 0,55. F = 100-102°C. [α]_{D}²⁰ = -22,9° (c 0,92; CHCl₃).
RMN ¹H (CDCl₃) δ ppm : 1,15, 1,20 (2d, J = 2 x 6,9 Hz, 2 x 6H, 2 x C(C*H*_{*3*})_{*2*}); (2,34-2,47 (m, 1H, C*H*(CH₃)₂ de N-*i*-but.); 2,37 (s, 3H, SCOC*H*_{*3*} recouvrant partiellement le m à 2,34-2,47); 2,69-2,82 (m, 1H, C*H*(CH₃)₂ de S-*i*-but.); 2,95-3,05 (m, 2H, NCH₂C*H*_{*2*}S); 3,26-3,32 (m, 2H, C*H*_{*2*} cys); 3,37-3,47 (m, 2H, NC*H*_{*2*}CH₂S); 4,47-4,59 (m, 1H, α *H* cys); 6,39 (d, J = 7,1 Hz, 1H, N*H* cys); 6,79-6,90 (m, 1H, N*H*CH₂).
SM : (FAB⁺/G-T) *m*/*z* 725 (2M+H)⁺, 363 (M+H)⁺.

| Analyse : C₁₅H₂₆N₂O₄S₂ (362) | | | |
|---|---|---|---|
| Calc. % | C 49,72 | H 7,18 | N 7,73 |
| Tr. % | 49,47 | 7,41 | 7,70 |

### 2.31. N-(N,S-BIS-ISOBUTYRYL-L-CYSTEINYL)-S-ISOBUTYRYLCYSTEAMINE (I-210)

La S-acylation de la **I-208** (0,28 mmole), avec du chlorure d'isobutyryle, est réalisée selon la méthode générale décrite dans l'exemple 2. Le mélange réactionnel est ensuite traité suivant le protocole décrit pour la synthèse de la **I-177**. Après les différents traitements, on recueille une gomme qui est purifiée par " flash " chromatographie sur colonne de gel de silice (éluant : CH₂Cl₂/éther 25%). On isole la **I-210** sous forme d'une gomme qui, après trituration dans de l'hexane, fournit une poudre incolore (Rdt = 63%). R_{f} (CH₂Cl₂/éther, 5 : 5) : 0,68. F = 94-96°C. [α]_{D}²⁰ = -11° (c 0,91; CHCl₃).
RMN ¹H (CDCl₃) δ ppm : 1,154, 1,158, 1,199, 1,202 (4d, J = 4 x 6,9 Hz, 2 x 3H, 2 x 6H, 3 x C(C*H*_{*3*})_{*2*}); (2,32-2,46 (m, 1H, C*H*(CH₃)₂ de N-*i*-but.); 2,69-2,86 (m, 2H, 2 x C*H*(CH₃)₂ de 2 x S-*i*-but); 2,93-3,07 (m, 2H, NCH₂C*H*_{*2*}S); 3,25 (dd, J = 5,0; 14,5 Hz, 1H, β *H*a cys); 3,32 (dd, J = 7,7; 14,5 Hz, 1H, β *H*b cys); 3,38-3,47 (m, 2H, NC*H*_{*2*}CH₂S); 4,45-4,56 (m, 1H, α *H* cys); 6,42 (d, J = 7,4 Hz, 1H, N*H* cys); 6,82-6,92 (m, 1H, N*H*CH₂).
SM : (FAB⁺/G-T) *m*/*z* 781 (2M+H)⁺, 391 (M+H)⁺.

| Analyse : C₁₇H₃₀N₂O₄S₂ (390) | | | |
|---|---|---|---|
| Calc. % | C 52,31 | H 7,69 | N 7,18 |
| Tr. % | 52,02 | 8,02 | 7,21 |

### 2.32. N-(N-ISOBUTYRYL-S-BENZOYL-L-CYSTEINYL)-S-ISOBUTYRYLCYSTEAMINE (I-211)

La S-acylation de la **I-208** (0,29 mmole), avec du chlorure de benzoyle, est réalisée selon la méthode générale décrite dans l'exemple 2. Le mélange réactionnel est ensuite traité suivant le protocole décrit pour la synthèse de la **I-177**. Après les différents traitements, on recueille une gomme qui est purifiée par " flash " chromatographie sur colonne de gel de silice (éluant : CH₂Cl₂/éther 35%). On isole la **I-210** sous forme d'une gomme qui, après trituration dans de l'éther de pétrole, fournit une poudre incolore (Rdt = 86%). R_{f} (CH₂Cl₂/éther, 6 : 4) : 0,52. F = 155-157°C. [α]_{D}²⁰ = +7,0° (c 1; CHCl₃).
RMN ¹H (CDCl₃) δ ppm : 1,12, 1,15, 1,18, (3d, J = 3 x 6,9 Hz, 2 x 3H, 1 x 6H, 2 x C(C*H*_{*3*})_{*2*}); (2,36-2,46 (m, 1H, C*H*(CH₃)₂ de N-*i*-but.); 2,68-2,80 (m, 1H, C*H*(CH₃)₂ de S-*i*-but); 2,95-3,05 (m, 2H, NCH₂C*H*_{*2*}S); 3,40-3,62 (m, 4H, C*H*_{*2*} cys, NC*H*_{*2*}CH₂S); 4,56-4,69 (m, 1H, α *H* cys); 6,54 (d, J = 7,0 Hz, 1H, *NH* cys); 6,84-6,95 (m, 1H, N*H*CH₂); 7,42-7,51, 7,57-7,65, 7,94-8,01 (m, 5H, *H* aromatiques).
SM : (FAB⁺/G-T) *m*/*z* 849 (2M+H)⁺, 425 (M+H)⁺.

| Analyse : C₂₀H₂₈N₂O₄S₂ (424) | | | |
|---|---|---|---|
| Calc. % | C 56,60 | H 6,60 | N 6,60 |
| Tr. % | 56,42 | 6,79 | 6,63 |

### 2.33. N-(N-ISOBUTYRYL-S-TRITYL-L-CYSTEINYL)-S-PIVALOYLCYSTEAMINE (16)

La réaction de couplage de **13** (3,93 mmoles) avec le chlorhydrate de S-pivaloylcystéamine est réalisée selon la méthode B décrite dans la première voie de synthèse (exemple 1). Après les différents traitements, le composé attendu est isolé par "flash" chromatographie sur colonne de gel de silice (éluant : AcOEt/éther de pétrole 70%). On recueille **16** sous forme d'une mousse qui, après trituration dans de l'hexane, fournit une poudre incolore (Rdt = 88%). R_{f} (CH₂Cl₂/éther, 6 : 4) : 0,82. F = 85-88°C. [α]_{D}²⁰ = +5,9° (c 1,02; CHCl₃).
RMN ¹H (CDCl₃) δ ppm : 1,11 (d, J = 6,9 Hz, 6H, C(C*H*_{*3*})_{*2*}); 1,21 (s, 9H, C(C*H*_{*3*})_{*3*}); 2,20-2,38 (m, 1H, C*H*(CH₃)₂); 2,51 (dd, J = 5,6; 12,9 Hz, 1H, β Ha cys); 2,72(dd, J = 6,7; 12,9 Hz, 1H, β *H*b cys); 2,84-2,96 (m, 2H, NCH₂C*H*_{*2*}S); 3,27-3,39 (m, 2H, NC*H*_{*2*}CH₂S); 4,03-4,17 (m, 1H, α H cys); 5,78 (d, J = 7,4 Hz, 1H, NH cys); 6,22-6,34 (m, 1H, N*H*CH₂); 7,18-7,35, 7,38-7,48 (2m, 15H, *H* aromatiques).
SM : (FAB⁺/G-T) *m*/*z* 577 (M+H)⁺.

| Analyse : C₃₃H₄₀N₂O₃S₂ (576) | | | |
|---|---|---|---|
| Calc. % | C 68,75 | H 6,94 | N 4,86 |
| Tr. % | 68,49 | 6,98 | 4,93 |

### 2.34. N-(N-ISOBUTYRYL -L-CYSTEINYL)-S-PIVALOYLCYSTEAMINE (I-214)

Ce composé est obtenu par la S-détritylation de **16** (2,78 mmoles). Le protocole utilisé est le même que celui décrit dans l'exemple 1 pour la synthèse de la **I-152.** Après les différents traitements, on recueille une gomme qui est purifiée par "flash" chromatographie sur colonne de gel de silice (éluant : CH₂Cl₂/éther 22%). On isole la **I-214** sous forme d'une gomme qui, après trituration dans de l'hexane, fournit une poudre incolore (Rdt = 70%). R_{f} (CH₂Cl₂/éther, 5 : 5) : 0,46. F = 120-122°C. [α]_{D}²⁰ = -25° (c 1,04; CHCl₃).
RMN ¹H (CDCl₃) δ ppm : 1,194, 1,198 (2d, J = 2 x 6,9 Hz, 2 x 3H, C(C*H*_{*3*})_{*2*}); 1,23 (s, 9H, C(C*H*_{*3*})_{*3*}); 1,61 (dd, J = 7,6; 10,2 Hz, 1H, S*H*); 2,47 (hept app., J = 6,9 Hz, 1H, C*H*(CH₃)₂); 2,72(ddd, J = 6,5; 10,2; 13,8 Hz, 1H, β *H*a cys); 2,95-3,13 (m, 3H, β Hb cys, NCH₂C*H*_{*2*}S); 3,40-3,52 (m, 2H, NC*H*_{*2*}CH₂S); 4,54-4,66 (m, 1H, a *H* cys); 6,49 (d, J = 7,5 Hz, 1H, N*H* cys); 6,73-6,88 (m, 1H, N*H*CH₂).
SM : (FAB⁺/G-T) *m*/*z* 669 (2M+H)⁺, 335 (M+H)⁺.

| Analyse : C₁₄H₂₆N₂O₃S₂ (334) | | | |
|---|---|---|---|
| Calc. % | C 50,30 | H 7,78 | N 8,38 |
| Tr. % | 50,19 | 7,92 | 8,35 |

### 2.35. N-(N-ISOBUTYRYL-S-ACETYL-L-CYSTEINYL)-S-PIVALOYLCYSTEAMINE (I-215)

La S-acylation de la **I-214** (0,27 mmole), avec de l'anhydride acétique, est réalisée selon la méthode générale décrite dans l'exemple 2. Le mélange réactionnel est ensuite traité suivant le protocole décrit pour la synthèse de la **I-177.** Après les différents traitements on recueille une gomme qui, après triturations dans de l'hexane, fournit une poudre incolore homogène sur CCM avec un rendement de 80%. R_{f} (CH₂Cl₂/éther, 6 : 4) : 0,45. F = 112-114°C. [α]_{D}²⁰ = -18,8° (c 0,9; CHCl₃).
RMN ¹H (CDCl₃) δ ppm : 1,16 (d, J = 6,9 Hz, 6H, C(C*H*_{*3*})_{*2*}); 1,24 (s, 9H, C(C*H*_{*3*})_{*3*}); (2,31-2,49 (m, 1H, C*H*(CH₃)₂); 2,37 (s, 3H, SCOC*H*_{*3*} recouvrant partiellement le m à 2,31-2,49); 2,88-3,08 (m, 2H, NCH₂C*H*_{*2*}S); 3,25-3,34 (m, 2H, C*H*_{*2*} cys); 3,35-3,47 (m, 2H, NC*H*_{*2*}CH₂S); 4,48-4,60 (m, 1H, a H cys); 6,38 (d, J = 7,3 Hz, 1H, N*H* cys); 6,74-6,89 (m, 1H, N*H*CH₂).
SM : (FAB⁺/G-T) *m*/*z* 377 (M+H)⁺.

| Analyse : C₁₅H₂₈N₂O₄S₂ (376) | | | |
|---|---|---|---|
| Calc. % | C 51,06 | H 7,45 | N 7,45 |
| Tr. % | 50,97 | 7,81 | 7,47 |

### 2.36. N-( N,S-BIS-ISOBUTYRYL -L-CYSTEINYL)-S-PIVALOYLCYSTEAMINE (I-216)

La S-acylation de la **I-214** (0,26 mmole), avec du chlorure d'isobutyryle, est réalisée selon la méthode générale décrite dans l'exemple 2. Le mélange réactionnel est ensuite traité suivant le protocole décrit pour la synthèse de la **I-177**. Après les différents traitements, on recueille une gomme qui est purifiée par "flash" chromatographie sur colonne de gel de silice (éluant : CH₂Cl₂/éther 15%). On isole la **I-216** sous forme d'une gomme qui, après trituration dans de l'hexane, fournit une poudre incolore (Rdt = 78%). R_{f} (CH₂Cl₂/éther, 6 : 4) : 0,61. F = 105-107°C. [α]_{D}²⁰ = -12,2° (c 1,07; CHCl₃).
RMN ¹H (CDCl₃) δ ppm : 1,154, 1,157, 1,196, 1,201 (4d, J = 4 x 6,9 Hz, 4 x 3H, 2 x C(C*H*_{*3*})_{*2*}); 1,24 (s, 9H, C(C*H*_{*3*})_{*3*}); 2,39 (hept app., J = 6,9 Hz, 1H, C*H*(CH₃)₂ de N-*i*-but); 2,79 (hept app., J = 6,9 Hz, 1H, C*H*(CH₃)₂ de S-*i*-but); 2,87-3,08 (m, 2H, NCH₂C*H*_{*2*}S); 3,25 (dd, J = 5,2; 14,5 Hz, 1H, β *H*a cys); 3,32 (dd, J = 7,5, 14,5 Hz, 1H, β *H*b cys); 3,37-3,47 (m, 2H, NC*H*_{*2*}CH₂S); 4,46-4,59 (m, 1H, α *H* cys); 6,43 (d, J = 7,0 Hz, 1H, N*H* cys); 6,81-6,93 (m, 1H, N*H*CH₂).
SM : (FAB⁺/G-T) *m*/*z* 809 (2M+H)⁺, 405 (M+H)⁺.

| Analyse : C₁₈H₃₂N₂O₄S₂ (404) | | | |
|---|---|---|---|
| Calc. % | C 53,47 | H 7,92 | N 6,93 |
| Tr. % | 53,33 | 8,09 | 6,95 |

### 2.37. N-(N-ISOBUTYRYL-S-PIVALOYL-L-CYSTEINYL)-S-PIVALOYLCYSTEAMINE (I-217)

La S-acylation de la **I-214** (0,26 mmole), avec du chlorure de pivaloyle, est réalisée selon la méthode générale décrite dans l'exemple 2. Le mélange réactionnel est ensuite traité suivant le protocole décrit pour la synthèse de la **I-177**. Après les différents traitements on recueille une gomme qui, après triturations dans de l'hexane, fournit une poudre incolore homogène sur CCM avec un rendement de 55%. R_{f} (CH₂Cl₂/éther, 6 : 4) : 0,63. F = 106-108°C. [α]_{D}²⁰ = -10,2° (c 1,18; CHCl₃).
RMN ¹H (CDCl₃) δ ppm : 1,151, 1,157, (2d, J = 2 x 6,9 Hz, 2 x 3H, C(C*H*_{*3*})_{*2*}); 1,240, 1,244 (2s, 2 x 9H, 2 x C(C*H*_{*3*})_{*3*}); 2,38 (hept app., J = 6,9 Hz, 1H, C*H*(CH₃)₂); 2,86-3,07 (m, 2H, NCH₂C*H*_{*2*}S); 3,24 (dd, J = 5,0; 14,2 Hz, 1H, β *H*a cys); 3,30 (dd, J = 6,5, 14,2 Hz, 1H, β *H*b cys); 3,33-3,48 (m, 2H, NC*H*_{*2*}CH₂S); 4,42-4,54 (m, 1H, α *H* cys); 6,39 (d, J = 7,0 Hz, 1H, NH cys); 6,74-6,86 (m, 1H, N*H*CH₂).
SM : (FAB⁺/G-T) *m*/*z* 837 (2M+H)⁺, 419 (M+H)⁺.

| Analyse : C₁₉H₃₄N₂O₄S₂ (418) | | | |
|---|---|---|---|
| Calc. % | C 54,81 | H 8,17 | N 6,73 |
| Tr. % | 54,50 | 8,27 | 6,74 |

### 2.38. N-(N-ISOBUTYRYL-S BENZOYL-L-CYSTEINYL)-S-PIVALOYLCYSTEAMINE (I-218)

La S-acylation de la **I-214** (0,26 mmole), avec du chlorure de benzoyle, est réalisée selon la méthode générale décrite dans l'exemple 2. Le mélange réactionnel est ensuite traité suivant le protocole décrit pour la synthèse de la **I-177**. Après les différents traitements, on recueille une gomme qui est purifiée par " flash " chromatographie sur colonne de gel de silice (éluant : CH₂Cl₂/éther 25%). On isole la **I-218** sous forme d'une gomme qui, après trituration dans de l'hexane, fournit une poudre incolore (Rdt = 73%). R_{f} (CH₂Cl₂/éther, 5 : 5) : 0,57. F = 123-124°C. [α]_{D}²⁰ = +8,7° (c 0,92; CHCl₃).
RMN ¹H (CDCl₃) δ ppm : 1,12, 1,14, (2d, J = 2 x 6,9 Hz, 2 x 3H, C(C*H*_{*3*})_{*2*}); 1,22 (s, 9H, C(C*H*_{*3*})_{*3*}); 2,40 (hept app., J = 6,9 Hz, 1H, C*H*(CH₃)₂); 2,89-3,09 (m, 2H, NCH₂C*H*_{*2*}S); 3,37-3,61 (m, 4H, NC*H*_{*2*}CH₂S, C*H*_{*2*} cys); 4,57-4,70 (m, 1H, a H cys); 6,55 (d, J = 7,3 Hz, 1H, N*H* cys); 6,84-6,94 (m, 1H, N*H*CH₂); 7,41-7,52, 7,55-7,66, 7,92-8,01 (3m, 5H, *H* aromatiques).
SM : (FAB⁺/G-T) *m*/*z* 877 (2M+H)⁺, 439 (M+H)⁺.

| Analyse : C₂₁H₃₀N₂O₄S₂ (438) | | | |
|---|---|---|---|
| Calc. % | C 57,53 | H 6,85 | N 6,39 |
| Tr. % | 57,53 | 6,89 | 6,37 |

### 2. 39. N-(N-ISOBUTYRYL-S-TRITYL-L-CYSTEINYL)-S-BENZOYLCYSTEAMINE (17)

La réaction de couplage de **13** (3,93 mmoles) avec le chlorhydrate de S-benzoylcystéamine est réalisée selon la méthode B décrite dans la première voie de synthèse (exemple 1). Après les différents traitements, le composé attendu est isolé par "flash" chromatographie sur colonne de gel de silice (éluant : AcOEt/éther de pétrole 70%). On recueille **17** sous forme d'une mousse qui, après trituration dans de l'hexane, fournit une mousse incolore (Rdt = 77%). R_{f} (CH₂Cl₂/éther, 6 : 4) : 0,76. [α]_{D}²⁰ = +7,8° (c 1,03; CHCl₃).
RMN ¹H (CDCl₃) δ ppm : 1,09 (d, J = 6,9 Hz, 6H, C(CH₃)₂); 2,18-2,31 (m, 1H, C*H*(CH₃)₂); 2,51 (dd, J = 5,6; 12,9 Hz, 1H, β *H*a cys); 2,74 (dd, J = 6,7, 12,9 Hz, 1H, β Hb cys); 3,07-3,25 (m, 2H, NCH₂C*H*_{*2*}S); 3,40-3,51 (m, 2H, NC*H*_{*2*}CH₂S); 4,07-4,19 (m, 1H, α *H* cys); 5,74 (d, J = 7,6 Hz, 1H, NH cys); 6,35-6,45 (m, 1H, N*H*CH₂); 7,17-7,33, 7,38-7,47, 7,53-7,62, 7,89-7,96 (4m, 20H, *H* aromatiques).
SM : (FAB⁺/G-T) m/z 597 (M+H)⁺.

| Analyse : C₃₅H₃₆N₂O₃S₂ (596) | | | |
|---|---|---|---|
| Calc. % | C 70,47 | H 6,04 | N 4,70 |
| Tr. % | 70,14 | 6,10 | 4,79 |

### 2.40. N-(N-ISOBUTYRYL-L-CYSTEINYL)-S-BENZOYLCYSTEAMINE (I-219)

Ce composé est obtenu par la S-détritylation de **17** (2,68 mmoles). Le protocole utilisé est le même que celui décrit dans l'exemple 1 pour la synthèse de la **I-152.** Après les différents traitements, on recueille une gomme qui est purifiée par " flash " chromatographie sur colonne de gel de silice (éluant : CH₂Cl₂/éther 25%). On isole la **I-219** sous forme d'une gomme qui, après trituration dans de l'hexane, fournit une poudre incolore (Rdt = 58%). Rf (CH₂Cl₂/éther, 6 : 4) : 0,35. F = 127-130°C. [α]_{D}²⁰ = -20,8° (c 1,06; CHCl₃).
RMN ¹H (CDCl₃) δ ppm : 1,17, 1,18, (2d, J = 2 x 6,9 Hz, 2 x 3H, C(C*H*_{*3*})_{*2*}); 1,59 (dd, J = 7,5 ; 10,3 Hz, 1H, SH) ; 2,44 (hept app., J = 6,9 Hz, 1H, C*H*(CH₃)₂) ; 2,70 (ddd, J = 6,5, 10,3, 13,8 Hz, 1H, β *H*a cys); 3,07 (ddd, J = 4,2, 7,5, 13,8 Hz, 1H, β *H*b cys); 3,17-3,34 (m, 2H, NCH₂C*H*_{*2*}S); 3,53-3,64 (m, 2H, NC*H*_{*2*}CH₂S); 4,62 (ddd, J = 4,2, 6,5, 8,0 Hz, 1H, α *H* cys); 6,47 (d, J = 8,0 Hz, 1H, N*H* cys); 6,80-6,91 (m, 1H, N*H*CH₂); 7,42-7,53, 7,56-7,65, 7,92-8,01 (3m, 5H, *H* aromatiques).
SM : (FAB⁺/G-T) *m*/*z* 709 (2M+H)⁺, 355 (M+H)⁺.

| Analyse : C₁₆H₂₂N₂O₃S₂ (354) | | | |
|---|---|---|---|
| Calc. % | C 54,23 | H 6,21 | N 7,91 |
| Tr. % | 54,20 | 6,18 | 7,94 |

### 2.41. N-(N-ISOBUTYRYL-S-ACETYL-L-CYSTEINYL)-S-BENZOYLCYSTEAMINE (I-220)

La S-acylation de la **I-219** (0,25 mmole), avec de l'anhydride acétique, est réalisée selon la méthode générale décrite dans l'exemple 2. Le mélange réactionnel est ensuite traité suivant le protocole décrit pour la synthèse de la **I-177.** Après les différents traitements, on recueille une gomme qui est purifiée par " flash " chromatographie sur colonne de gel de silice (éluant : CH₂Cl₂/éther 15%). On isole la **I-220** sous forme d'une gomme qui, après trituration dans de l'hexane, fournit une poudre incolore (Rdt = 70%). R_{f} (CH₂Cl₂/éther, 7,5 : 2,5) : 0,43. F = 174-176°C. [α]_{D}²⁰ = -17,6° (c 0,91; CHCl₃).
RMN ¹H (CDCl₃) δ ppm : 1,13, 1,14, (2d, J = 2 x 6,9 Hz, 2 x 3H, C(C*H*_{*3*})_{*2*}); 2,29-2,45 (m, 1H, C*H*(CH₃)₂); 2,32 (s, 3H, SCOC*H*_{*3*} recouvrant partiellement le m à 2,29-2,45); 3,18-3,38 (m, 4H, NCH₂C*H*_{*2*}S, C*H*_{*2*} cys); 3,45-3,62 (m, 2H, NC*H*_{*2*}CH₂S); 4,50-4,62 (m, 1H, α *H* cys); 6,39 (d, J = 7,1 Hz, 1H, N*H* cys); 6,88-6,98 (m, 1H, N*H*CH₂); 7,42-7,52, 7,55-7,64, 7,92-8,01 (3m, 5H, *H* aromatiques).
SM : (FAB⁺/G-T) *m*/*z* 793 (2M+H)⁺, 397 (M+H)⁺.

| Analyse : C₁₈H₂₄N₂O₄S₂ (396) | | | |
|---|---|---|---|
| Calc. % | C 54,54 | H 6,06 | N 7,07 |
| Tr. % | 54,46 | 6,02 | 7,08 |

### 2.42. N-(N,S-BIS-ISOBUTYRYL-L-CYSTEINYL)-S-BENZOYLCYSTEAMINE (I-221)

La S-acylation de la **I-219** (0,25 mmole), avec du chlorure d'isobutyryle, est réalisée selon la méthode générale décrite dans l'exemple 2. Le mélange réactionnel est ensuite traité suivant le protocole décrit pour la synthèse de la I-177. Après les différents traitements on recueille une gomme qui, après triturations dans de l'hexane, fournit une poudre incolore homogène sur CCM avec un rendement de 86%. R_{f} (CH₂Cl₂/éther, 7,5 : 2,5) : 0,42. F = 141-143°C. [α]_{D}²⁰ = -9° (c 1,11; CHCl₃).
RMN ¹H (CDCl₃) δ ppm : 1,13, 1,14 (2d, J = 2 x 6,9 Hz, 2 x 3H, C(C*H*_{*3*})_{*2*} de N-*i*-but.); 1,18 (d, J = 6,9 Hz, 6H, C(C*H*_{*3*})_{*2*} de S-*i*-but.); 2,37 (hept app., J = 6,9 Hz, 1H, C*H*(CH₃)₂ de N-*i*-but); 2,75 (hept app., J = 6,9 Hz, 1H, C*H*(CH₃)₂ de S-*i*-but); 3,16-3,38 (m, 4H, C*H*_{*2*} cys, NCH₂C*H*_{*2*}S); 3,42-3,64 (m, 2H, NC*H*_{*2*}CH₂S); 4,47-4,58 (m, 1H, α *H* cys); 6,42 (d, J = 7,0 Hz, 1H, N*H* cys); 6,87-7,01 (m, 1H, N*H*CH₂); 7,41-7,50, 7,55-7,63, 7,93-8,01 (3m, 5H, *H* aromatiques).
SM : (FAB⁺/G-T) *m*/*z* 849 (2M+H)⁺, 425 (M+H)⁺.

| Analyse : C₂₀H₂₈N₂O₄S₂ (424) | | | |
|---|---|---|---|
| Calc. % | C 56,60 | H 6,60 | N 6,60 |
| Tr. % | 56,67 | 6,62 | 6,63 |

### 2.43. N-(N-ISOBUTYRYL-S-PIVALOYL-L-CYSTEINYL)-S-BENZOYLCYSTEAMINE (I-222)

La S-acylation de la **I-219** (0,25 mmole), avec du chlorure de pivaloyle, est réalisée selon la méthode générale décrite dans l'exemple 2. Le mélange réactionnel est ensuite traité suivant le protocole décrit pour la synthèse de la I-177. Après les différents traitements, on recueille une gomme qui est purifiée par " flash " chromatographie sur colonne de gel de silice (éluant : CH₂Cl₂/éther 20%). On isole la **I-222** sous forme d'une gomme (Rdt = 50%) qui, après trituration dans de l'hexane, fournit une poudre incolore. R_{f} (CH₂Cl₂/éther, 5 : 5) : 0,55. F = 112-114°C. [α]_{D}²⁰ = +4,6° (c 1,08; CHCl₃).
RMN ¹H (CDCl₃) δ ppm : 1,12, 1,13 (2d, J = 2 x 6,9 Hz, 2 x 3H, C(C*H*_{*3*})_{*2*}); 1,22 (s, 9H; C(C*H*₃)_{*3*}); 2,36 (hept app., J = 6,9 Hz, 1H, C*H*(CH₃)₂); 3,18-3,36 (m, 4H, C*H*_{*2*} cys, NCH₂C*H*_{*2*}S); 3,45-3,62 (m, 2H, NC*H*_{*2*}CH₂S); 4,48-4,60 (m, 1H, α *H* cys); 6,48 (d, J = 7,2 Hz, 1H, N*H* cys); 6,96-7,08 (m, 1H, N*H*CH₂); 7,40-7,51, 7,54-7,63, 7,92-8,01 (3m, 5H, *H* aromatiques).
SM : (FAB⁺/G-T) *m*/*z* 877 (2M+H)⁺, 439 (M+H)⁺.

| Analyse : C₂₁H₃₀N₂O₄S₂ (438) | | | |
|---|---|---|---|
| Calc. % | C 57,53 | H 6,85 | N 6,39 |
| Tr. % | 57,31 | 6,86 | 6,34 |

### 2.44. N-(N-ISOBUTYRYL-S-BENZOYL-L-CYSTEINYL)-S-BENZOYLCYSTEAMINE (I-223)

La S-acylation de la **I-219** (0,26 mmole), avec du chlorure de benzoyle, est réalisée selon la méthode générale décrite dans l'exemple 2. Le mélange réactionnel est ensuite traité suivant le protocole décrit pour la synthèse de la I-177. Après les différents traitements, on recueille une gomme qui est purifiée par "flash" chromatographie sur colonne de gel de silice (éluant : CH₂Cl₂/éther 20%). On isole la **I-223** sous forme d'une gomme (Rdt = 84%) qui, après trituration dans de l'hexane, fournit une poudre incolore. R_{f} (CH₂Cl₂/éther, 7 : 3) : 0,41. F = 154-156°C. [α]_{D}²⁰ = +7,6° (c 0,92; CHCl₃).
RMN ¹H (CDCl₃) δ ppm : 1,10, 1,11 (2d, J = 2 x 6,9 Hz, 2 x 3H, C(C*H*_{*3*})_{*2*}); 2,38 (hept app., J = 6,9 Hz, 1H, C*H*(CH₃)₂); 3,19-3,28 (m, 2H, NCH₂C*H*_{*2*}S); 3,43-3,64 (m, 4H, C*H*_{*2*} cys, NC*H*_{*2*}CH₂S); 4,58-4,71 (m, 1H, α *H* cys); 6,55 (d, J = 7,2 Hz, 1H, N*H* cys); 6,92-7,01 (m, 1H, N*H*CH₂); 7,39-7,50, 7,53-7,64, 7,91-7,99 (3m, 10H, *H* aromatiques).
SM : (FAB⁺/G-T) *m*/*z* 917 (2M+H)⁺, 459 (M+H)⁺.

| Analyse : C₂₃H₂₆N₂O₄S₂ (458) | | | |
|---|---|---|---|
| Calc. % | C 60,26 | H 5,68 | N 6,11 |
| Tr. % | 60,22 | 5,67 | 6,00 |

### 2.45. N-(NACETYL-S-TRITYL-L-CYSTEINYL)THIAZOLIDINE (18)

La réaction de couplage de **7** (2 mmoles) avec la thiazolidine est réalisée selon la méthode A décrite dans la première voie de synthèse (exemple 1). Après retour à la température ambiante, l'agitation est poursuivie 12 h. Le milieu réactionnel est alors dilué avec 50 ml d'AcOEt puis lavé (eau, 70 ml; bicarbonate de sodium saturé et glacé, 50 ml; eau, 2 x 50 ml), séché sur sulfate de sodium et évaporé à sec sous vide. La mousse incolore obtenue est ensuite purifiée par par "flash" chromatographie sur colonne de gel de silice (éluant : AcOEt/éther de pétrole 30%). On isole **18,** sous forme d'une gomme incolore, avec un rendement de 80%. R_{f} (AcOEt/éther de pétrole, 8 : 2) : 0,40. Cristallise du MeOH en aiguilles incolores. F = 197-198°C. [α]_{D}²⁰ = +0,86° (c 1,16; CHCl₃).
RMN ¹H (CDCl₃) δ ppm (mélange isomérique, 5,2 : 4,8): 1,95 (s, 3H, NCOC*H*_{*3*}); 2,48-2,66 (m, 2H, C*H*_{*2*} cys); 2,88-3,02 (m, 2H, *H*5, *H*5' Thz); 3,24-3,34, 3,64-3,75, 3,77-3,86 (3m, 2H, *H*4, *H*4' Thz); 3,96, 4,41 et 4,45, 4,54 ( 2 x 2d, J = 2 x 8,8 et 2 x 10,3 Hz, 2H, *H*2, *H*2' Thz), 4,60-4,69 (m, 1H, α *H* cys); 6,05-6,15 (m, 1H, N*H* cys); 7,18-7,34, 7,36-7,44 (2m, 15H, *H* aromatiques).
SM : (FAB⁺/G-T) *m*/*z* 953 (2M+H)⁺, 477 (M+H)⁺.

| Analyse : C₂₇H₂₈N₂O₂S₂ (476) | | | |
|---|---|---|---|
| Calc. % | C 68,07 | H 5,88 | N 5,88 |
| Tr. % | 67,97 | 5,84 | 5,89 |

### 2.4 6. N-(N-ACETYL-L-CYSTEINYL)THIAZOLIDINE (I-212)

Ce composé est obtenu par la S-détritylation de **18** (0,77 mmoles). Le protocole utilisé est le même que celui décrit dans l'exemple 1 pour la synthèse de la **I-152.** Après une nuit d'agitation à la température ambiante, on obtient une solution. Cette solution est évaporée à sec sous vide et le résidu pâteux obtenu est coévaporé avec du toluène (3 x 5 ml) puis lavé avec 4 x 15 ml d'éther pour fournir le sulfure d'argent correspondant sous forme d'une poudre jaune. Ce sulfure est ensuite traité de la même façon que celle décrite dans l'exemple 1. Après les différents traitements, on recueille une gomme translucide qui est purifiée par "flash" chromatographie sur colonne de gel de silice (éluant : AcOEt). On isole la **I-212,** sous forme d'une gomme incolore, avec un rendement de 64%. R_{f} (AcOEt/MeOH, 9,7 : 0,3) : 0,40. Cristallise dans un mélange d'AcOEt/hexane sous forme d'aiguilles incolores. F = 90-91°C. [α]_{D}²⁰ = -31° (c 1; CHCl₃).
RMN ¹H (CDCl₃) δ ppm (mélange isomérique, 5,8 : 4,2): 1,55 (t app., J = 8,9 Hz, 1H, S*H*); 2,02 (s, 3H, NCOC*H*_{*3*}); 2,75-2,84, 2,85-2,95 (2m, 2H, C*H*_{*2*} cys); 2,99-3,18 (m, 2H, *H*5, *H*5' Thz); 3,79-4,03 (m, 2H, *H*4, *H*4' Thz); 4,57, 4,63 et 4,71 (2d, J = 2 x 10,4 et 1s app., 2H, *H*2, *H*2' Thz), 4,94-5,04 (m, 1H, α *H* cys); 6,41-6,52 (m, 1H, N*H* cys).
SM : (FAB⁺/G-T) *m*/*z* 235 (M+H)⁺.

| Analyse : C₈H₁₄N₂O₂S₂ (234) | | | |
|---|---|---|---|
| Calc. % | C 41,03 | H 5,98 | N 11,97 |
| Tr. % | 41,12 | 6,01 | 11,96 |

### 2.47. N-(N,S-BIS-ACETYL-L-CYSTEINYL)THIAZOLIDINE (I-213)

La S-acylation de la **I-212** (0,26 mmole), avec de l'anhydride acétique, est réalisée selon la méthode générale décrite dans l'exemple 2. Le mélange réactionnel est ensuite traité suivant le protocole décrit pour la synthèse de la **I-177**. Après les différents traitements, on recueille une gomme qui est purifiée par " flash " chromatographie sur colonne de gel de silice (éluant : AcOEt/éther de pétrole 10%). On isole la **I-213** sous forme d'une gomme (Rdt = 80%) qui cristallise dans un mélange d'AcOEt/éther de pétrole en aiguilles incolores. R_{f} (AcOEt) : 0,3. F = 107-108°C. [α]_{D}²⁰ = +6,6° (c 1,36; CHCl₃). RMN ¹H (CDCl₃) δ ppm (mélange isomérique, 5,9 : 4,1): 2,01 (s, 3H, NCOC*H*_{*3*}); 2,36 (s, 3H, SCOC*H*_{*3*}); 2,97-3,20, 3,26-3,29, 3,30-3,33 (3m, 4H, C*H*_{*2*} cys, *H*5, *H*5' Thz); 3,73-3,81, 3,82-3,89, 3,96-4,06 (3m, 2H, H4, *H*4' Thz); 4,49, 4,61, 4,71, 4,81 (2 x 2d, J = 2 x 10,3 et 2 x 8,9 Hz, 2H, *H*2, *H*2' Thz), 4,93-5,04 (m, 1H, a H cys); 6,39-6,50 (m, 1H, N*H* cys).
SM : (FAB⁺/G-T) *m*/*z* 553 (2M+H)⁺, 277 (M+H)⁺.

| Analyse : C₁₀H₁₆N₂O₃S₂ (276) | | | |
|---|---|---|---|
| Calc. % | C 43,48 | H 5,80 | N 10,14 |
| Tr. % | 43,58 | 5,82 | 10,11 |

### EXEMPLE 3: MISE EN EVIDENCE DE L'ACTIVITE ANTIVIRALE DES COMPOSES OBTENUS AUX EXEMPLES 1 et 2.

### 3.1. Préambule

Les manipulations du matériel infectieux ont été réalisées dans un laboratoire de haute sécurité de type L3. De façon à être le plus proche des conditions physiopathologiques, l'ensemble des études a été mené à l'aide de cultures primaires de MDM, de CMSP ou de LSP obtenus à partir de donneurs de sang sains.

Dans toutes les expériences, les effets des nouvelles molécules ont été comparés à ceux des molécules de référence : NAC, ou MEA.

### 3.2. Isolement, culture et activation des cellules

### 3.2.1. Milieux de culture

Le milieu A est composé de milieu de culture cellulaire RPMI 1640 (Life Technologies) supplémenté par 10% de sérum de veau foetal (SVF, Boehringer Mannheim) décomplémenté par la chaleur à 56 °C pendant 30 min, de 2 mM de L-Glutamine (Boehringer Mannheim), et d'une solution à 100 µg/ml de 3 antibiotiques (pénicilline, streptomycine, néomycine; PSN, Life Technologies). Le milieu B est constitué de milieu A supplémenté par 20 UI/ml d'IL-2 humaine recombinante (Boehringer Mannheim).

### 3.2.2 Isolement des cellules mononuclées du sang périphérique

Les CMSP sont séparées des autres éléments figurés du sang par centrifugation en gradient de ficoll (MSL 2000, Eurobio) : 30 ml de sang, d'un donneur sain, dilués au tiers sont déposés sur un coussin de 20 ml de ficoll. Après 20 min de centrifugation à 850 g, l'anneau de CMSP est prélevé puis lavé 2 fois dans du RPMI 1640, après 10 min de centrifugation à 750 g et 5 min à 400 g.

### 3.2.3. Isolement des monocytes et des lymphocytes

Les monocytes et les lymphocytes sont isolés à partir des CMSP par élutriation à contre-courant selon le protocole décrit par C. Figdor et coll. (*Cell. Biophys.* 1983, 5, 105-118). Les deux populations cellulaires ainsi séparées sont immunophénotypées puis analysées à l'aide d'un cytomètre en flux (FACScan, Becton Dickinson). La pureté des monocytes et des LSP, ainsi obtenus, est supérieure ou égale à 95%.

### 3.2.4. Culture et activation des cellules

Un million de monocytes, dans 1 ml de milieu de culture A, sont répartis dans chaque puits d'une plaque de 48 puits (Becton-Dickinson). Les monocytes sont laissés à différencier en macrophages pendant 7 jours. Les macrophages ainsi différenciés sont maintenus en culture dans du milieu A.

Pour certaines expériences, les CMSP ainsi que les LSP sont activés pendant 48 h par 1 µg/ml d'un mitogène, la PHA-P (Difco Laboratories). Les CMSP et les LSP sont cultivés en milieu A (quiescentes) ou B (activées). Les cellules sont cultivées à 37 °C, en atmosphère saturée en humidité, sous 5% de CO₂. Les surnageants de culture sont prélevés, et les milieux de culture sont renouvelés tous les trois ou quatre jours. A chaque renouvellement des milieux de culture, la viabilité cellulaire est évaluée par une coloration au bleu Trypan ou par une observation microscopique.

### 3.3. Evaluation de l'activité antivirale de la I-152 et de ses dérivés

### 3.3.1. Préparation des composés

Lors de la première série d'évaluation de l'activité antivirale et lors de l'étude du mécanisme d'action de la I-152, la I-152 et les produits de référence ont été solubilisés dans le milieu A. Les molécules ont été resuspendues à des concentrations stock (NAC : 20 mM, MEA et I-152 : 10 mM) et ont été conservées à -80°C. Les dilutions ont ensuite été préparées extemporanément dans du milieu A.

Lors de la deuxième série d'évaluation de l'activité antivirale, la I-152 et ses dérivés (insolubles dans le milieu A), ont été solubilisés dans le DMSO puis dilués dans du milieu A. La concentration en DMSO au cours de cette étude est de 1,5%. Les solutions et les dilutions ont été réalisées extemporanément afin d'éviter ou de réduire l'oxydation de la I-152, par le DMSO, en son disulfure.

### 3.3.2. Virus et infection des cellules

Les MDM ont été infectés par l'isolat de référence à tropisme macrophagique, VIH-1/Ba-L. Les CMSP et les LSP ont été infectés par l'isolat de référence à tropisme lymphocytaire VIH-1-LAI. Les stock viraux ont été constitués en amplifiant *in vitro* ces souches à l'aide de cellules mononucléées du sang ombilical (CMSO) activées préalablement par 1 µg/ml de PHA-P et cultivées dans du milieu A supplémenté par 20 UI/ml d'IL-2. Afin d'éliminer les facteurs solubles tels que les cytokines, les surnageants de culture ont été ultracentrifugés à 360 000 g pendant 5 min, et les culots ont été resuspendus dans du RPMI 1640. Les stocks viraux ainsi constitués ont été ensuite titrés à l'aide de CMSP activées par la PHA-P. Les TCID50 (50% Tissue Culture Infectious Dose) ont été calculées en utilisant la formule de Kärber.

Un million de MDM ont été infectés par 10 000 TCID50 de la souche VIH-1/Ba-L. Cette quantité de virus correspond à une multiplicité d'infection (m.o.i.) égale à 0,01. L'excès de virus est éliminé 24 h après, en lavant les cellules à l'aide de RPMI 1640. Les LSP et les CMSP ont été infectés par 10 000 TCID50 de la souche VIH-1-LAI (moi = 0,01). Les cellules sont lavées au terme du deuxième jour d'infection.

### 3.3.3. Dosage de la réplication virale dans les surnageants de culture

### 3.3.3.1 Dosage de l'activité transcriptase inverse (TI)

La réplication virale est mesurée par le dosage de l'activité TI dans les surnageants de culture selon la technique décrite par F. Rey et coll. (*Biochem. Biophys. Res. Comm.* 1984, 121, 126-133). La radioactivité incorporée lors de l'élongation du brin complémentaire d'une matrice synthétique poly-rA en présence d'une amorce oligo-dT₁₂₋₁₈ et d'un substrat radiomarqué, la [³H-méthyl]thymidine-5'-triphosphate ([³H]TTP), permet de doser l'activité enzymatique de la TI. 400 µl de surnageant sont ultracentrifugés à 360 000 g pendant 5 min. La TI est libérée par la lyse du culot viral dans 20 µl de NTE-Triton (NaCl 100 mM, Tris 10 mM, EDTA 1 mM, Triton X-100 0,1%). Ces 20 µl sont ensuite incubés avec 40 µl du mélange réactionnel suivant : Tris 62,5 mM, pH 7,8; KCI 25 mM; MgCl₂ 6,25 mM; dithiothréitol (DTT) 1,25 mM; poly-rA et oligo-dT₁₂₋₁₈ 2,5 x 10⁻³ UDO, [³H]TTP 5,55 x 10⁻³ TBq. Au bout d'une heure à 37 °C, la réaction enzymatique est arrêtée, et les brins néosynthétisés sont précipités pendant 20 min à 4 °C par l'ajout de 1 ml de pyrophosphate de sodium (PPNa), de 50 µl d'ADN de levure (0,1 mg/ml en acide trichloroacétique (TCA) 5%), et de 4 ml de TCA 20%. Le mélange est filtré au moyen d'une membrane d'acétate de cellulose (Millipore) qui retient les chaînes poly-dT radiomarquées. Le filtre est lavé à l'aide de 20 ml de TCA 5%, l'eau résiduelle est éliminée par ajout de 25 µl d'éthanol à 70%. Le filtre est séché à l'étuve pendant 10 minutes à 80 ° C puis est introduit dans des fioles contenant 8 ml de liquide scintillant. La radioactivité β est quantifiée au moyen d'un compteur à scintillation (Packard Bell). Les résultats sont exprimés en pM de [³H]-TMP incorporées/h/ml de surnageant ou, plus simplement, en cpm/h/ml.

### 3.3.3.2 Dosage de la protéine P25

Le dosage de la protéine p25 est effectué à l'aide de la trousse ELISA de DuPont de Nemours. 200 µl du surnageant de culture à tester sont placés dans un puits d'une plaque de microtitration. L'ajout de 20 µl de tampon de lyse libère les protéines virales dans le milieu. L'antigène libéré se fixe à un anticorps monoclonal de souris anti-p25 immobilisé au fond des puits. Après incubation de 2 h à 37 °C, 3 lavages à l'aide de 5 ml de tampon de lavage sont réalisés puis 100 µl d'un anticorps polyclonal biotinylé réagissant avec l'antigène immobilisé sont incubés pendant 1 h à 37 °C. Une série de 3 lavages dans le même tampon et avec le même volume est de nouveau effectuée avant l'ajout pendant 15 mn à 37 °C de 100 µl de conjugué peroxydase de Raifort-streptavidine qui permettra d'ampliflier la réaction colorimétrique. Le complexe formé est révélé, après 3 lavages à l'aide de 5 ml du tampon de lavage de la trousse, par 100 µl de dichlorhydrate d'ophénylènediamine (OPD). Au terme de 30 min d'incubation à la température ambiante, la réaction est arrêtée par l'ajout de 100 µl d'acide sulfurique 4N. La DO de la coloration ainsi obtenue est lue à 490 nm. Cette absorbance est directement proportionnelle à la quantité d'antigène fixé. La relation linéaire liant la D.O. à la concentration de p25 est établie grâce à une gamme étalon réalisée à partir d'une solution de p25 recombinante.

### 3.3.4. Analyse des résultats et détermination des doses effectrices 50%

Les doses effectrices 50% (DE50) sont calculées à partir des activités TI cumulées en utilisant le logiciel "Dose-effects analysis with microcomputers" mis au point par J. Chou & T.C. Chou.

### 3.3.5. Mesure de la viabilité cellulaire

Ces tests sont systématiquement menés en parallèle de l'évaluation de l'activité antivirale. Compte-tenu du pouvoir oxydo-réducteur des molécules testées, le test au sel de tétrazolium, mesurant l'activité des déshydrogénases mitochondriales n'a pu être utilisé.

### 3.3.5.1. Mesure à l'aide d'un colorant d'exclusion, le bleu Trypan

Les cellules non adhérentes, comme les CMSP et les LSP, sont numérées à liaide d'une cellule de Malassez et d'un colorant d'exclusion, le bleu Trypan (BT). 25 µl de la suspension cellulaire sont ajoutés à 475 µl de BT. Cette numération est effectuée après l'isolement des CMSP et des LSP, avant la mise en plaque, et à chaque renouvellement du milieu de culture.

### 3.3.5.2. Mesure à l'aide d'un colorant vital, le rouge neutre

Le rouge neutre (RN) est un colorant vital qui permet de mesurer la viabilité des cellules adhérentes comme les MDM. 600 µl de surnageant de culture sont éliminés et remplacés par 400 µl d'une solution de RN (0,001% m/v dans du tampon phosphate, PBS, Boehringer Mannhein), filtrée à 0,45 µm. Les cellules sont incubées 1 h à 37 ° C, et sont ensuite lavées (2 x 1 ml de PBS). Les cellules sont alors lysées à -20 °C avec 200 µl d'un mélange d'éthanol à 50% contenant 1% d'acide acétique glacial. La DO est mesurée deux fois sur 100 µl de solution à l'aide d'un spectrophotomètre.

### 3.4. Etude du mécanisme d'action de la I-152

### 3.4.1. Ouantification des ADN proviraux par PCR

La I-152 est constituée de MEA et de NAC qui sont susceptibles d'interagir avec les phases précoces du cycle biologique du VIH. A ce titre, elle est capable de diminuer l'intégration du provirus au sein du génome cellulaire. Afin de mesurer ces effets, les ADN proviraux ont été quantifiés par PCR. Les cellules ont été lysées à l'aide de 1 ml de la solution de lyse suivante: 10 mM de Tris HCl pH 8; 100 mM d'EDTA pH 8; 0,5% de dodecyl sulfate de sodium (SDS); 20 µg/ml de RNase pancréatique bovine DNAse-free. 200 µg/ml de protéinase K sont ensuite ajoutés à cette suspension. Les ADN ont été ensuite extraits à l'aide de 1 ml d'une solution saturée et glacée de phénol, et de 1 ml d'une solution de phénol/Chisam.

Les ADN viraux ont alors été amplifiés au moyen d'amorces spécifiques du gène *gag* (SK01/SK39) et d'une gamme étalon de la lignée 8E5, lignée chroniquement infectée dont les cellules sont porteuses d'une copie provirale. Le gène de la β-globine a été utilisé comme gène rapporteur afin de s'assurer de la qualité de l'extraction d'ADN.

### 3.4.2. Test acellulaire de l'activité enzymatique de la TI

La molécule I-152 est constituée de NAC et de MEA qui sont susceptibles d'inhiber l'activité de la TI (A. Bergamini et coll. *J. Clin. Invest.* 1994, 93, 2251-2257). De ce fait, le pouvoir inhibiteur de la I-152 vis à vis de l'activité TI a été mesuré à l'aide d'un test acellulaire. Ce test a été réalisé selon le protocole décrit précédemment (§ 3.3.3.1.). Dans le mélange réactionnel, seuls les 20 µl d'eau sont remplacés par 20 µl d'une concentration de I-152 ou des composés de référence. L'héparine, connue pour inhiber l'activité de la TI et des autres ADN polymérases, est utilisée comme contrôle positif d'inhibition.

### 3.4.3. Dosage du glutathion total

La méthode de dosage du glutathion (GSH + GSSG) que nous avons utilisée est une adaptation, au système de culture de MDM, de celle décrite par O.W. Griffith et coll. (*Anal Biochem.* 1980, 106, 207-212). Les dosages ont été réalisés 24 h après le début du traitement par les différents composés. Un million de cellules sont lavées trois fois en PBS, puis lysées par 150 µl d'un tampon de lyse (Phosphate 0,1 M pH = 7,4; NaCl 0,15 M; BSA 0,1%; Azide 0,01%; Triton X-100 0,1%; acide 5'-sulfosalycilique 0,05%). La gamme étalon de raison deux s'échelonne de 50 µM à 1,5 nM de GSSG ou de GSH. Le test est réalisé en triplicat. 85 µl de 0,6 mM de NADPH, 25 µl de 6 mM de DTNB, et 130 µl d'eau pure sont rajoutés dans les échantillons. Ces derniers sont incubés à 30°C pendant 10 min. Au moment de la lecture, 20 µl de GSSG réductase à 1 U/ml sont ajoutés dans tous les puits. L'absorbance est mesurée au long cours à 412 nm. La concentration en glutathion total est ensuite déterminée par rapport aux valeurs de la courbe d'étalonnage, réalisée en parallèle du dosage, et par l'extrapolation au niveau de la partie linéaire de la courbe.

### EXEMPLE 4: ACTIVITE ANTI-VIH DE LA I-152 VIS-A-VIS DES MACROPHAGES

### 4.1. Activité antivirale de la I-152 vis-à-vis de MDM infectés extemporanément

### 4.1.1. Doses effectrices 50, 70 et 90% et cytotoxicité de la I-152

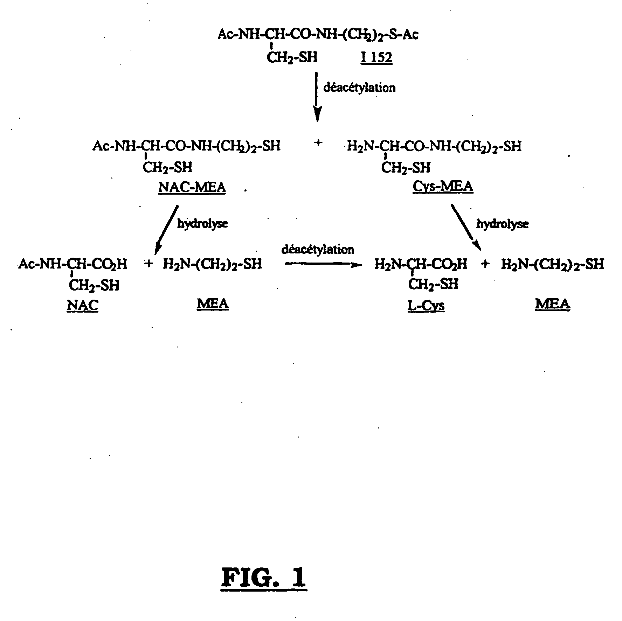

Les cellules de la lignée macrophagique jouent un rôle majeur dans les " process " oxydatifs. La molécule pro-GSH, I-152, a donc été testée vis-à-vis de MDM infectés par la souche VIH-1/Ba-L. La I-152 après métabolisation intracellulaire est susceptible de libérer de la NAC, de la MEA et de la cystéine (figure 1).

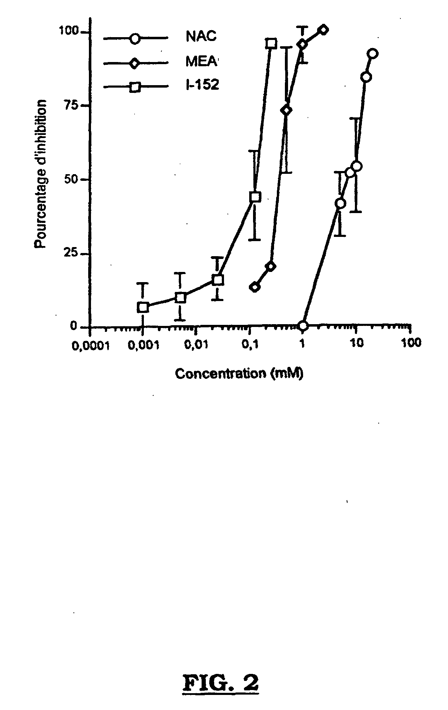

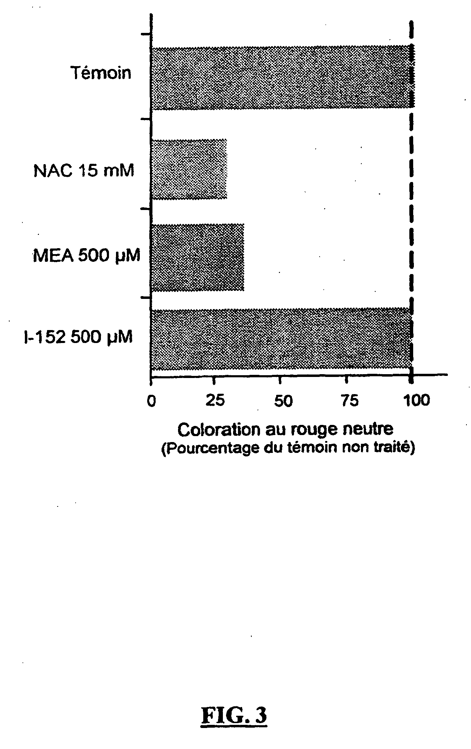

Nous avons comparé les activités de la I-152 à celles de ses deux composantes, la NAC et la MEA, dans notre système expérimental. La I-152 possède une forte activité antivirale, supérieure à celle de la NAC ou de la MEA (Figure 2, Tableau I). Les concentrations inhibitrices de la NAC, de la MEA, et de la I-152 sont respectivement égales à 9,4 mM; 300 µM et 50 µM. A la vue de ces chiffres, la I-152 apparaît donc 6 et 188 fois plus efficace que la MEA et la NAC. Toutefois, ces valeurs ne reflètent pas l'écart entre ces trois produits. En effet, aux doses antivirales, la NAC et la MEA sont cytotoxiques alors que la I-152 ne l'est pas. Ainsi, la NAC est cytotoxique dès 10 ou 15 mM (Figure 3 : NAC 15 mM : 70% de cytotoxicité; NAC 40 mM : 91%), et la MEA diminue la viabilité des MDM de 65% à la concentration de 500 µM (Figure 3). En revanche, la I-152 même à des doses 10 fois supérieures à sa dose effectrice (DE) 50% n'est pas cytotoxique (Figure 3: 500 µM).

### 4.1.1.2. Effet de la I-152 sur la production de la protéine majeure de la nucléocapside virale

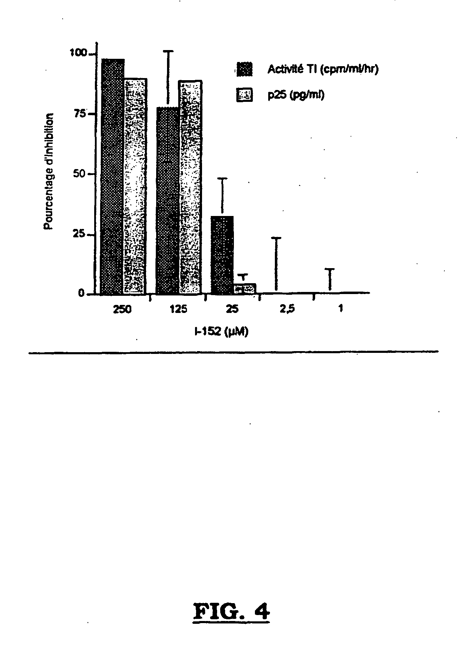

La réplication virale peut être mesurée dans les surnageants de culture en dosant soit l'activité enzymatique de la TI soit la protéine majeure de la nucléocapside virale, p25. Dans les cultures de MDM infectés, l'inhibition de la production de la protéine p25 est concomitante de celle de l'activité TI (Figure 4). Ces résultats confirment donc l'efficacité antivirale de la I-152.

### 4.1.1.3. Effet de la multiplicité d'infection sur l'activité antivirale de la I-152

Au cours de nos premières expériences, les cellules étaient infectées à l'aide de 10 000 TCID50 (m.o.i. : 0,01). Afin de mesurer les effets de la charge virale sur l'activité anti-VIH de la I-152, les cellules ont été infectées, dans un second temps, à l'aide de 1 000 TCID50 (m.o.i. : 0,001).

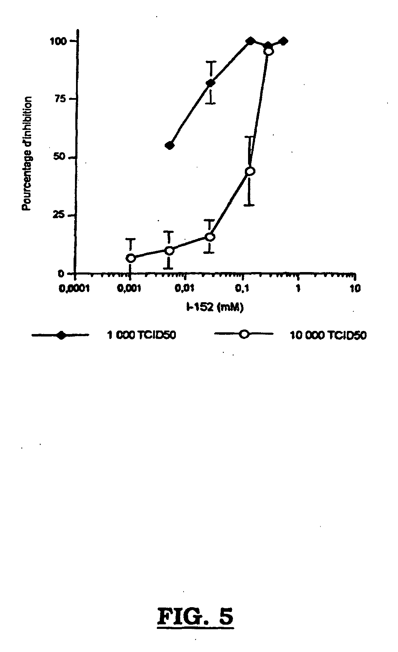

L'activité antivirale de la I-152 augmente lorsque la m.o.i. est diminuée (Figure 5), et la DE s'en trouve diminuée (Tableau II, 3 µM *vs.* 50 µM).

### 4.1.2. Activité antivirale de la I-152 vis-à-vis de MDM préalablement infectés

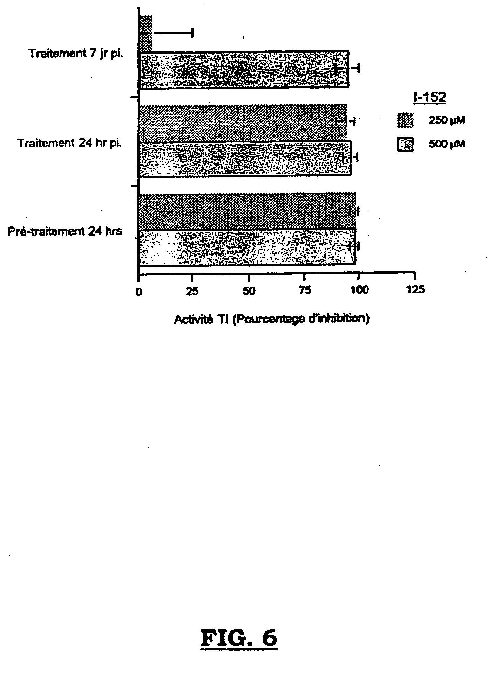

Le traitement des cellules 7 jours après l'infection, confirme l'activité antivirale de la I-152 (Figure 6). En effet, à la dose de 500 µM, la réplication virale est éteinte. Toutefois, dans ces conditions expérimentales, la dose de 250 µM est inefficace. Ce décalage du potentiel inhibiteur, observé entre les différents modes de traitement, suggère 1) que la I-152 inhibe la réplication virale en associant probablement deux mécanismes : un précoce et un tardif, et 2) qu'à forte dose (≥ 500 µM), l'inhibition de la phase tardive du cycle biologique est suffisante.

### 4.2. Activité antivirale de la I-152 dans des cultures primaires de lymphocytes et de cellules mononucléées du sang périphérique

Après avoir montré l'activité antivirale de la I-152 vis-à-vis des cellules de la lignée macrophagique, ses effets ont été mesurés vis-à-vis de lymphocytes du sang périphérique (LSP) et d'une population mixte contenant des monocytes/macrophages et des lymphocytes, les CMSP. De plus, afin de mesurer les effets de l'activation cellulaire sur l'activité antivirale de la I-152, les cellules ont été ou non activées par un mitogène, la phytohæmagglutinine-P (PHA-P).

### 4.2.1. Viabilité des CMSP et des LSP traités par la I-152

La viabilité cellulaire a été mesurée en parallèle à l'estimation de l'activité antivirale. A chaque renouvellement du milieu de culture, les cellules viables ont été numérées au moyen d'un colorant d'exclusion, le Bleu Trypan. Dans les cultures de CMSP et de LSP, la I-152 n'est pas cytotoxique. En effet, la viabilité des lymphocytes n'est pas diminuée dans les cultures de LSP et de CMSP, et, d'autre part dans ces dernières, lorsque les cellules ne sont pas exposées à la PHA-P, les monocytes se différencient en macrophages avec un aspect pseudofibroblastique. En revanche, la NAC et la MEA sont cytotoxiques pour les deux types cellulaires dès les doses de 10 ou 15 mM et 500 µM, respectivement.

### 4.2.2 Activité antivirale de la I-152 vis-à-vis de CMSP quiescentes ou activées par la PHA-P, et infectées

Les CMSP, quiescentes ou activées par la PHA-P, ont été infectées par la souche de référence à tropisme lymphocytaire VIH-1-LAI. Toutes les cultures ont été menées en parallèle, et les drogues ont été maintenues tout au long de la culture.

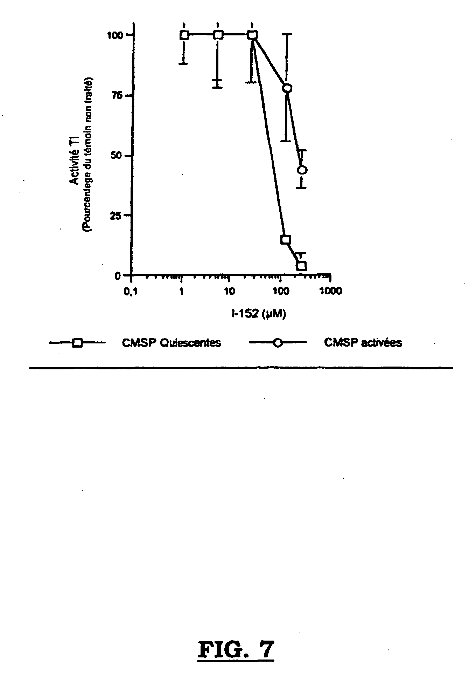

Dans les deux populations cellulaires, la MEA et la NAC n'inhibent pas la réplication virale, aux doses non cytotoxiques (Résultats non présentés). Dans les CMSP quiescentes, la I-152 inhibe la réplication virale de 97 et 88%, respectivement aux doses de 250 et 125 µM et, dans les CMSP activées, la réplication virale est diminuée de 42 et 25% aux mêmes doses (Figure 7).

### 4.2.3. Activité antivirale de la I-152 vis-à-vis de LSP quiescents ou activés par la PHA-P, et infectés par la souche VIH-1-LAI

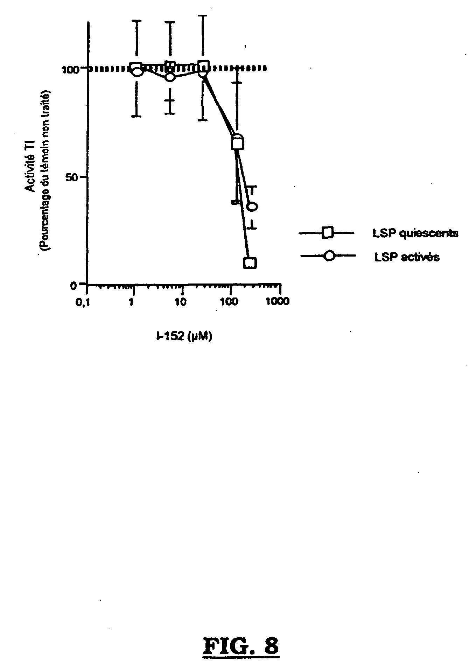

Comme dans les cultures de CMSP, la MEA et la NAC n'ont pas ou peu d'activité antivirale dans les LSP (Résultats non présentés). En revanche, la I-152 inhibe la réplication virale dans les LSP quiescents ou activés par la PHA-P (Figure 8).

### 4.3. Activité anti-VIH de la I-152 vis-à-vis de macrophages tissulaires

### 4.3.1. Isolement des monocytes/macrophages spléniques

La rate est disséquée et tamisée, puis les cellules mononucléées sont isolées par un gradient de densité sur un coussin de Ficoll. Les monocytes/macrophages sont obtenus par adhérence au plastique. Les monocytes ont été laissés à différencier en macrophages pendant 7 jours, date à laquelle ils ont été infectés.

### 4.3.2. Résultats

Le monocyte/macrophage joue un rôle délétère dans la physiopathologie des infections par le VIH parce qu'il constitue un site important de la réplication rétrovirale au niveau des tissus. Parce que ces tissus sont peu accessibles à la thérapeutique anti-VIH actuelle, cette population cellulaire est considérée comme un " réservoir ".

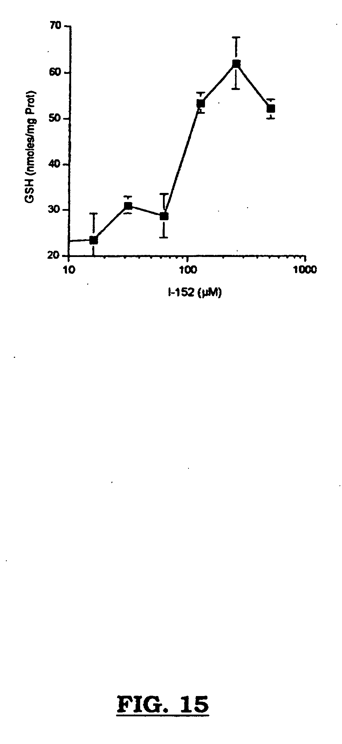

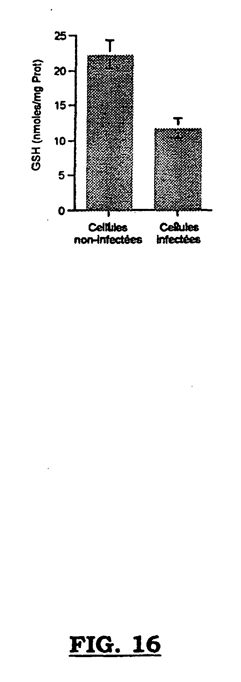

La réplication rétrovirale au sein de cette population cellulaire, et donc l'efficacité d'une molécule comme la I-152, est conditionnée, pour une part, par le niveau de différenciation cellulaire. Il était donc important de s'assurer de l'efficacité antivirale de la I-152 dans des monocytes/macrophages dont le degré de maturation est susceptible d'être différent de celui des MDM. Nous avons donc évalué l'activité antivirale de la I-152 et sa capacité à régénérer le taux intracellulaire de GSH dans des macrophages spléniques. Dans cette population cellulaire, la souche VIH-1/Ba-L se réplique également à haut bruit. La molécule I-152 s'est avérée aussi efficace dans les macrophages spléniques humains que dans les macrophages dérivés des monocytes sanguins. En effet, la concentration de 38 µM diminue de 50% la réplication du VIH dans cette population cellulaire splénique (Tableau III). De même, comme dans les macrophages dérivés des monocytes sanguins, la I-152 augmente le taux intracellulaire de GSH de façon dose-dépendante et saturable à partir de 250 µM (Figure 15). Il est à noter que dans les macrophages spléniques comme dans les macrophages dérivés des monocytes sanguins, l'infection par le VIH engendre un déficit en GSH (MDM; Figure 16).

### 4.4. Mécanisme d'action de la I-152

### 4.4.1. Effet de la I-152 sur l'intégration du génome proviral au sein du génome cellulaire

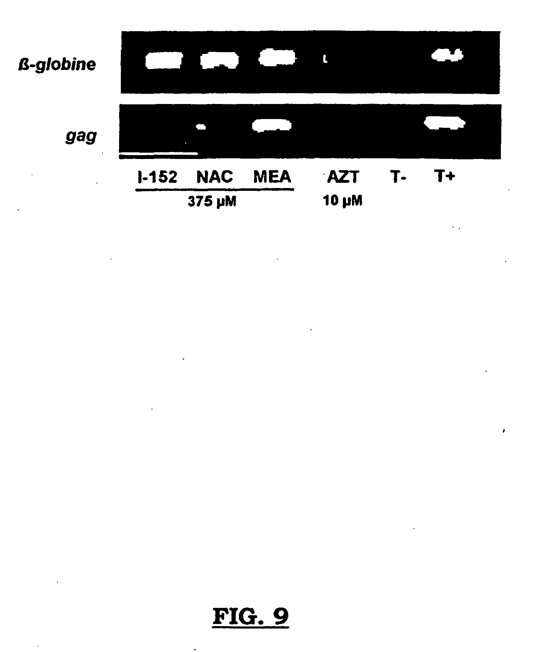

Les expériences de culture cellulaire suggéraient que la I-152 inhibait la réplication du VIH en associant probablement deux mécanismes, l'un précoce et l'autre tardif. Afin de mesurer les effets de cette molécule sur les phases précoces du cycle biologique du VIH, l'intégration du provirus au sein du génome cellulaire a été quantifiée par PCR. La I-152 inhibe l'intégration du provirus. Cette inhibition est largement supérieure à celle induite par la NAC ou la MEA. En revanche, elle n'est pas totale à la différence de celle induite par 10 µM d'AZT (Figure 9).

### 4.4.2. Effet de la I-152 sur l'activité enzymatique de la TI dans un sytème acellulaire

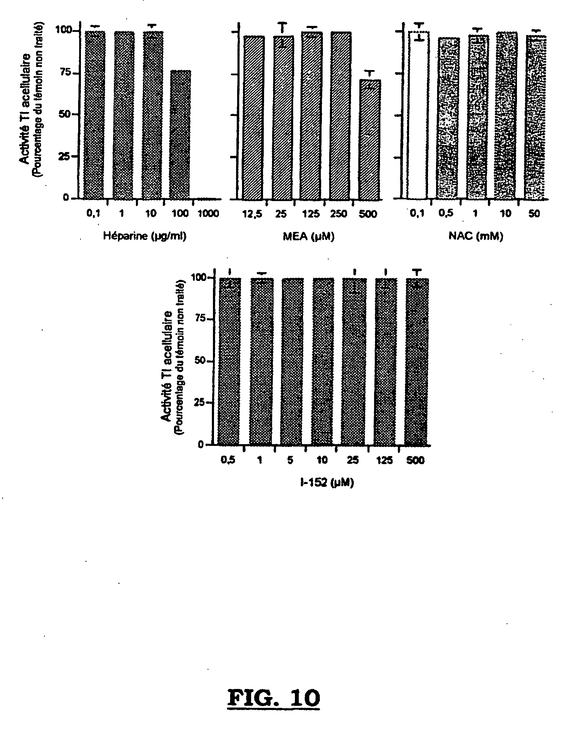

Compte-tenu des résultats précédents et parce que la MEA est susceptible d'inhiber l'activité de la TI, les effets de la I-152 sur l'activité de cette enzyme ont été mesurés dans un système acellulaire. L'expérience a été doublée, et l'héparine a été utilisée comme témoin de l'inhibition de l'activité de la TI. Dans les deux cas, l'héparine à la concentration de 1 mg/ml inhibe totalement l'activité TI de l'isolat VIH-1-LAI. Cette inhibition est dose-dépendante. De même, la MEA diminue de 29 ± 1% (Exp. 1, Figure 10) et 48 ± 4% (Exp. 2 non présentée) à la dose de 500 µM. En revanche, la NAC et la I-152 ne présentent pas d'activité inhibitrice vis-à-vis de la TI du VIH dans notre système expérimental (Figure 10). Ces résultats confirment que la I-152, en libérant la MEA, peut interagir avec une étape précoce du cycle biologique du VIH.

### 4.4.3. Effet de la I-152 sur la concentration intracellulaire du glutathion

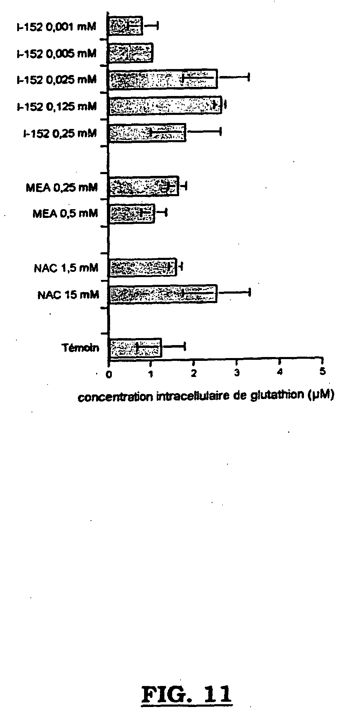

Les effets tardifs de la I-152 sont probablement la conséquence plus directe de son activité pro-glutathion. Afin de s'assurer de cette activité dans les lymphocytes et les macrophages, les concentrations intracellulaires de glutathion total (GSH + GSSG) ont été déterminées dans les cultures de CMSP (Figure 11). Des deux molécules de référence, la NAC possède la meilleure capacité à augmenter le taux intracellulaire de glutathion dans les CMSP quiescentes. En effet, la MEA ne module pas significativement le taux intracellulaire de glutathion, tout du moins aux doses testées. Dans nos expériences, les doses utilisées de NAC sont plus élévées que celles de MEA, ceci peut expliquer la différence observée dans le taux intracellulaire de glutathion.

La I-152 augmente le taux intracellulaire de glutathion. Cette augmentation suit une courbe en cloche avec un optimum à 25 et 125 µM. La dose de 125 µM de I-152 augmente la concentration cellulaire jusqu'à 2,64 ± 0,13 µM. Cette concentration intracellulaire est équivalente à celle obtenue avec la dose de 15 mM de NAC. En considérant les concentrations de molécules supplémentant le milieu de culture, la I-152 est 120 fois plus active que la NAC. Il est à noter que les concentrations intracellulaires de glutathion diminuent aux fortes concentrations, tout du moins pour la MEA (500 µM) et la I-152 (250 µM). Ce phénomène est probablement la conséquence des processus de régulation du glutathion.

### 4.5. Activité antivirale des dérivés S-acylés de la I-152: I-176, I-177, I-178 vis-à-vis des MDM infectés extemporanément

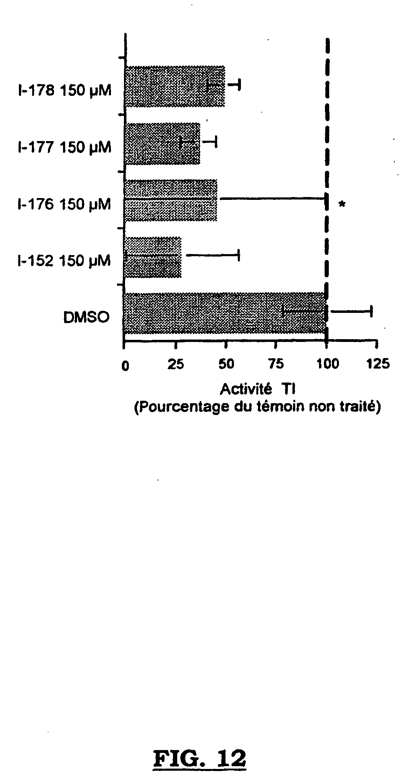

Lors d'une deuxième série d'expériences, les activités antirétrovirales des dérivés de la I-152 ont été testées vis-à-vis des MDM infectés par la souche VIH-1/Ba-L (Figure 12). Ces molécules sont lipophiles et ont donc été solublisées dans du diméthyl sulfoxyde (DMSO) puis diluées. La I-152, servant de référence, a été traitée de la même façon. Dans ces conditions expérimentales, la I-152 s'est avérée la plus efficace à inhiber la réplication virale. Toutefois, il est à noter que le DMSO diminue son efficacité antivirale, et que l'écart type est important car l'activité antivirale a été diminuée sur deux des trois puits du triplicat de culture. Le composé I-176 est légèrement toxique à 150 µM, ceci peut expliquer aussi la variabilité au sein du triplicat de culture et la taille de l'écart-type observé. A ce jour, aucun des dérivés S-acylés n'a montré, *in vitro*, une activité supérieure à celle de la molécule mère. Néanmoins, les I-177 et 178 ont des activités proches de la I-152 et, compte-tenu de l'importance de cette dernière, ces deux molécules, plus lipophiles, sont à retenir. Elle peuvent présenter des avantages dans le cas d'expérimentations cliniques. En effet, elles sont susceptibles d'avoir des formes galéniques et des modes d'administrations différents de ceux de la I-152.

### EXEMPLE 5: EFFET DE LA I-I52 SUR LA CONCENTRATION INTRACELLULAIRE DU GSH

### 5.1. Matériels et méthodes

A l'exception de matériels et méthodes présentés ci-après, les modes opératoires sont semblables à ceux décrits dans les précédents exemples.

Le GSH intracellulaire a été dosé au moyen de la trousse de dosage commercialisée par la Société Cayman. Cette méthode de dosage du glutathion est une adaptation de celle décrite par O.W. Griffith *et al*. (1980).

Le composé I-152 et ses dérivés sont préparés extemporanément en étant solubilisés dans du diméthylsulfoxyde (DMSO) à la concentration de 100 mM. Ils sont ensuite stockés à -20°C après avoir été dilués 10 fois dans du tampon phosphate (PBS).

### 5.2. Résultats

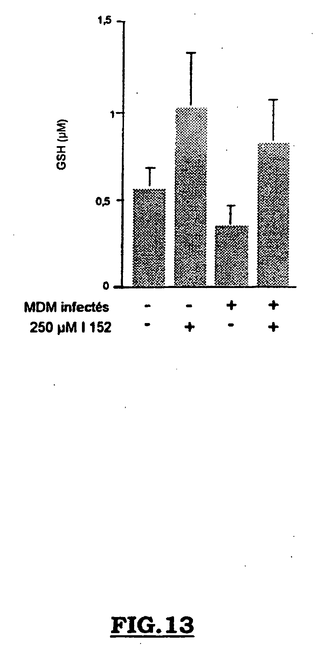

Les effets du composé I-152 à augmenter la concentration intracellulaire de GSH ont été démontrés dans des cellules non exposées à un stress oxydatif. Les résultats présents montrent que cette molécule est également capable de régénérer un taux intracellulaire anormalement bas. En effet, l'infection des macrophages dérivés des monocytes sanguins (MDM) humains engendre un déficit intracellulaire de GSH (Figure 13); ce déficit s'accompagne d'une augmentation de l'expression des enzymes impliquées dans le maintien de ce taux et d'une augmentation du 8 iso-prostane, témoin d'un stress oxydant (résultats non présentés).

### EXEMPLE 6: EFFET DE LA I-152 SUR LA SYNTHESE MACROPHAGIQUE DE TNF-α

### 6.1. Matériels et méthodes

A l'exception de matériels et méthodes présentés ci-après, les modes opératoires sont semblables à ceux décrits dans les précédents exemples.

La synthèse du facteur nécrosant des tumeurs (TNF-α) a été quantifiée dans les surnageants de culture de MDM à l'aide de la trousse colorimétrique commercialisée par la Société Cayman.

Le composé I-152 et ses dérivés sont préparés extemporanément en étant solubilisés dans du diméthylsulfoxyde (DMSO) à la concentration de 100 mM. Ils sont ensuite stockés à -20°C après avoir été dilués 10 fois dans du tampon phosphate (PBS).

### 6.2. Résultats

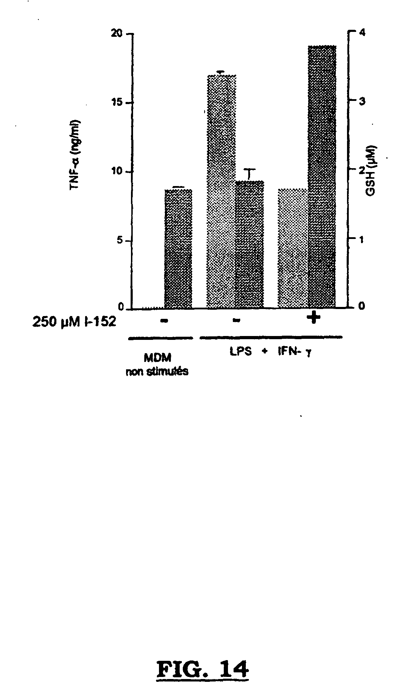

Le facteur nécrosant des tumeurs (TNF)-α comme les éléments radicalaires oxygénés ou azotés joue un rôle majeur dans les processus apoptotiques associés à l'infection par le VIH. De plus, les éléments radicalaires peuvent favoriser la synthèse de cette cytokine pro-inflammatoire. De ce fait, les effets de la I-152 à diminuer la synthèse du TNF-α ont donc été recherchés dans les MDM stimulés par un lipopolysaccharide bactérien et l'interféron (IFN)-γ. Le composé I-152 inhibe la synthèse de TNF-α et augmente la concentration de GSH dans ces conditions expérimentales (Figure 14).

### EXEMPLE 7 : POTENTIALISATION DE L'ACTIVITE ANTI-RETROVIRALE DE L'AZT PAR LE COMPOSE I-152

### 7.1. Matériels et méthodes

A l'exception de matériels et méthodes présentés ci-après, les modes opératoires sont semblables à ceux décrits dans les précédents exemples.

La réplication virale a été mesurée en dosant l'activité transcriptase inverse dans les surnageants de culture à l'aide de la trousse RetroSys de la société Innovagen en respectant les recommandations de la Société.

Le composé I-152 et ses dérivés sont préparés extemporanément en étant solubilisés dans du diméthylsulfoxyde (DMSO) à la concentration de 100 mM. Ils sont ensuite stockés à -20°C après avoir été dilués 10 fois dans du tampon phosphate (PBS).

### 7.2. Résultats

Les composés pro-GSH de la " famille I-152 " sont de préférence administrés comme thérapeutique adjuvante aux antirétroviraux actuels. Les inventeurs ont donc cherché à mesurer *in vitro* les effets de I-152 vis-à-vis de l'efficacité anti-VIH de ces molécules. Cette étude a été réalisée au moyen de macrophages dérivés des monocytes sanguins humains infectés par la souche VIH-1/Ba-L, et selon la méthodologie décrite par J. et TC. Chou pour quantifier les effets synergiques, additifs ou antagonistes entre deux molécules.

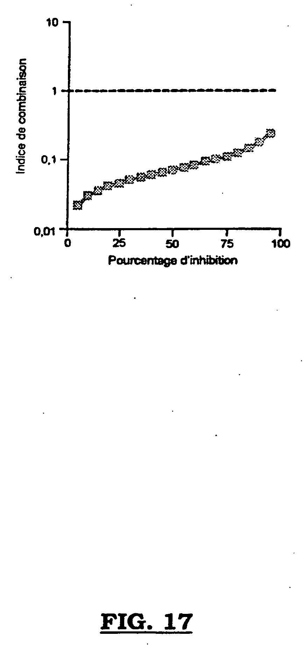

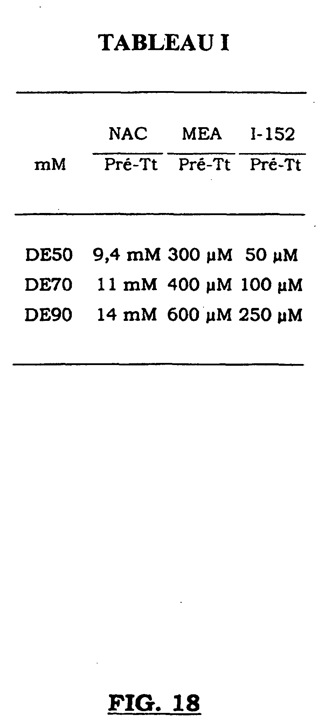

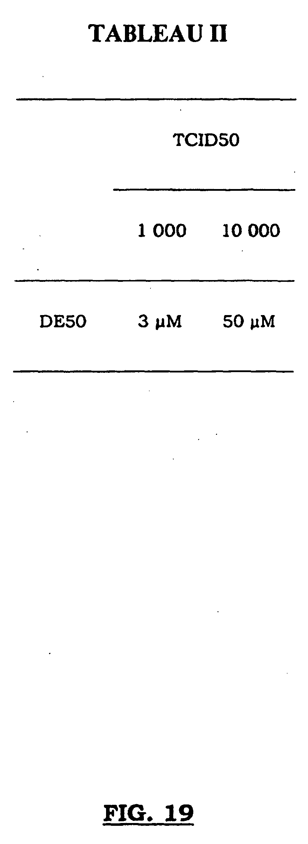

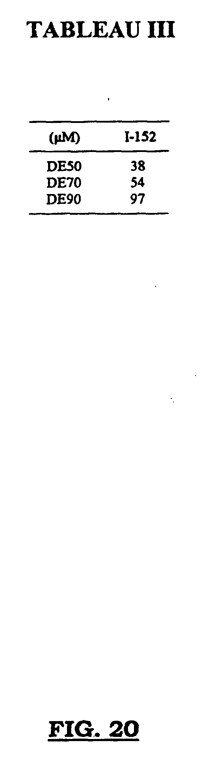

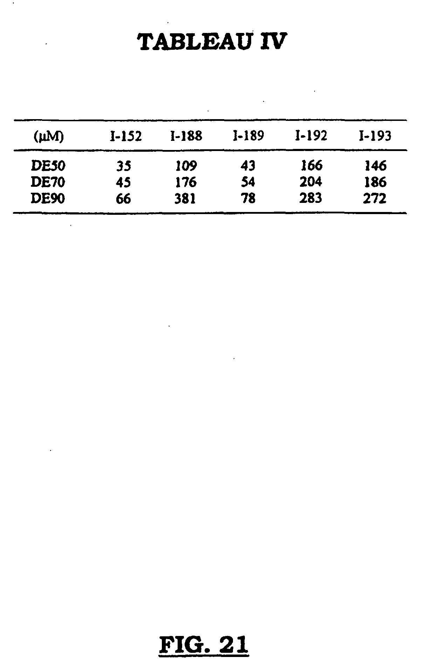

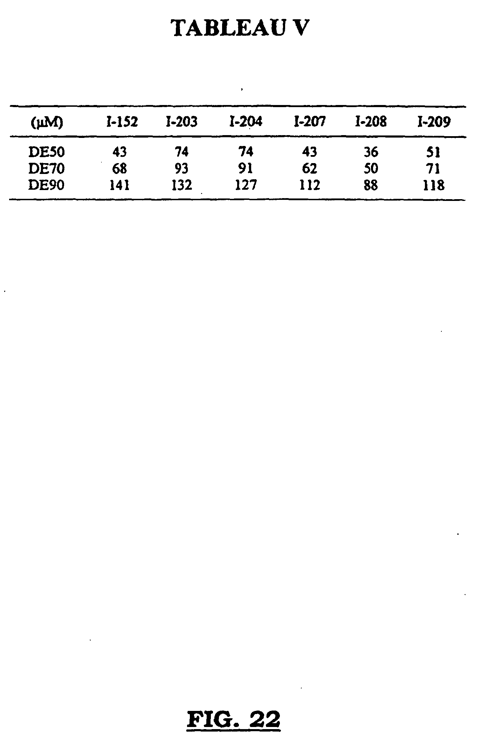

Dans notre modèle expérimental, le composé I-152 potentialise l'activité anti-VIH de l'AZT. En effet, l'indice de combinaison (IC) est inférieur à 1 ce qui témoigne d'une synergie entre les deux composés testés (Figure 17).

### EXEMPLE 8 : ACTIVITE ANTI-RETROVIRALE DES ANALOGUES ACYLES DE LA I-152 OU DE SES DERIVES

### 8.1. Matériels et méthodes

A l'exception de matériels et méthodes présentés ci-après, les modes opératoires sont semblables à ceux décrits dans les précédents exemples.

Les doses effectrices 50% (DE50) sont calculées à partir des activités TI cumulées en utilisant le logiciel "Dose-effects analysis with microcomputers" mis au point par J. Chou & T.C. Chou.

Le composé I-152 et ses dérivés sont préparés extemporanément en étant solubilisés dans du diméthylsulfoxyde (DMSO) à la concentration de 100 mM. Ils sont ensuite stockés à -20°C après avoir été dilués 10 fois dans du tampon phosphate (PBS).

### 8.2. Résultats

L'activité anti-VIH d'une vingtaine de dérivés de la I-152, en plus de celle des composés S-acylés I-176, I-177 et I-178, a été évaluée dans le système de MDM infectés in vitro par la souche VIH-1/Ba-L. Ces composés cherchaient, pour une part, à être plus lipophiles; ils sont ainsi susceptibles de permettre des formes galéniques et des modes d'administrations différents de ceux de I-152. D'autre part, ces dérivés constituent le premier maillon de l'étude de structure-activité qui doit permettre d'identifier les résidus importants dans l'activité de cette famille de composés. Les solutions " stocks " de ces composés ont été soniqués afin d'améliorer la solubilité des produits.

Neuf composés ont démontré une activité antivirale significative du même ordre que celle de la I-152 (Tableaux IV et V). Les valeurs obtenues avec le composé I-152, présentées dans ces deux tableaux, illustrent parfaitement la reproductibilté des effets anti-VIH de la I-152 d'une expérience à l'autre et d'un donneur de cellules à l'autre. En effet, les valeurs présentées dans le tableau III sont issues d'une première expérience réalisée à l'aide des cellules d'un donneur, et celles présentées dans le tableau IV, d'une seconde expérience réalisée à l'aide des cellules d'un autre donneur.

En conclusion, le composé I-152 ou l'un de ses analogues ou de ses dérivés constitue une excellente thérapeutique adjuvante aux antirétroviraux actuels tels que l'AZT et éventuellement aux autres classes de molécules antirétrovirales actuellement utilisées en clinique humaine (i.e. les inhibiteurs non-nucléosidiques de la transcriptase inverse et les inhibiteurs de la protéase virale) en étant capable de réorganiser aussi bien les atteintes du système immunitaire que celles du métabolisme oxydatif. De plus, ces résultats ont mis en évidence les activités biologiques d'autres molécules dérivées et/ou analogues de la I-152 objet de la présente invention; ce qui constitue dorénavant une famille de molécules biologiquement actives et potentiellement utilisables comme thérapie.

La capacité à augmenter le taux intracellulaire de GSH et également à régénérer ce tri-peptide dans des conditions de stress où des molécules de référence comme la NAC et la MEA sont inefficaces, est également très intéressante. Ces puissantes activités, anti-oxydante et probablement anti-apoptotique (si l'on considère que les éléments radicalaires et le TNF-α jouent un rôle majeur dans ces processus délétères), sont en faveur d'un développement du composé I-152 dans d'autres situations non-infectieuses.

### REFERENCES

Abrams, 1991, Am. J. Med., 91, 106-112.
Barnett *et al*., 1969, J. Amer. Chem. Soc., 91, 2358-2369.
Bergamini *et al*., 1994, J. Clin. Invest., 93, 2251-2257.
Bosegaard *et al*., 1993, J. Pharmacol. Exp. Ther. 265, 1239-1244.
Brückner *et al*., 1989, J. Chromatogr. 476, 73-82.
Figdor *et al*., 1983, Cell. Biophys., 5, 105-118.
Griffith *et al*., 1980, Anal Biochem., 106, 207-212.
Heller *et al*., 1997, Advances in Pharmacology, 38, 629-638.
Herzenberg *et al*., 1997, Proc. Natl. Acad. Sci., 94, 1967-1972.
Horowitz, 1991, Am. J. Med., 91, 113-117.
Rabinovitch *et al*., 1992, Diabetologie, 35, 409-413.
Rey *et al*., 1984, Biochem. Biophys. Res. Comm., 121, 126-133.
Volante, 1981, Tetrahedron Lett., 22, 3119-3122.
Wieland et Bokelman, 1952, Ann. Chem. 576, 20-34.
Zee-cheng *et al*., 1970, J. Med. Chem. 13, 414-418.

## Revendications

1. Composés de formule générale: 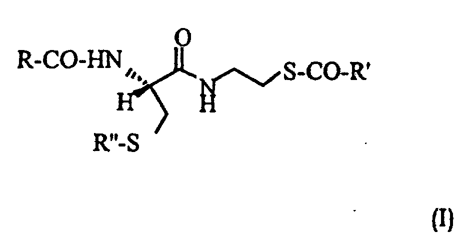 dans laquelle:
- R et R' représentent indépendamment un radical alkyle en C₁-C₇, linéaire ou ramifié, ou un groupe aryle non substitué ou substitué par un ou plusieurs radicaux choisis parmi les halogènes, les radicaux alkyles en C₁-C₃ linéaires ou ramifiés, et les radicaux -OH ;
- R" est l'hydrogène ou un groupe CO-R¹ dans lequel R¹ est un radical alkyle en C₁-C₇ linéaire ou ramifié, ou un groupe aryle non substitué ou substitué par un ou plusieurs radicaux choisis parmi les halogènes, les radicaux alkyles en C₁-C₃ linéaires ou ramifiés, et les radicaux -OH ;
ainsi que les dimères formés par un pont disulfure à partir de l'un et/ou l'autre des deux atomes de soufre des composés de formule générale I constitué par les radicaux R" ou par les radicaux R'CO- des deux molécules ainsi que les formes thiazolidines correspondantes.

2. Composés selon la revendication 1 **caractérisés en ce que** R est un groupe méthyle (-CH₃).

3. Composés selon la revendication 2 **caractérisés en ce que** R' est un groupe méthyle (-CH₃).

4. Composé selon la revendication 3 **caractérisé en ce que** R" est l'hydrogène (Composé N-(N-acétyl-L-cystéinyl)-S-acétylcystéamine).

5. Composé selon la revendication 3 **caractérisé en ce que** R" est un groupe acétyle (-COCH₃) (Composé N-(N,S-Bis-acétyl-L-cystéinyl)-S-acétylcystéamine).

6. Composé selon la revendication 3 **caractérisé en ce que** R" est un groupe isobutyryle (-COCH(CH₃)₂) (Composé N-(N-acétyl-S-isobutyryl-L-cystéinyl)-S-acétylcystéamine).

7. Composé selon la revendication 3 **caractérisé en ce que** R" est un groupe pivaloyle (-COC(CH₃)₃) (Composé N-(N-acétyl-S-pivaloyl-L-cystéinyl)-S-acétylcystéamine).

8. Composé selon la revendication 2 **caractérisé en ce que** R' est sélectionné parmi le groupe isopropyle (-CH(CH₃)₂), tertiobutyle (-C(CH₃)₃) et phényle (-C₆H₅).

9. Composé selon la revendication 8 **caractérisé en ce que** R" est sélectionné parmi l'hydrogène (-H), le groupe acétyle (-COCH₃), le groupe isobutyryle (-COCH(CH₃)₂), le groupe pivaloyle (-COC(CH₃)₃), le groupe benzoyle (-CO-C₆H₅).

10. Composés selon la revendication 1 **caractérisés en ce que** R est un groupe isopropyle (-CH(CH₃)₂).

11. Composé selon la revendication 10 **caractérisé en ce que** R' est sélectionné parmi le groupe méthyle (-CH₃), isopropyle (-CH(CH₃)₂), tertiobutyle (-C(CH₃)) et phényle (-C₆H₅).

12. Composé selon la revendication 11 **caractérisé en ce que** R" est sélectionné parmi l'hydrogène (-H), le groupe acétyle (-COCH₃), le groupe isobutyryle (-COCH(CH₃)₂), le groupe pivaloyle (-COC(CH₃)₃), le groupe benzoyle (-CO-C₆H₅).

13. Composé selon la revendication 8 **caractérisé en ce que** R" est le groupe trityle.

14. Composé selon la revendication 11 **caractérisé en ce que** R" est le groupe trityle.

15. Composé selon la revendication 1 à 14 **caractérisé en ce qu'**il est sous forme thiazolidine.

16. Procédé de préparation d'un composé selon l'une quelconque des revendications 1 à 14 **caractérisé en ce qu'**il comprend les étapes suivantes:
a) Protection de la N-acyl-L-cystéine pour fournir le composé N-acyl-S-trityl-L-cystéine ; puis
b) Couplage de la N-acyl-S-trityl-L-cystéine protégée avec le chlorhydrate de S-acylcystéamine pour fournir le composé N-(N-acyl-S-trityl-L-cystéinyl)-S-acylcystéamine.

17. Procédé de préparation d'un composé selon la revendication 15 **caractérisé en ce qu'**il comprend les étapes suivantes :
a) Protection de la N-acyl-L-cystéine pour fournir le composé N-acyl-S-trityl-L-cystéine ; puis
b) Couplage de la N-acyl-S-trityl-L-cystéine protégée avec la thiazolidine.

18. Procédé selon les revendications 16 et 17 pour la préparation d'un composé selon les revendications 1 à 12 et 15 **caractérisé en ce qu'**il comprend en outre les étapes suivantes:
c) Déprotection dudit composé obtenu à ladite étape b) ; puis
d) Libération du thiol libre de formule I.

19. Procédé selon les revendications 16 et 18 pour la préparation du composé selon la revendication 4 **caractérisé en ce que** ledit composé N-acyl-S-trityl-L-cystéine de l'étape a) est le composé N-acétyl-S-trityl-L-cystéine et que ledit chlorhydrate de S-acylcystéamine de l'étape b) est le chlorhydrate de S-acétylcystéamine.

20. Procédé selon la revendication 18 pour la préparation d'un composé selon les revendications 1 à 3, 5 à 12 et 15 **caractérisé en ce qu'**il comprend en outre une étape e) de S-acylation.

21. Procédé de préparation d'un composé selon la revendication 1 dans lequel R = R' et R" est un hydrogène et **caractérisé en ce qu'**il comprend les étapes suivantes:
a) Estérification de la fonction carboxylique de la N-Boc-L-sérine (1) par la N-hydroxysuccinimide dans du N, N-diméthylformamide (DMF) en présence de 1,3-dicyclohexylcarbodiimide (DCC) pour former l'ester actif (1'); puis,
b) Condensation *in situ* de l'ester actif formé (1') avec l'éthanolamine (2) pour fournir le composé N-(N-Boc-L-séryl)-2-amirioéthanol (3); puis,
c) Réaction de Mitsunobu sur le composé (3) avec de la triphénylphosphine et du diisopropyl azodicarboxylate en présence d'acide thiocarboxylique dans le tétrahydrofuranne pour fournir le composé N-(N-Boc-S-acyl-L-cystéinyl)-S-acylcystéamine (4); puis,
d) Déprotection du composé (4) avec l'acide trifluoroacétique.

22. Procédé selon la revendication 21 pour la préparation du composé selon la revendication 4 **caractérisé en ce que** ledit acide thiocarboxylique de l'étape c) est l'acide thioacétique.

23. Procédé de préparation des composés selon les revendications 1 à 3, 5 à 12 par S-acylation du composé de la revendication 4 en solution dans la pyridine en présence d'un anhydride R₂O ou d'un chlorure d'acide R-Cl **caractérisé en ce que** R est choisi dans le groupe CO-R¹ dans lequel R¹ est un radical alkyle en C1-C7, linéaire ou ramifié ou un groupe aryle substitué ou non par un ou plusieurs atomes d'halogènes.

24. Procédé de préparation du composé selon la revendication 5 par S-acylation du composé de la revendication 4 en solution dans la pyridine en présence d'anhydride acétique.

25. Procédé de préparation du composé selon la revendication 6 par S-acylation du composé de la revendication 4 en solution dans la pyridine en présence de chlorure d'isobutyryle.

26. Procédé de préparation du composé selon la revendication 7 par S-acylation du composé de la revendication 4 en solution dans la pyridine en présence de chlorure de pivaloyle.

27. Précurseur d'un composé intervenant dans la voie de biosynthèse du glutathion **caractérisé en ce qu'**il s'agit d'un composé selon l'une quelconque des revendications 1 à 15.

28. Utilisation des composés selon l'une quelconque des revendications 1 à 15 pour la préparation d'un produit intervenant dans la voie de biosynthèse du glutathion.

29. Utilisation selon la revendication 28 **caractérisée en ce que** ledit produit est choisi dans le groupe formé par la N-acétyl-L-cystéine, la cystéamine, la L-cystéine.

30. Utilisation d'un composé selon l'une quelconque des revendications 1 à 15 en tant qu'agent antioxydant.

31. Composé selon l'une des revendications 1 à 15 à titre de médicament.

32. Composé selon l'une des revendications 1 à 15 à titre de médicament destiné à augmenter le taux intracellulaire et/ou extracellulaire de glutathion.

33. Utilisation d'un composé selon l'une quelconque des revendications 1 à 15 pour la préparation de médicament destiné à augmenter le taux intracellulaire et/ou extracellulaire de glutathion.

34. Composition pharmaceutique, notamment dermatologique, **caractérisée en ce qu'**elle comprend une quantité efficace d'un composé selon l'une des revendications 1 à 15 et un véhicule pharmaceutiquement acceptable.

35. Utilisation d'un composé selon l'une des revendications 1 à 15 pour la préparation d'un médicament ou d'une composition pharmaceutique selon la revendication 34 pour le traitement et/ou la prévention de pathologies ou de troubles liés à une déplétion en glutathion intra et/ou extracellulaire.

36. Utilisation selon la revendication 35 **caractérisée en ce que** lesdites pathologies sont choisies parmi les infections virales, les infections bactériennes, les infections parasitaires, les maladies du tractus respiratoire, les maladies neurodégénératives, les maladies auto-immunes, les maladies cardio-vasculaires, les cancers, les maladies du système immunitaire, le diabète, et de préférence le diabète de type I, les pathologies ophtalmiques, les maladies dermatologiques.

37. Utilisation selon la revendication 36 **caractérisée en ce que** lesdites pathologies sont les infections virales.

38. Utilisation selon les revendications 36 et 37 **caractérisée en ce que** lesdites infections virales sont des infections causées par les virus à ADN et les virus à ARN, plus particulièrement par les virus rétroïdes, plus particulièrement le virus de l'immunodéficience humaine (VIH) et de manière préférée le virus de l'immunodéficience humaine de type 1 (VIH-1).

39. Utilisation selon les revendications 36 à 38 **caractérisée en ce que** ledit composé est le composé selon la revendication 4.

40. Utilisation selon les revendications 36 à 38 **caractérisée en ce que** ledit composé est le composé selon l'une des revendications 5 à 15.

41. Composition pharmaceutique pour le traitement préventif et curatif du SIDA et des atteintes tissulaires associées **caractérisée en ce qu'**elle contient une quantité thérapeutiquement efficace d'un composé selon les revendications 1 à 15 et un véhicule pharmaceutiquement acceptable.

42. Produit comprenant au moins un composé selon l'une des revendications 1 à 15 et au moins un inhibiteur de la transcriptase inverse comme produit de combinaison pour une utilisation simultanée, séparée ou étalée dans le temps en thérapie antivirale.

43. Produit selon la revendication 42, **caractérisée en ce que** ledit inhibiteur de la transcriptase inverse est choisi par exemple parmi le 3'-azido-3'désoxythymidine (AZT), le 2',3'-didésoxyinosine (ddI), le 2',3'-didésoxycytidine (ddC), le (-)2',3'-didésoxy-3'-thiacytidine (3TC), le 2',3'-didéshydro-2',3'didésoxythymidine (d4T) et le (-)2'-désoxy-5-fluoro-3'-thiacytidine (FTC), le TIBO, le HEPT, le TSAO, l'α-APA, la névirapine, le BAHP, l'acide phosphonoformique (PFA).

44. Utilisation selon la revendication 35 **caractérisée en ce que** lesdites pathologies sont des maladies cardio-vasculaires choisies de préférence parmi le groupe composé de l'hypertension artérielle, de l'artériosclérose, des ischémies cérébrales, des ischémies cardiaques, des arythmies ventriculaires, des fibrillations ventriculaires, de l'infarctus du myocarde.

45. Utilisation selon la revendication 35 **caractérisée en ce que** lesdites pathologies sont les pathologies ophtalmiques, notamment la cataracte.

46. Utilisation selon la revendication 35 **caractérisée en ce que** lesdites pathologies sont des maladies du tractus respiratoire, notamment l'emphysème pulmonaire, la fibrose pulmonaire idiopathique, la mucoviscidose, la bronchite chronique, la bronchite aiguë, le syndrome de détresse respiratoire de l'adulte.

47. Utilisation d'un composé selon l'une quelconque des revendications 1 à 15 pour la préparation d'un médicament ou d'une composition pharmaceutique selon la revendication 34 destinés au traitement préventif et/ou curatif des pertes auditives liées au bruit.

48. Utilisation d'un composé selon l'une quelconques des revendications 1 à 15 pour la préparation d'un médicament ou d'une composition pharmaceutique selon la revendication 34 destinés au traitement des empoisonnements liés à l'administration par voie orale ou parentérale, en surdose ou non, de substances choisies de préférence parmi le groupe composé de l'acétaminophène, les nitrites, l'éthanol, l'acrylonitrile, les métaux lourds et plus particulièrement l'or, l'argent, le mercure.

49. Utilisation selon la revendication 30 d'un composé selon l'une des revendications 1 à 15 dans le domaine de la cosmétique.

50. Utilisation selon la revendication 49 pour:
(i) prévenir, effacer et traiter les rides, les ridules de la peau; et/ou
(ii) lutter contre le relâchement cutané et/ou sous cutané; et/ou
(iii) améliorer la texture de la peau et raviver l'éclat de la peau; et/ou
(iv) éliminer les poils indésirés de la peau; et/ou
(v) diminuer les tailles des pores de la peau; et/ou
(vi) déformer en permanence les cheveux.

51. Composition cosmétique pour le traitement de la peau et/ou des cheveux et/ou des poils **caractérisée en ce qu'**elle contient un composé selon l'une des revendications 1 à 15 et un excipient cosmétiquement acceptable.

52. Procédé de traitement cosmétique de la peau pour prévenir, effacer et traiter les rides, les ridules de la peau et/ou lutter contre le relâchement cutané et/ou sous cutané et/ou améliorer la texture de la peau et raviver l'éclat de la peau et/ou éliminer les poils indésirés de la peau et/ ou diminuer les tailles des pores de la peau comprenant l'application sur la peau d'une composition cosmétique selon la revendication 51.

53. Procédé de traitement cosmétique des cheveux pour la déformation permanente des cheveux, comprenant l'application sur les cheveux d'une composition cosmétique selon la revendication 51.

54. Utilisation selon la revendication 30 dans l'industrie agroalimentaire et notamment pour la conservation des propriétés organoleptiques et nutritionnelles des boissons notamment des jus de fruit et/ou des aliments.

## Patentansprüche

1. Verbindungen der allgemeinen Formel 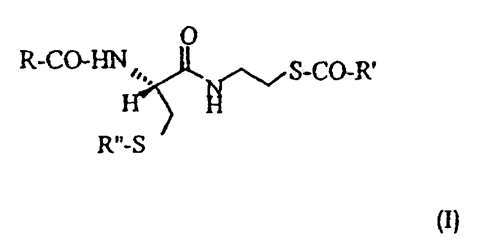 in welcher:
- R und R' unabhängig für einen linearen oder verzweigten C₁-C₇-Alkylrest oder eine nicht substituierte oder durch einen oder mehrere Reste, ausgewählt unter den Halogenen, den linearen oder verzweigten C₁-C₃-Alkylresten und den Resten -OH, substituierte Arylgruppe stehen;
- R" Wasserstoff oder eine Gruppe CO-R¹ ist, in welcher R¹ ein linearer oder verzweigter C₁-C₇-Alkylrest oder eine nicht substituierte oder durch einen oder mehrere Reste, ausgewählt unter den Halogenen, den linearen oder verzweigten C₁-C₃-Alkylresten und den Resten -OH, substituierte Arylgruppe ist;
sowie die durch eine Disulfidbrücke ausgehend von dem einen und/oder dem anderen der beiden Schwefelatome der Verbindungen der allgemeinen Formel I, gebildet durch die Reste R" oder durch die Reste R'CO- der beiden Moleküle, gebildeten Dimere sowie die entsprechenden Thiazolidin-Formen.

2. Verbindungen nach Anspruch 1, **dadurch gekennzeichnet, dass** R eine Methylgruppe (-CH₃) ist.

3. Verbindungen nach Anspruch 2, **dadurch gekennzeichnet, dass** R' eine Methylgruppe (-CH₃) ist.

4. Verbindung nach Anspruch 3, **dadurch gekennzeichnet, dass** R'' Wasserstoff ist (Verbindung N-(N-Acetyl-L-cysteinyl)-Sacetylcysteamin).

5. Verbindung nach Anspruch 3, **dadurch gekennzeichnet, dass** R'' eine Acetylgruppe (-COCH₃) ist (Verbindung N-(N,S-Bisacetyl-L-cysteinyl)-S-acetylcysteamin).

6. Verbindung nach Anspruch 3, **dadurch gekennzeichnet, dass** R'' eine Isobutyrylgruppe (-COCH(CH₃)₂) ist (Verbindung N-(N-Acetyl-S-isobutyryl-L-cysteinyl)-S-acetylcysteamin).

7. Verbindung nach Anspruch 3, **dadurch gekennzeichnet, dass** R'' eine Pivaloylgruppe (-COC(CH₃)₃) ist (Verbindung N-(N-Acetyl-S-pivaloyl-L-cysteinyl)-S-acetylcysteamin).

8. Verbindung nach Anspruch 2, **dadurch gekennzeichnet, dass** R' unter der Isopropylgruppe (-CH(CH₃)₂), der tert.-Butylgruppe (-C(CH₃)₃) und der Phenylgruppe (-C₆H₅) ausgewählt wird.

9. Verbindung nach Anspruch 8, **dadurch gekennzeichnet, dass** R" unter Wasserstoff (-H), der Acetylgruppe (-COCH₃), der Isobutyrylgruppe (-COCH(CH₃)₂), der Pivaloylgruppe (-COC(CH₃)₃), der Benzoylgruppe (-CO-C₆H₅) ausgewählt wird.

10. Verbindungen nach Anspruch 1, **dadurch gekennzeichnet, dass** R eine Isopropylgruppe (-CH(CH₃)₂) ist.

11. Verbindung nach Anspruch 10, **dadurch gekennzeichnet, dass** R' unter der Methylgruppe (-CH₃), Isopropylgruppe (-CH(CH₃)₂), tert.-Butylgruppe (-C(CH₃)₃) und Phenylgruppe (-C₆H₅) ausgewählt wird.

12. Verbindung nach Anspruch 11, **dadurch gekennzeichnet, dass** R'' unter Wasserstoff (-H), der Acetylgruppe (-COCH₃), der Isobutyrylgruppe (-COCH(CH₃)₂), der Pivaloylgruppe (-COC(CH₃)₃), der Benzoylgruppe (-CO-C₆H₅) ausgewählt wird.

13. Verbindung nach Anspruch 8, **dadurch gekennzeichnet, dass** R'' die Tritylgruppe ist.

14. Verbindung nach Anspruch 11, **dadurch gekennzeichnet, dass** R'' die Tritylgruppe ist.

15. Verbindung nach Anspruch 1 bis 14, **dadurch gekennzeichnet, dass** sie in Thiazolidin-Form vorliegt.

16. Verfahren zur Herstellung einer Verbindung nach einem der Ansprüche 1 bis 14, **dadurch gekennzeichnet, dass** es die folgenden Schritte umfasst:
a) Schützen von N-Acyl-L-cystein, um die Verbindung N-Acyl-S-trityl-L-cystein bereitzustellen; dann
b) Koppeln des geschützten N-Acyl-S-trityl-L-cysteins mit S-Acylcysteaminhydrochlorid, um die Verbindung N-(N-Acyl-S-trityl-L-cysteinyl)-S-acylcysteamin bereitzustellen.

17. Verfahren zur Herstellung einer Verbindung nach Anspruch 15, **dadurch gekennzeichnet, dass** es die folgenden Schritte umfasst:
a) Schützen von N-Acyl-L-cystein, um die Verbindung N-Acyl-S-trityl-L-cystein bereitzustellen; dann
b) Koppeln des geschützten N-Acyl-S-trityl-L-cysteins mit Thiazolidin.

18. Verfahren nach den Ansprüchen 16 und 17 zur Herstellung einer Verbindung nach den Ansprüchen 1 bis 12 und 15, **dadurch gekennzeichnet, dass** es außerdem die folgenden Schritte umfasst:
c) Entfernen der Schutzgruppe von der in dem Schritt b) erhaltenen Verbindung; dann
d) Freisetzung des freien Thiols der Formel I.

19. Verfahren nach den Ansprüchen 16 und 18 zur Herstellung der Verbindung nach Anspruch 4, **dadurch gekennzeichnet, dass** die Verbindung N-Acyl-S-trityl-L-cystein des Schritts a) die Verbindung N-Acetyl-S-trityl-L-cystein ist und dass das S-Acylcysteaminhydrochlorid des Schritts b) S-Acetylcysteaminhydrochlorid ist.

20. Verfahren nach Anspruch 18 zur Herstellung einer Verbindung nach den Ansprüchen 1 bis 3, 5 bis 12 und 15, **dadurch gekennzeichnet, dass** es außerdem einen Schritt e) einer S-Acylierung umfasst.

21. Verfahren zur Herstellung einer Verbindung nach Anspruch 1, in welcher R = R' und R" ein Wasserstoff ist, und welches **dadurch gekennzeichnet ist, dass** es die folgenden Schritte umfasst:
a) Veresterung der Carboxylfunktion von N-Boc-L-serin (1) durch N-Hydroxysuccinimid in N,N-Dimethylformamid (DMF) in Gegenwart von 1,3-Dicyclohexylcarbodiimid (DCC), um den aktiven Ester (1') zu bilden; dann
b) *in situ*-Kondensation des gebildeten aktiven Esters (1') mit Ethanolamin (2), um die Verbindung N-(N-Boc-L-seryl)-2-aminoethanol (3) bereitzustellen; dann
c) Mitsunobu-Reaktion an der Verbindung (3) mit Triphenylphosphin und Diisopropylazodicarboxylat in Gegenwart von Thiocarbonsäure in Tetrahydrofuran, um die Verbindung N-(N-Boc-S-acyl-L-cysteinyl)-S-acylcysteamin (4) bereitzustellen; dann
d) Entfernung der Schutzgruppe von der Verbindung (4) mit Trifluoressigsäure.

22. Verfahren nach Anspruch 21 zur Herstellung der Verbindung nach Anspruch 4, **dadurch gekennzeichnet, dass** die Thiocarbonsäure des Schritts c) Thioessigsäure ist.

23. Verfahren zur Herstellung der Verbindungen nach den Ansprüchen 1 bis 3, 5 bis 12 durch S-Acylierung der Verbindung von Anspruch 4 in Lösung in Pyridin in Gegenwart eines Anhydrids R₂O oder eines Säurechlorids R-Cl, **dadurch gekennzeichnet, dass** R in der Gruppe CO-R¹, in welcher R¹ ein linearer oder verzweigter C₁-C₇-Alkylrest oder eine durch ein oder mehrere Halogenatome substituierte oder nicht substituierte Arylgruppe ist, ausgewählt wird.

24. Verfahren zur Herstellung der Verbindung nach Anspruch 5 durch S-Acylierung der Verbindung von Anspruch 4 in Lösung in Pyridin in Gegenwart von Essigsäureanhydrid.

25. Verfahren zur Herstellung der Verbindung nach Anspruch 6 durch S-Acylierung der Verbindung von Anspruch 4 in Lösung in Pyridin in Gegenwart von Isobutyrylchlorid.

26. Verfahren zur Herstellung der Verbindung nach Anspruch 7 durch S-Acylierung der Verbindung von Anspruch 4 in Lösung in Pyridin in Gegenwart von Pivaloylchlorid.

27. Vorstufe einer Verbindung, welche in den Biosyntheseweg von Glutathion Eingang findet, **dadurch gekennzeichnet, dass** es sich um eine Verbindung nach einem der Ansprüche 1 bis 15 handelt.

28. Verwendung der Verbindungen nach einem der Ansprüche 1 bis 15 zur Herstellung eines Produkts, welches in den Biosyntheseweg von Glutathion Eingang findet.

29. Verwendung nach Anspruch 28, **dadurch gekennzeichnet, dass** das Produkt in der aus N-Acetyl-L-cystein, Cysteamin, L-Cystein gebildeten Gruppe ausgewählt wird.

30. Verwendung einer Verbindung nach einem der Ansprüche 1 bis 15 als Antioxidationsmittel.

31. Verbindung nach einem der Ansprüche 1 bis 15 als Arzneimittel.

32. Verbindung nach einem der Ansprüche 1 bis 15 als Arzneimittel, welches dazu bestimmt ist, den intrazellulären und/oder extrazellulären Gehalt von Glutathion zu erhöhen.

33. Verwendung einer Verbindung nach einem der Ansprüche 1 bis 15 zur Herstellung eines Arzneimittels, welches dazu bestimmt ist, den intrazellulären und/oder extrazellulären Gehalt von Glutathion zu erhöhen.

34. Pharmazeutische, insbesondere dermatologische Zusammensetzung, **dadurch gekennzeichnet, dass** sie eine wirksame Menge einer Verbindung nach einem der Ansprüche 1 bis 15 und einen pharmazeutisch annehmbaren Träger umfasst.

35. Verwendung einer Verbindung nach einem der Ansprüche 1 bis 15 zur Herstellung eines Arzneimittels oder einer pharmazeutischen Zusammensetzung nach Anspruch 34 für die Behandlung und/oder Verhütung von Pathologien oder Störungen, welche mit einer intra- und/oder extrazellulären Erschöpfung des Glutathions verbunden sind.

36. Verwendung nach Anspruch 35, **dadurch gekennzeichnet, dass** die Pathologien unter den viralen Infektionen, den bakteriellen Infektionen, den Parasiteninfektionen, den Erkrankungen der Atemwege, den neurodegenerativen Erkrankungen, den Autoimmunerkrankungen, den Herz-Kreislauf-Erkrankungen, den Krebserkrankungen, den Erkrankungen des Immunsystems, Diabetes und vorzugsweise Diabetes vom Typ I, den ophthalmischen Pathologien, den dermatologischen Erkrankungen ausgewählt werden.

37. Verwendung nach Anspruch 36, **dadurch gekennzeichnet, dass** die Pathologien virale Infektionen sind.

38. Verwendung nach den Ansprüchen 36 und 37, **dadurch gekennzeichnet, dass** die viralen Infektionen Infektionen sind, die durch DNA-Viren und RNA-Viren, insbesondere durch Retroviren, insbesondere das Human Immunodeficiency Virus (HIV) und bevorzugt das Human Immunodeficiency Virus vom Typ 1 (HIV-1) hervorgerufen werden.

39. Verwendung nach den Ansprüchen 36 bis 38, **dadurch gekennzeichnet, dass** die Verbindung die Verbindung nach Anspruch 4 ist.

40. Verwendung nach den Ansprüchen 36 bis 38, **dadurch gekennzeichnet, dass** die Verbindung die Verbindung nach einem der Ansprüche 5 bis 15 ist.

41. Pharmazeutische Zusammensetzung zur vorbeugenden und heilenden Behandlung von AIDS und damit verbundenen Gewebeerkrankungen, **dadurch gekennzeichnet, dass** sie eine therapeutisch wirksame Menge einer Verbindung nach den Ansprüchen 1 bis 15 und einen pharmazeutisch annehmbaren Träger enthält.

42. Produkt, welches wenigstens eine Verbindung nach einem der Ansprüche 1 bis 15 und wenigstens einen Inhibitor der reversen Transkriptase umfasst, als Kombinationsprodukt für eine gleichzeitige, getrennte oder zeitlich gestaffelte Verwendung bei einer antiviralen Therapie.

43. Produkt nach Anspruch 42, **dadurch gekennzeichnet, dass** der Inhibitor der reversen Transkriptase beispielsweise unter 3'-Azido-3'-desoxythymidin (AZT), 2',3'-Didesoxyinosin (ddI), 2',3'-Didesoxycytidin (ddC), (-)-2',3'-Didesoxy-3'-thiacytidin (3TC), 2',3'-Dideshydro-2',3'-didesoxythymidin (d4T) und (-)-2'-Desoxy-5-fluor-3'-thiacytidin (FTC), TIBO, HEPT, TSAO, α-APA, Nevirapin, BAHP, Phosphonoameisensäure (PFA) ausgewählt wird.

44. Verwendung nach Anspruch 35, **dadurch gekennzeichnet, dass** die Pathologien Herz-Kreislauf-Erkrankungen sind, die vorzugsweise in der Gruppe gebildet aus arterieller Hypertonie, Arteriosklerose, zerebralen Ischämien, Herzischämien, ventrikulären Arrhythmien, Kammerflimmern, Myokardinfarkt ausgewählt werden.

45. Verwendung nach Anspruch 35, **dadurch gekennzeichnet, dass** die Pathologien ophthalmische Pathologien, insbesondere Katarakt sind.

46. Verwendung nach Anspruch 35, **dadurch gekennzeichnet, dass** die Pathologien Erkrankungen der Atemwege, insbesondere Lungenemphysem, idiopathische Lungenfibrose, Mukoviszidose, chronische Bronchitis, akute Bronchitis, "adult respiratory distress syndrome" (ARDS) sind.

47. Verwendung einer Verbindung nach einem der Ansprüche 1 bis 15 zur Herstellung eines Arzneimittels oder einer pharmazeutischen Zusammensetzung nach Anspruch 34, welche für die vorbeugende und/oder heilende Behandlung von mit Lärm verbundenen Hörverlusten bestimmt sind.

48. Verwendung einer Verbindung nach einem der Ansprüche 1 bis 15 zur Herstellung eines Arzneimittels oder einer pharmazeutischen Zusammensetzung nach Anspruch 34, welche für die Behandlung von Vergiftungen, die mit der oralen oder parenteralen, in Form einer Überdosis erfolgenden oder nicht in Form einer Überdosis erfolgenden Verabreichung von Substanzen, vorzugsweise ausgewählt in der Gruppe, gebildet aus Acetaminophen, den Nitriten, Ethanol, Acrylnitril, den Schwermetallen und insbesondere Gold, Silber, Quecksilber, verbunden sind, bestimmt sind.

49. Verwendung nach Anspruch 30 einer Verbindung nach einem der Ansprüche 1 bis 15 auf dem Gebiet der Kosmetik.

50. Verwendung nach Anspruch 49, um:
(i) Falten, Fältchen der Haut zu verhindern, verschwinden zu lassen und zu behandeln; und/oder
(ii) die kutane und/oder subkutane Erschlaffung zu bekämpfen; und/oder
(iii) die Textur der Haut zu verbessern und die Frische der Haut wiederaufleben zu lassen; und/oder
(iv) die unerwünschten Körperhaare der Haut zu entfernen; und/oder
(v) die Größe der Poren der Haut zu verringern; und/oder
(vi) die Kopfhaare permanent zu verformen.

51. Kosmetische Zusammensetzung für die Behandlung der Haut und/oder der Kopfhaare und/oder der Körperhaare, **dadurch gekennzeichnet, dass** sie eine Verbindung nach einem der Ansprüche 1 bis 15 und eine kosmetisch annehmbare Trägersubstanz enthält.

52. Verfahren zur kosmetischen Behandlung der Haut, um Falten, Fältchen der Haut zu verhindern, verschwinden zu lassen und zu behandeln und/oder die kutane und/oder subkutane Erschlaffung zu bekämpfen und/oder die Textur der Haut zu verbessern und/oder die Frische der Haut wiederaufleben zu lassen und/oder die unerwünschten Körperhaare der Haut zu entfernen und/oder die Größe der Poren der Haut zu verringern, welches das Auftragen einer kosmetischen Zusammensetzung nach Anspruch 51 auf die Haut umfasst.

53. Verfahren zur kosmetischen Behandlung der Kopfhaare zur dauerhaften Verformung der Kopfhaare, welches das Auftragen einer kosmetischen Zusammensetzung nach Anspruch 51 auf die Kopfhaare umfasst.

54. Verwendung nach Anspruch 30 in der agrarwirtschaftlichen Industrie und insbesondere für die Bewahrung der organoleptischen und Ernährungs- oder Nährstoffeigenschaften von Getränken, insbesondere Fruchtsäften, und/oder Nahrungsmitteln.

## Claims

1. Compound of general formula: 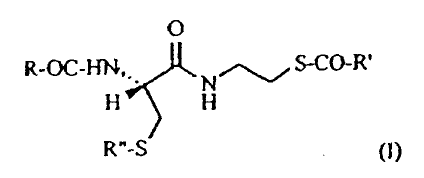 in which:
- R and R' independently represent a linear or branched C₁-C₇ alkyl radical or an aryl group which is unsubstituted or substituted by one or more radicals chosen from halogens, linear or branched C₁-C₃ alkyl radicals and -OH radicals;
- R" is hydrogen or a CO-R¹ group in which R¹ is a linear or branched C₁-C₇ alkyl radical or an aryl group which is unsubstituted or substituted by one or more radicals chosen from halogens, linear or branched C₁-C₃ alkyl radicals and -OH radicals;
and the dimers formed by a disulphide bridge from one and/or other of the two sulphur atoms of the compounds of general formula I composed of the R" radicals or of the R'CO- radicals of the two molecules, and the corresponding thiazolidine forms.

2. Compound according to Claim 1, **characterized in that** R is a methyl group (-CH₃).

3. Compound according to Claim 2, **characterized in that** R' is a methyl group (-CH₃).

4. Compound according to Claim 3, **characterized in that** R" is hydrogen (Compound N-(N-acetyl-L-cysteinyl)-S-acetylcysteamine).

5. Compound according to Claim 3, **characterized in that** R" is an acetyl group (-COCH₃) (Compound N-(N,S-bisacetyl-L-cysteinyl)-S-acetylcysteamine).

6. Compound according to Claim 3, **characterized in that** R" is an isobutyryl group (-COCH(CH₃)₂) (Compound N-(N-acetyl-S-isobutyryl-L-cysteinyl)-S-acetylcysteamine).

7. Compound according to Claim 3, **characterized in that** R" is a pivaloyl group (-COC(CH₃)₃) (Compound N-(N-acetyl-S-pivaloyl-L-cysteinyl)-S-acetylcysteamine).

8. Compound according to Claim 2, **characterized in that** R' is selected from the isopropyl group (-CH(CH₃)₂), the tert-butyl group (-C(CH₃)₃) and the phenyl group (-C₆H₅).

9. Compound according to Claim 8, **characterized in that** R" is selected from hydrogen (-H), the acetyl group (-COCH₃), the i sobutyryl group (-COCH(CH₃)₂), the pivaloyl group (-COC(CH₃)₃) or the benzoyl group (-CO-C₆H₅).

10. Compound according to Claim 1, **characterized in that** R is an isopropyl group (-CH(CH₃)₂).

11. Compound according to Claim 10, **characterized in that** R' is selected from the methyl group (-CH₃), the isopropyl group (-CH(CH₃)₂), the tert-butyl group (-C(CH₃)₃) and the phenyl group (-C₆H₅).

12. Compound according to Claim 11, **characterized in that** R" is selected from hydrogen (-H), the acetyl group (-COCH₃), the isobutyryl group (-COCH(CH₃)₂), the pivaloyl group (-COC(CH₃)₃) or the benzoyl group (-CO-C₆H₅).

13. Compound according to Claim 8, **characterized in that** R" is the trityl group.

14. Compound according to Claim 11, **characterized in that** R" is the trityl group.

15. Compound according to Claims 1 to 14, **characterized in that** it is in the thiazolidine form.

16. Process for the preparation of a compound according to any one of Claims 1 to 14, **characterized in that** it comprises the following stages:
a) protection of the N-acyl-L-cysteine to provide the N-acyl-S-trityl-L-cysteine compound; then
b) coupling of the protected N-acyl-S-trityl-L-cysteine with the S-acylcysteamine hydrochloride to provide the N-(N-acyl-S-trityl-L-cysteinyl)-S-acylcysteamine compound.

17. Process for the preparation of a compound according to Claim 15, **characterized in that** it comprises the following stages:
a) protection of the N-acyl-L-cysteine to provide the N-acyl-S-trityl-L-cysteine compound; then
b) coupling of the protected N-acyl-S-trityl-L-cysteine with thiazolidine.

18. Process according to Claims 16 and 17 for the preparation of a compound according to Claims 1 to 12 and 15, **characterized in that** it further comprises the following stages:
c) deprotection of said compound obtained in said stage b); then
d) release of the free thiol of formula I.

19. Process according to Claims 16 and 18 for the preparation of the compound according to Claim 4, **characterized in that** said N-acyl-S-trityl-L-cysteine compound of stage a) is the compound N-acetyl-S-trityl-L-cysteine and **in that** said S-acylcysteamine hydrochloride of stage b) is S-acetylcysteamine hydrochloride.

20. Process according to Claim 18 for the preparation of a compound according to Claims 1 to 3, 5 to 12 and 15, **characterized in that** it further comprises an S-acylation stage e).

21. Process for the preparation of a compound according to Claim 1 in which R = R' and R" is hydrogen, **characterized in that** it comprises the following stages:
a) esterification of the carboxyl functional group of N-Boc-L-serine (1) with N-hydroxysuccinimide in N,N-dimethylformamide (DMF) in the presence of 1,3-dicyclohexylcarbodiimide (DCC) to form the active ester (1'); then,
b) *in situ* condensation of the active ester formed (1') with ethanolamine (2) to provide the compound N-(N-Boc-L-seryl)-2-aminoethanol (3); then,
c) Mitsunobu reaction on the compound (3) with triphenylphosphine and diisopropyl azodicarboxylate in the presence of thiocarboxylic acid in tetrahydrofuran to provide the compound N-(N-Boc-S-acyl-L-cysteinyl)-S-acylcysteamine (4); then,
d) deprotection of the compound (4) with trifluoroacetic acid.

22. Process according to Claim 21 for the preparation of the compound according to Claim 4, **characterized in that** said thiocarboxylic acid of stage c) is thioacetic acid.

23. Process for the preparation of a compound according to Claims 1 to 3 and 5 to 12 by S-acylation of the compound of Claim 4 in solution in pyridine in the presence of an anhydride R₂O or of an acid chloride R-Cl, **characterized in that** R is chosen from the CO-R¹ group in which R¹ is a linear or branched C₁-C₇ alkyl radical or an aryl group which is unsubstituted or substituted by one or more halogen atoms.

24. Process for the preparation of the compound according to Claim 5 by S-acylation of the compound of Claim 4 in solution in pyridine in the presence of acetic anhydride.

25. Process for the preparation of the compound according to Claim 6 by S-acylation of the compound of Claim 4 in solution in pyridine in the presence of isobutyryl chloride.

26. Process for the preparation of the compound according to Claim 7 by S-acylation of the compound of Claim 4 in solution in pyridine in the presence of pivaloyl chloride.

27. Precursor of a compound involved in the pathway for the biosynthesis of glutathione, **characterized in that** it is a compound according to any one of Claims 1 to 15.

28. Use of a compound according to any one of Claims 1 to 15 in the preparation of a product involved in the pathway for the biosynthesis of glutathione.

29. Use according to Claim 28, **characterized in that** said product is chosen from the group formed by N-acetyl-L-cysteine, cysteamine and L-cysteine.

30. Use of a compound according to any one of Claims 1 to 15 as antioxidizing agent.

31. Compound according to one of Claims 1 to 15 as medicament.

32. Compound according to one of Claims 1 to 15 as medicament intended to increase the intracellular and/or extracellular level of glutathione.

33. Use of a compound according to any one of Claims 1 to 15 in the preparation of a medicament intended to increase the intracellular and/or extracellular level of glutathione.

34. Pharmaceutical composition, in particular dermatological composition, **characterized in that** it comprises an effective amount of a compound according to one of Claims 1 to 15 and a pharmaceutically acceptable vehicle.

35. Use of a compound according to one of Claims 1 to 15 in the preparation of a medicament or of the pharmaceutical composition according to Claim 34 for the treatment and/or prevention of pathologies or disorders related to an intra- and/or extracellular depletion of glutathione.

36. Use according to Claim 35, **characterized in that** said pathologies are chosen from viral infections, bacterial infections, parasitic infections, diseases of the respiratory tract, neurodegenerative diseases, autoimmune diseases, cardiovascular diseases, cancers, diseases of the immune system, diabetes and preferably type I diabetes, ophthalmic pathologies or dermatological diseases.

37. Use according to Claim 36, **characterized in that** said pathologies are viral infections.

38. Use according to Claims 36 and 37, **characterized in that** said viral infections are infections caused by DNA viruses and RNA viruses, more particularly by retroid viruses, more particularly the human immunodeficiency virus (HIV) and preferably the type-1 human immunodeficiency virus (HIV-1) .

39. Use according to Claims 36 to 38, **characterized in that** said compound is the compound according to Claim 4.

40. Use according to Claims 36 to 38, **characterized in that** said compound is the compound according to one of Claims 5 to 15.

41. Pharmaceutical composition for the preventive and curative treatment of AIDS and the associated affected tissues, **characterized in that** it comprises a therapeutically effective amount of a compound according to Claims 1 to 15 and a pharmaceutically acceptable vehicle.

42. Product comprising at least one compound according to one of Claims 1 to 15 and at least one reverse transcriptase inhibitor as a combination product for simultaneous, separate or sequential use in antiviral therapy.

43. Product according to Claim 42, **characterized in that** said reverse transcriptase inhibitor is chosen, for example, from 3'-azido-3'-deoxythymidine (AZT), 2',3'-dideoxyinosine (ddI), 2',3'-dideoxycytidine (ddC), (-)-2',3'-dideoxy-3'-thiacytidine (3TC), 2',3'-didehydro-2',3'-dideoxythymidine (d4T) and (-)-2'-deoxy-5-fluoro-3'-thiacytidine (FTC), TIBO, HEPT, TSAO, α-APA, nevirapine, BAHP or phosphonoformic acid (PFA).

44. Use according to Claim 35, **characterized in that** said pathologies are cardiovascular diseases preferably chosen from the group consisting of arterial hypertension, arteriosclerosis, cerebral ischemia, cardiac ischaemia, ventricular arrhythmias, ventricular fibrillation and myocardial infarction.

45. Use according to Claim 35, **characterized in that** said pathologies are ophthalmic pathologies, in particular cataracts.

46. Use according to Claim 35, **characterized in that** said pathologies are diseases of the respiratory tract, in particular pulmonary emphysema, idiopathic pulmonary fibrosis, cystic fibrosis, chronic bronchitis, acute bronchitis or adult respiratory distress syndrome.

47. Use of a compound according to any one of Claims 1 to 15 in the preparation of a medicament or of the pharmaceutical composition according to Claim 34 intended for the preventive and/or curative treatment of noise-related hearing loss.

48. Use of a compound according to any one of Claims 1 to 15 in the preparation of a medicament or of the pharmaceutical composition according to claim 34 intended for the treatment of poisoning related to the oral or parenteral administration, as or not as an overdose, of substances preferably chosen from the group consisting of acetaminophen, nitrites, ethanol, acrylonitrile and heavy metals, more particularly gold, silver and mercury.

49. Use according to Claim 30 of a compound according to one of Claims 1 to 15 in the field of cosmetics.

50. Use according to Claim 49 to:
(i) prevent, erase and treat wrinkles or fine lines of the skin; and/or
(ii) combat cutaneous and/or subcutaneous slackening; and/or
(iii) improve the texture of the skin and rekindle the radiance of the skin; and/or
(iv) remove undesired hairs from the skin; and/or
(v) decrease the sizes of the pores of the skin; and/or
(vi) permanently deform the hair.

51. Cosmetic composition for the treatment of the skin and/or hair and/or body hair, **characterized in that** it comprises a compound according to one of Claims 1 to 15 and a cosmetically acceptable excipient.

52. Process for the cosmetic treatment of the skin for preventing, erasing and treating wrinkles or fine lines of the skin and/or combating cutaneous and/or subcutaneous slackening and/or improving the texture of the skin and rekindling the radiance of the skin and/or removing undesired hairs from the skin and/or decreasing the sizes of the pores of the skin which comprises the application, to the skin, of the cosmetic composition according to Claim 51.

53. Process for the cosmetic treatment of the hair for the permanent deformation of the hair comprising the application, to the hair, of a cosmetic composition according to Claim 51.

54. Use according to Claim 30 in the farm-produce industry and in particular for the preservation of the organoleptic and nutritional properties of drinks, in particular fruit juices, and/or food.
